# EUROPÄISCHE PATENTSCHRIFT

(11) **EP 3 223 738 B1**
(45) Veröffentlichungstag und Bekanntmachung des Hinweises auf die Patenterteilung: **01.09.2021**
(21) Anmeldenummer: 15807819.6
(22) Anmeldetag: 17.11.2015
(51) Int. Cl.: A61B 46/10, A61B 34/30, A61B 34/37, A61B 90/00, A61B 90/30

(54) **VORRICHTUNG ZUR ROBOTERGESTÜTZTEN CHIRURGIE**
DEVICE FOR ROBOT-ASSISTED SURGERY
DISPOSITIF DE CHIRURGIE ASSISTÉE PAR ROBOT

(30) Priorität: 27.11.2014 DE 102014117407
(43) Veröffentlichungstag der Anmeldung: 04.10.2017
(73) Patentinhaber: avateramedical GmbH, 07745 Jena (DE)
(72) Erfinder: BRAUN, Marcus, 71093 Weil im Schönbuch (DE); BARBER, Stephan, 76356 Weingarten (DE); SEEBER, Marcel, 07745 Jena (DE)
(74) Vertreter: Schaumburg und Partner Patentanwälte mbB
(86) Internationale Anmeldenummer: PCT/EP2015/076769
(87) Internationale Veröffentlichungsnummer: WO 2016/083189

(56) Entgegenhaltungen:
- WO-A2-2014/005689
- US-A1- 2004 049 205
- US-A1- 2006 235 436
- US-A1- 2012 184 955

## Beschreibung

Die Erfindung betrifft eine Vorrichtung zur robotergestützten Chirurgie mit mindestens in einem nicht sterilen Bereich angeordneten Manipulatorarm mit einer Koppeleinheit, die mindestens ein erstes Übertragungsmittel hat. Die Vorrichtung hat mindestens eine in einem sterilen Bereich angeordnete Sterileinheit, die mindestens ein zweites Übertragungsmittel hat und eine sterile Abdeckung zum Abschirmen des Manipulatorarms von dem sterilen Bereich. Die Erfindung betrifft weiterhin eine Anordnung zur robotergestützten Chirurgie, die eine solche Vorrichtung zur robotergestützten Chirurgie und mindestens eine Eingabeeinrichtung zur Eingabe mindestens eines Eingabekommandos umfasst. Ferner betrifft die Erfindung eine Sterilschleuse, insbesondere zum Einsatz in einer solchen Vorrichtung, sowie ein Verfahren zur robotergestützten Chirurgie, bei dem eine Sterileinheit mehrmals nacheinander mit einem Manipulatorarm verbindbar ist.

In der minimal invasiven Chirurgie werden zunehmend sogenannte Telemanipulatorsysteme, die auch als Roboterassistenzsysteme bezeichnet werden, eingesetzt. Das sterile Operationsfeld wird vor den nicht sterilen Elementen des Telemanipulatorsystems mit Hilfe einer sterilen Abdeckung geschützt. Durch die sterile Abdeckung wird sowohl eine Kontamination des sterilen Operationsfeldes als auch einer Verschmutzung des Telemanipulatorsystems durch Körperflüssigkeiten und/oder Gewebe des operierten Patienten oder des Operationspersonals verhindert. Dadurch wird das Risiko von Kreuzkontaminationen verringert.

Mit Hilfe des Telemanipulatorsystems werden chirurgische Instrumente und/oder Endoskope aufgrund von Bedieneingaben in ihrer Lage und Orientierung gesteuert und kommen dabei zwangsläufig in Körperkontakt mit dem zu operierenden Patienten, sodass die chirurgischen Instrumente und/oder Endoskope mit Körperflüssigkeiten und/oder Gewebe des operierten Patienten verunreinigt werden. Gleichzeitig müssen die chirurgischen Instrumente mechanisch, elektrisch und/oder optisch an das Telemanipulatorsystem angekoppelt werden, um eine aktive Positionierung und Ausrichtung des chirurgischen Instruments sowie eine gewünschte Betätigung eines chirurgischen Instruments realisieren zu können. Hierzu haben die chirurgischen Instrumente, Endoskope oder zu bedienende medizinische Geräte eine Koppelschnittstelle, die als Koppeleinheit ausgebildet sein kann und auch als Sterileinheit bezeichnet wird.

Das während eines chirurgischen Eingriffs genutzte Material einschließlich der verwendeten chirurgischen Apparate und Instrumente und die weiteren Bestandteile des Telemanipulatorsystems können in drei Kategorien eingeteilt werden:
Kategorie 1: Das Material ist steril und wird während des chirurgischen Eingriffs kontaminiert. Das Material wird nach der Operation entsorgt. Es erfolgt somit eine Einmalverwendung des Materials.
Kategorie 2: Das Material ist steril, wird während des chirurgischen Eingriffs kontaminiert und nach der Operation gereinigt und sterilisiert. Es erfolgt somit eine Mehrfachverwendung des Materials. Solche mehrfach verwendeten Materialien müssen entsprechend den Anforderungen an eine prozessfähige Sterilisierbarkeit konstruiert und produziert sein.
Kategorie 3: Das Material ist nicht steril. Während des chirurgischen Eingriffs wird durch eine sterile Abdeckung und Umverpackung eine Kontamination des sterilen Operationsfeldes verhindert. Gleichzeitig wird das nicht sterile Material vor Kontakt mit Körperflüssigkeiten und/oder Gewebe geschützt.

Ist es erforderlich, Geräte der Kategorie 1 oder der Kategorie 2 mit Geräten der Kategorie 3 zu koppeln, ist eine Sterilschnittstelle erforderlich, die eine Kontamination der Geräte der Kategorie 1 bzw. der Kategorie 2 durch die nicht sterilen Geräte der Kategorie 3 verhindert und umgekehrt eine Verunreinigung der Geräte der Kategorie 3 verhindert, da diese im allgemeinen als nicht sterilisierbare autoklavierbare Komponenten technisch ausgeführt sind. Die Ausführung von Geräten als sterilisierbare und autoklavierbare Komponenten erfordert eine besondere technische Auslegung des Geräts für den Sterilisationsprozess, sodass hierfür ein höherer Entwicklungsaufwand sowie ein erheblicher Validierungsaufwand zum Nachweis der Wirksamkeit des Sterilisationsprozesses erforderlich sind. Für einen solchen Nachweis ist es insbesondere erforderlich, das Gerät mehrfach nacheinander zu kontaminieren, zu sterilisieren, eine Wirksamkeitsprüfung der Sterilisation durchzuführen sowie eine Funktionsprüfung nach erfolgter Sterilisation durchzuführen. Dabei ist ein Nachweis zu erbringen, dass die Geräte nach jeder Sterilisation sicher sterilisiert und somit wieder verwendet werden könnten.

Aus dem Dokument US 7,666,191 B1 ist ein Telemanipulatorsystem bekannt, bei dem die nicht sterilen Manipulatorarme mittels einer Sterilfolie abgedeckt werden. Die Koppeleinheit des Manipulatorarms umfasst vier Rotationsaktuatoren, die mit einer ersten Seite eines in der Sterilfolie integrierten Steriladapters gekoppelt werden. Mit Hilfe des Steriladapters werden die Drehbewegungen der vier Rotationsaktuatoren der Koppeleinheit des Manipulatorarms in Eingriff mit vier in den Steriladapter integrierten drehbar gelagerten Übertragungsmittel gekoppelt. An der sterilen Außenseite des Steriladapters können diese an der Außenseite des Steriladapters sterilen Übertragungsmittel mit angetriebenen Elementen des sterilen chirurgischen Instruments in Eingriff gebracht werden. Ferner können über diesen Steriladapter elektrische Signale zwischen der Innenseite und der Außenseite des Steriladapters übertragen werden.

Somit wird mit Hilfe des Steriladapters verhindert, dass die Rotationsaktuatoren und die elektrischen Anschlüsse des sterilen chirurgischen Instruments direkt in Kontakt mit den Rotationsaktuatoren und den elektrischen Anschlüssen der Koppeleinheit des nicht sterilen Manipulatorarms kommen. Eine Kontamination des chirurgischen Instruments durch den Kontakt mit nicht sterilen Teilen des Manipulatorarms wird durch den Steriladapter verhindert. Bei dieser Lösung ist es jedoch erforderlich, dass der Steriladapter drehbar gelagerte Übertragungsmittel haben muss sowie Übertragungsmittel zur Übertragung von elektrischen Signalen, wodurch der Adapter aufwendig herzustellen und störanfällig ist. Insbesondere ist es aufwendig, die Drehbarkeit der Übertragungsmittel sicherzustellen, wenn die Übertragungsmittel in Kontakt mit Körperflüssigkeit kommen.

Grundsätzlich ist jedes Element in der Funktionskette zur Kopplung des Manipulatorarms und des Instruments eine mögliche Fehlerquelle und ist mit zusätzlichen Kosten verbunden. Der Steriladapter selbst ist als Teil der Sterilfolie zur Einmalverwendung vorgesehen.
Aus dem Dokument US 8,074,657 B2 ist ein weiterer Steriladapter bekannt, der eine Aktuatoreinheit zur Übertragung mechanischer Energie auf ein mit dem Steriladapter gekoppelten chirurgischen Instrument ermöglicht.

Aus dem Dokument US 2012/0184955 A1 ist eine sterile Barriere 224 bekannt, die zwei flexible Laufbuchsen zur Führung eines Katheters hat. Der Katheter wird zwischen beide Laufbuchsen in einen Führungskanal gefädelt und am anderen Ende, wo der Katheter aus dem Führungskanal austritt, ist jeweils eine Öffnung vorhanden. Dokument US2006235436 A1 offenbart weiteren einschlägigen Stand der Technik.

Aufgabe der vorliegenden Erfindung ist es, eine Vorrichtung und ein Verfahren zur robotergestützten Chirurgie anzugeben, bei denen eine sterile Kopplung eines in einem nicht sterilen Bereich angeordneten Manipulatorarms mit einer in einem sterilen Bereich angeordneten Sterileinheit einfach möglich ist. Ferner sind eine Anordnung zur robotergestützten Chirurgie innerhalb eines sterilen Bereichs sowie eine Sterilschleuse zum Koppeln einer Koppeleinheit eines Manipulatorarms mit einer Sterileinheit anzugeben.

Diese Aufgabe wird durch eine Vorrichtung zur robotergestützten Chirurgie mit den Merkmalen des Anspruchs 1 sowie durch eine Anordnung, durch eine Sterilschleuse und durch ein Verfahren mit den Merkmalen des jeweiligen unabhängigen Anspruchs gelöst. Vorteilhafte Weiterbildungen der Erfindung sind in den abhängigen Ansprüchen angegeben.

Bei der Erfindung wird insbesondere durch das Vorsehen einer Sterilschleuse, die sowohl mit einer Koppeleinheit als auch mit einer Sterileinheit verbindbar ist, beim Verbinden der Sterileinheit mit der Sterilschleuse die Schleusenklappe, vorzugsweise automatisch mechanisch, geöffnet, sodass das erste Übertragungsmittel der Koppeleinheit und das zweite Übertragungsmittel der Sterileinheit einander gegenüberliegend angeordnet sind, ohne dass ein weiteres Übertragungselement zwischen ihnen angeordnet ist. Dabei können sich die Übertragungsmittel direkt kontaktieren oder es erfolgt eine Übertragung über einen Luftspalt zwischen dem ersten Übertragungsmittel und dem zweiten Übertragungsmittel. Dadurch wird bei einer Verbindung der Sterileinheit mit der Sterilschleuse das durch die Schleusenklappe steril abgedeckte erste Übertragungsmittel für eine Übertragung mit dem zweiten Übertragungsmittel freigegeben. Beim Trennen der Sterileinheit von der Sterilschleuse wird zumindest das erste Übertragungsmittel und wieder steril abgeschirmt. Vorzugsweise ist die Sterilschleuse beim Verbinden und Trennen der Sterileinheit mit bzw. von der Sterilschleuse bereits mit der Koppeleinheit verbunden. Vorzugsweise bleibt die Sterilschleuse über den gesamtem Zeitraum des chirurgischen Eingriffs mit der Koppeleinheit verbunden, wobei die Sterileinheit mehrfach von der Koppeleinheit des Manipulatorarms getrennt und wieder mit diesen verbunden bzw. gegen eine weitere Sterileinheit ausgetauscht werden kann. Ferner ist es möglich, die Sterilschleuse mit der Koppeleinheit und der Sterileinheit derart zu verbinden, dass das erste Übertragungsmittel mit dem zweiten Übertragungsmittel in direkter Verbindung, vorzugsweise in direktem Eingriff, steht.

Durch die Erfindung ist es somit insbesondere möglich, die Sterilschleuse ohne mechanische und/oder elektrische Übertragungsmittel auszustatten, sodass sowohl eine sichere sterile Abschirmung des nicht sterilen Manipulatorarms und der nicht sterilen Koppeleinheit als auch eine sichere Kopplung des ersten Übertragungsmittels mit dem zweiten Übertragungsmittel ohne Zwischenschaltung von weiteren Übertragungsmitteln, insbesondere ohne Zwischenschaltung von weiteren mechanischen Übertragungsmitteln, möglich ist. Die sterile Abdeckung umfasst insbesondere ein steriles flexibles Material, wie eine Sterilfolie, und die mindestens eine Sterilschleuse.

Es ist vorteilhaft, wenn die Sterileinheit mindestens eine Sterilklappe hat, die in einem geschlossenen Zustand das zweite Übertragungsmittel steril abschirmt. Beim Verbinden der Sterileinheit mit der Sterilschleuse erfolgt dann jeweils eine Bewegung der Schleusenklappe und der Sterilklappe vom geschlossenen Zustand in den geöffneten Zustand, sodass eine direkte Übertragung zwischen dem ersten Übertragungsmittel und dem zweiten Übertragungsmittel durch eine von der Schleusenklappe und der Sterilklappe im geöffneten Zustand freigegebene Öffnung möglich ist. Beim Trennen der Sterileinheit von der Sterilschleuse erfolgt eine Bewegung der Schleusenklappe und der Sterilklappe jeweils vom geöffneten Zustand in den geschlossenen Zustand, sodass die Schleusenklappe nach dem Trennen das erste Übertragungsmittel und die Sterilklappe nach dem Trennen das zweite Übertragungsmittel vom sterilen Bereich abschirmen.

Vorzugsweise umfasst das erste Übertragungsmittel der Koppeleinheit mindestens ein Antriebselement und/oder mindestens eine erste elektrische Schnittstelle und/oder mindestens eine erste optische Schnittstelle. Das zweite Übertragungsmittel der Sterileinheit mindestens ein angetriebenes Element und/oder eine zweite elektrische Schnittstelle und/oder eine mindestens eine zweite optische Schnittstelle. Die Sterilschleuse ist mit der Koppeleinheit und der Sterileinheit derart verbindbar, dass das mindestens eine Antriebselement mit dem mindestens einen angetriebenen Element mechanisch direkt in Eingriff steht. Dadurch können zwischen der Koppeleinheit und der Sterileinheit einfach und sicher Momente übertragen werden. Ist die Sterileinheit Teil einer chirurgischen Instrumenteneinheit kann mit Hilfe der von der Koppeleinheit zur Sterileinheit übertragenen Momente das chirurgische Instrument der Instrumenteneinheit bewegt und/oder betätigt werden, indem mindestens ein Moment vom Antriebselement zum angetriebenen Element übertragen wird. Alternativ oder zusätzlich können die erste elektrische Schnittstelle mit der zweiten elektrischen Schnittstelle und/oder die erste optische Schnittstelle mit der zweiten optischen Schnittstelle gekoppelt werden.

Die Sterileinheit ist gemäß der Definition der Beschreibungseinleitung Material der Kategorie 1 und 2 und ist somit steril.

Ferner ist es vorteilhaft, wenn die Koppeleinheit mit einem ersten Verbindungsbereich der Sterilschleuse verbindbar ist und wenn die Sterileinheit mit einem zweiten Verbindungsbereich der Sterilschleuse verbindbar ist. Der erste Verbindungsbereich und der zweite Verbindungsbereich sind vorzugsweise auf voneinander abgewandten Seiten der Sterilschleuse angeordnet. Dadurch ist eine einfache Kopplung und somit eine einfache Handhabung sowohl der sterilen Abdeckung als auch der Sterileinheit vor, während und nach einem chirurgischen Eingriff möglich. Ferner ist es besonders vorteilhaft, wenn der zweite Verbindungsbereich als Aufnahmebereich ausgebildet ist, in dem die Sterileinheit beim Verbinden mit dem zweiten Verbindungsbereich zumindest teilweise aufnehmbar ist. Dadurch kann eine einfache und sichere Verbindung zwischen der Sterileinheit und der Sterilschleuse hergestellt werden. Insbesondere kann die Sterileinheit zumindest teilweise in den Aufnahmebereich hineingedrückt und dort verriegelt werden.

Ferner ist es vorteilhaft, wenn die Sterilschleuse einen dritten Verbindungsbereich hat, mit dem die flexible Abdeckung verbindbar ist, wobei der dritte Verbindungsbereich vorzugsweise umlaufend um die Sterilschleuse, insbesondere an der Mantelfläche umlaufend, vorzugsweise zwischen dem ersten und zweiten Verbindungsbereich, angeordnet ist. Durch die Sterilschleuse erfolgt eine einfache Verbindung des sterilen Bereichs und des nicht sterilen Bereichs zur Kopplung der Koppeleinheit mit der Sterileinheit, ohne dass die Sterileinheit derart kontaminiert wird, dass sie nach einer Trennung von der Sterilschleuse nicht mehr im sterilen Bereich verbleiben kann.

Vorzugsweise ist die Koppeleinheit am proximalen Ende des Manipulatorarms angeordnet. Alternativ oder zusätzlich ist die Sterileinheit Bestandteil eines chirurgischen Instruments, eines Endoskops und/oder eines medizinischen Geräts, wobei die Sterileinheit insbesondere am distalen Ende des chirurgischen Instruments, des Endoskops und/oder des medizinischen Geräts angeordnet ist. Dadurch kann die Sterilschleuse für verschiedene bei einer Operation eines Patienten benötigten Instrumente und Geräte genutzt werden, ohne dass unterschiedliche Sterilschleusen bzw. Sterilschleusen mit unterschiedlichen Wirkprinzipien, eingesetzt werden müssen.

Ferner ist es vorteilhaft, wenn der erste Verbindungsbereich der Sterilschleuse über eine erste lösbare Rastverbindung mit der Koppeleinheit verbindbar ist und der zweite Verbindungsbereich der Sterilschleuse über eine zweite lösbare Rastverbindung mit der Sterileinheit verbindbar ist. Dadurch ist die Sterilschleuse sowohl mit der Koppeleinheit als auch mit der Sterileinheit sicher verbindbar und einfach von diesen wieder trennbar, sodass eine einfache Handhabung sowohl der sterilen Abdeckung mit der Sterilschleuse als auch der Sterileinheit, insbesondere während einer Operation, möglich ist.

Besonders vorteilhaft ist es, wenn die Koppeleinheit mindestens einen Koppelsensor umfasst, der das Vorhandensein einer korrekt mit der Sterilschleuse verbundenen Sterileinheit detektiert. Ferner hat die Vorrichtung eine Steuereinheit, die eine Übertragung zwischen dem ersten Übertragungsmittel und dem zweiten Übertragungsmittel nur dann zulässt, wenn mit Hilfe des Koppelsensors eine korrekt mit der Sterilschleuse verbundene Sterileinheit detektiert worden ist. Bei einer weiteren vorteilhaften Ausführungsform detektiert der Koppelsensor mit Hilfe eines an der Sterileinheit vorgesehenen Detektionselements, das bei einer Verbindung mit der Sterilschleuse bis in den ersten Verbindungsbereich, mit dem die Koppeleinheit verbunden ist, ragt, dass sowohl die Sterileinheit mit dem zweiten Verbindungsbereich als auch die Koppeleinheit mit dem ersten Verbindungsbereich korrekt verbunden sind. Die Steuereinheit gibt eine Übertragung zwischen dem ersten Übertragungsmittel und dem zweiten Übertragungsmittel vorzugsweise nur dann frei bzw. lässt diese nur dann zu, wenn der Koppelsensor eine korrekte Verbindung zwischen der Sterileinheit und dem zweiten Verbindungsbereich und der Koppeleinheit mit dem ersten Verbindungsbereich detektiert hat.

Zusätzlich kann mit Hilfe des Koppelsensors einfach detektiert werden, ob zumindest die Sterileinheit korrekt mit der Sterilschleuse verbunden ist, sodass dann davon ausgegangen werden kann, dass die Sterileinheit korrekt mit der Sterilschleuse und über die Sterilschleuse korrekt mit der Koppeleinheit des Manipulatorarms verbunden ist. Dadurch ist eine gefahrlose Übertragung zwischen dem ersten Übertragungsmittel und dem zweiten Übertragungsmittel möglich.

Weiterhin ist es vorteilhaft, wenn die Koppeleinheit als erstes Übertragungsmittel mehrere Antriebselemente hat und wenn die Sterileinheit als zweites Übertragungsmittel mehrere angetriebene Elemente hat. Die Antriebselemente stehen dann mit den angetriebenen Elementen zur mechanischen Kopplung der Koppeleinheit mit der Sterileinheit bei einer Verbindung der Sterileinheit mit der Sterilschleuse bei einer Verbindung der Koppeleinheit mit der Sterilschleuse in direktem mechanischen Eingriff. Alternativ oder zusätzlich hat die Koppeleinheit als erstes Übertragungsmittel mindestens zwei erste elektrische Kontaktelemente und die Sterileinheit als zweites Übertragungsmittel zwei zu den ersten elektrischen Kontaktelementen komplementäre zweite elektrische Kontaktelemente. Die ersten Kontaktelemente und die zweiten Kontaktelemente stellen bei einer Verbindung der Koppeleinheit mit der Sterilschleuse und bei einer Verbindung der Sterileinheit mit der Sterilschleuse eine direkte elektrische Verbindung zwischen der Koppeleinheit und der Sterileinheit her. Diese elektrische Verbindung kann insbesondere zur Übertragung von hochfrequenter elektrischer Energie, insbesondere zur Hochfrequenzchirurgie, genutzt werden. Somit kann die Sterileinheit Bestandteil eines chirurgischen Hochfrequenzinstruments sein. Sind mehrere Antriebselemente und mehrere angetriebene Elemente vorgesehen, sind auf einfache Weise verschiedene Bewegungen und/oder Betätigungen eines über die Sterileinheit mit der Koppeleinheit gekoppelten chirurgischen Instruments möglich.

Besonders vorteilhaft ist es, wenn die Schleusenklappe den ersten Verbindungsbereich vom zweiten Verbindungsbereich trennt und wenn die Schleusenklappe beim Verbinden der Sterileinheit mit dem zweiten Verbindungsbereich automatisch öffnet. Beim Trennen der Sterileinheit von dem zweiten Verbindungsbereich schließt die Schleusenklappe automatisch. Dadurch ist eine einfachere und sichere Abdeckung der nicht sterilen Elemente der Koppeleinheit möglich, sodass Kontaminationen des sterilen Bereichs durch nicht sterile Elemente der Koppeleinheit einfach verhindert werden. Dabei ist es vorteilhaft, wenn die Schleusenklappe beim Verbinden der Sterileinheit mit dem zweiten Verbindungsbereich automatisch entriegelt wird und wenn die Schleusenklappe beim Trennen der Sterileinheit von dem zweiten Verbindungsbereich automatisch verriegelt wird. Dadurch ist eine sichere Abdeckung der nicht sterilen Elemente der Koppeleinheit sicher gewährleistet. Ein versehentliches Öffnen der Schleusenklappe, beispielsweise durch Berühren, wird auf einfache Weise wirkungsvoll vermieden.

Ferner ist es vorteilhaft, wenn die Sterilklappe der Sterileinheit das mindestens eine zweite Übertragungsmittel abdeckt und wenn die Sterilklappe beim Verbinden der Sterileinheit mit dem zweiten Verbindungsbereich automatisch öffnet sowie die Sterilklappe beim Trennen der Sterileinheit vom zweiten Verbindungsbereich automatisch schließt. Dadurch ist auch das möglicherweise kontaminierten zweite Übertragungsmittel sicher steril abgeschirmt, wenn die Sterileinheit wieder von der Sterilschleuse getrennt worden ist.

Ferner ist es vorteilhaft, wenn die Sterilklappe beim Verbinden der Sterileinheit mit dem zweiten Verbindungsbereich automatisch entriegelt wird und wenn die Sterilklappe beim Trennen der Sterileinheit von dem zweiten Verbindungsbereich automatisch verriegelt wird. Durch das automatische Ver- und Entriegeln wird ein versehentlicher Kontakt mit den durch einen möglichen Kontakt zwischen dem ersten Übertragungsmittel und dem zweiten Übertragungsmittel kontaminierten zweiten Übertragungsmittel der Sterileinheit einfach verhindert, indem die zweiten Übertragungsmittel mit Hilfe der Sterilklappe abgeschirmt werden und diese sicher verriegelt wird, sodass ein versehentlicher Kontakt mit dem zweiten Übertragungsmittel nach der Trennung der Sterileinheit von der Sterilschleuse nicht möglich ist.

Ferner ist es vorteilhaft, wenn die sterile Außenseite der Sterilklappe beim Verbinden der Sterileinheit mit dem zweiten Verbindungsbereich der dem zweiten Verbindungsbereich zugewandten sterilen Außenseite der Schleusenklappe gegenüberliegend angeordnet ist, wenn sowohl die Sterilklappe als auch die Schleusenklappe geöffnet sind. Besonders vorteilhaft ist es, wenn die sterilen Außenseiten der Sterilklappe und der Schleusenklappe im geöffneten Zustand einander zugewandt sind, sich vorzugsweise berühren. Durch das gegenüberliegende Anordnen der sterilen Außenseite der Schleusenklappe und der sterilen Außenseite der Sterilklappe ist eine Kontaminierung der Außenseite der jeweils anderen Klappe nicht möglich, da lediglich die Innenseiten durch einen Kontakt mit mindestens einem nicht sterilen Übertragungselement kontaminiert werden können.

Besonders vorteilhaft ist es, wenn die Sterileinheit Bestandteil eines chirurgischen Instruments ist, wobei die Sterileinheit insbesondere am distalen Ende des chirurgischen Instruments angeordnet ist.

Besonders vorteilhaft ist es, wenn die sterile Abdeckung und/oder die Sterilschleuse aus Polyethylen, Polyurethan und/oder Polycarbonat hergestellt sind. Dadurch ist sowohl eine einfache Herstellung der Abdeckung bzw. der Sterilschleuse als auch eine einfache uns sichere Handhabung der Abdeckung und der Sterilschleuse möglich.

Das chirurgische Instrument umfasst vorzugsweise mindestens einen in eine Körperöffnung eines Patienten einführbaren Endeffektor, wie eine Klemme, eine Schere, einen Greifer, einen Nadelhalter, einen Mikrodissektor, eine Klammervorrichtung, eine Heftvorrichtung, eine Spül- und/oder Absaugvorrichtung, eine Schneidklinge, eine Kauterisationssonde, einen Katheter und/oder eine Saugdüse. Dadurch kann das chirurgische Instrument wahlweise unterschiedliche Endeffektoren haben, die für gängige minimal-invasive Eingriffe, insbesondere bei der laparoskopischen Chirurgie, eingesetzt werden können. Es können jedoch auch andere chirurgische Instrumente zusätzlich oder alternativ eingesetzt werden. Insbesondere kann das chirurgische Instrument auch ein optisches chirurgisches Instrument, wie ein Endoskop, sein, welches dann weitere optische und elektrische Übertragungsmittel oder Schnittstellen, wie z. B. elektrische Kontakte zur Kamerasteuerung oder zur Bilddatenübertragung optischer Lichtleitfaserverbindungen, insbesondere zur Beleuchtung, aufweist.

Ein zweiter Aspekt betrifft eine Anordnung zur robotergestützten Chirurgie, insbesondere zu einer telerobotergestützten Prozedur innerhalb eines sterilen Feldes mit Hilfe eines sterilen chirurgischen Instruments. Diese Anordnung umfasst mindestens eine Vorrichtung nach Anspruch 1 oder nach einer zuvor angegebenen Weiterbildung; eine Anzeigeeinheit, die mindestens ein Bild des Operationsgebiets, in dem sich der Endeffektor des chirurgischen Instruments befinden kann, in Echtzeit, vorzugsweise als Bildfolge, ausgibt und mindestens eine Einrichtung zur Eingabe mindestens eines Eingabekommandos. Die Anordnung hat ferner eine Steuereinheit, die den Manipulatorarm und die über die Sterilschleuse mit der Koppeleinheit des Manipulatorarms verbundene Sterileinheit abhängig von dem Eingabekommando mit Hilfe mindestens einer Antriebseinheit positioniert. Dadurch ist eine einfache Steuerung des Manipulatorarms zum Positionieren der Sterileinheit und/oder eine Bedienhandlung zum Betätigen der Sterileinheit einfach möglich. Vorzugsweise hat die Eingabeeinrichtung ein von einer Bedienperson, wie einem Chirurgen, betätigbares Betätigungselement, wobei die Eingabeeinrichtung eine Raumpositionsänderung des Betätigungselements erfasst und ein der erfassten Raumpositionsänderung entsprechendes Eingabekommando erzeugt. Die Steuereinheit erzeugt abhängig vom Eingabekommando mindestens ein Steuerkommando, durch das dieselbe oder eine maßstäblich verkleinerte Raumpositionsänderung zumindest eines Endes der Sterileinheit und/oder des chirurgischen Instruments, an dessen distalen Ende die Sterileinheit angeordnet ist, und/oder durch das eine Betätigung oder eine untersetzte Betätigung des chirurgischen Instruments, an dessen distalen Ende die Sterileinheit angeordnet ist, bewirkt wird. Dadurch ist eine einfache Positionierung und/oder Betätigung des chirurgischen Instruments durch eine entfernt vom Patienten im Operationssaal oder außerhalb des Operationssaals befindliche Bedienperson einfach möglich. Als Ausgabe eines Bildes in Echtzeit wird die unmittelbare Ausgabe eines mit Hilfe einer Bilderfassungseinheit erfassten Bildes vorzugsweise als Videosequenz ohne die bei der Bildverarbeitung entstehenden Verzögerungen hinausgehende Verzögerungen.

Ferner ist es vorteilhaft, wenn die Anordnung mehrere Vorrichtungen zur robotergestützten Chirurgie nach Anspruch 1 oder nach einer angegebenen Weiterbildung hat. Die Eingabeeinrichtung hat vorzugsweise mindestens zwei von einer Bedienperson betätigbare Betätigungselemente, wobei die Eingabeeinrichtung eine Raumpositionsänderung jedes Betätigungselements erfasst und jeweils ein der erfassten Raumpositionsänderung entsprechendes Eingabekommando erzeugt. Die Steuereinheit erzeugt abhängig von jedem Eingabekommando jeweils mindestens ein Steuerkommando, durch das dieselbe oder eine maßstäblich veränderte Raumpositionsänderung zumindest eines Endes eines chirurgischen Instruments, an dessen distalen Ende die Sterileinheit angeordnet ist, der dem jeweiligen Betätigungselement zum Zeitpunkt der Betätigung zugeordneten Vorrichtung zur robotergestützten Chirurgie und/oder durch das eine Betätigung oder eine skalierte Betätigung dieses chirurgischen Instruments bewirkt wird. Dadurch kann die Operation mit mehreren Instrumenten durchgeführt werden, die sich gleichzeitig im Operationsfeld befinden bzw. die bei laparoskopischen Eingriffen gleichzeitig in dem Bauchraum des Patienten befinden.

Ein dritter Aspekt der Erfindung betrifft eine Sterilschleuse, die insbesondere zum Einsatz in einer Vorrichtung zur robotergestützten Chirurgie nach Anspruch 1 oder einer Weiterbildung dieser Vorrichtung geeignet ist. Die Sterilschleuse hat einen ersten Verbindungsbereich zum Verbinden der Sterileinheit mit einer nicht sterilen Koppeleinheit und einen zweiten Verbindungsbereich zum Verbinden der Sterilschleuse mit einer in einem sterilen Bereich angeordneten Sterileinheit. Die Sterilschleuse hat ferner einen umlaufenden dritten Verbindungsbereich zum Verbinden der Sterilschleuse mit einer flexiblen sterilen Abdeckung zum Trennen des sterilen Bereichs vom nicht sterilen Bereich. Ferner hat die Sterilschleuse mindestens eine Schleusenklappe, die in einem geschlossenen Zustand eine Öffnung zwischen dem ersten Verbindungsbereich und dem zweiten Verbindungsbereich steril verschließt und die in einem geöffneten Zustand die Öffnung zwischen dem ersten Verbindungsbereich und dem zweiten Verbindungsbereich freigibt. Durch eine solche Sterilschleuse wird eine einfache Handhabung der Sterileinheit beim Verbinden mit der Koppeleinheit ermöglicht, wobei sowohl die nicht sterilen Übertragungsmittel der Koppeleinheit steril abgeschirmt werden als auch eine direkte Kopplung eines ersten in der Koppeleinheit angeordneten Übertragungsmittels mit einem zweiten in der Sterileinheit angeordneten Übertragungsmittel einfach möglich ist. Insbesondere können Antriebselemente der Koppeleinheit und angetriebene Elemente der Sterileinheit bei geöffneter Schleusenklappe direkt miteinander in Eingriff stehen.

Besonders vorteilhaft ist es, wenn die Sterilschleuse mit der Sterileinheit und mit der Koppeleinheit derart verbindbar ist, dass mindestens ein als erstes Übertragungsmittel dienendes Antriebselement der Koppeleinheit mit mindestens einem als zweites Übertragungsmittel dienendes Antriebselement der Sterileinheit mechanisch direkt in Eingriff steht. Durch den direkten mechanischen Eingriff können Momente von dem Antriebselement zu dem angetriebenen Element übertragen werden, sodass eine Übertragung von Momenten zwischen dem nicht sterilen Bereich und dem sterilen Bereich durch die Sterilschleuse hindurch ohne Zwischenschaltung von weiteren Übertragungsmitteln einfach möglich ist. Somit sind zusätzliche Übertragungsmittel zur Verbindung der Antriebselemente und der angetriebenen Elemente nicht erforderlich. Solche Übertragungsmittel sind sowohl störanfällig als auch relativ aufwendig in eine sterile Abdeckung zu integrieren.

Besonders vorteilhaft ist es, wenn der dritte Verbindungsbereich der Sterilschleuse an einer Außenseite der Sterilschleuse zwischen dem ersten Verbindungsbereich und dem zweiten Verbindungsbereich angeordnet ist. Insbesondere ist der dritte Verbindungsbereich umlaufend an einer Mantelfläche der Sterilschleuse angeordnet. Die Verbindung zwischen der Sterilschleuse und einem sterilen flexiblem Abdeckungsmaterial kann dabei mit Hilfe einer Klemm-, Klett-, Schweiß und/oder Klebeverbindung erfolgen. Dadurch kann das sterile flexible Abdeckungsmaterial einfach mit der Außenseite der Sterilschleuse verbunden werden, sodass das Abdeckungsmaterial zusammen mit der Sterilschleuse eine zusammenhängende sterile Abdeckung bildet.

Zum Ausbilden einer Klemmverbindung kann der dritte Verbindungsbereich als Klemmbereich ausgeführt sein, sodass das flexible Abdeckungsmaterial mit den Klemmelementen des dritten Verbindungsbereichs verbunden werden kann. Alternativ oder zusätzlich kann der dritte Verbindungsbereich als Klebebereich ausgebildet sein, durch den das sterile flexible Abdeckungsmaterial mit Hilfe von Klebstoff mit dem dritten Verbindungsbereich verbindbar ist. Alternativ oder zusätzlich kann das sterile flexible Abdeckungsmaterial über eine Schweißverbindung mit dem dritten Verbindungsbereich verbunden sein.

Ferner ist es vorteilhaft, wenn die Schleusenklappe beim Verbinden der Sterileinheit mit dem ersten Verbindungsbereich automatisch öffnet und wenn die Sterilschleuse beim Trennen der Steuereinheit von dem ersten Verbindungsbereich die Schleusenklappe automatisch wieder schließt. Das Öffnen und Schließen der Schleusenklappe erfolgt dabei vorzugsweise mechanisch, wobei die Schleusenklappe gegen eine Federkraft geöffnet und durch die Federkraft geschlossen werden kann. Vorzugsweise ist die Schleusenklappe im geschlossenen Zustand verriegelt, sodass sie nicht durch eine Kraft auf die geschlossene Schleusenklappe geöffnet werden kann. Dadurch ist eine einfache und sichere Handhabung der Sterilschleuse möglich. Insbesondere werden nicht sterile Bereiche der Koppeleinheit mit Hilfe der Schleusenklappe abgedeckt, wenn die Sterileinheit nicht mit der Sterilschleuse verbunden ist.

Ein vierter Aspekt der Erfindung betrifft ein Verfahren zum sterilen Abdecken eines Manipulatorarms für robotergestützte Chirurgie, insbesondere unter Verwendung einer Vorrichtung nach Anspruch 1 oder einer zuvor beschriebenen Weiterbildung, einer Anordnung gemäß dem zweiten Aspekt der Erfindung oder einer Weiterbildung dieser Anordnung oder unter Verwendung einer Sterilschleuse gemäß dem dritten Aspekt der Erfindung oder einer angegebenen Weiterbildung in dieser Sterilschleuse. Bei dem Verfahren wird ein im nicht sterilen Bereich angeordneter Manipulatorarm mit Hilfe einer sterilen Abdeckung und einer in die Abdeckung integrierten Sterilschleuse von einem sterilen Bereich abgeschirmt. Eine nicht sterile Koppeleinheit des Manipulatorarms wird mit einem ersten Verbindungsbereich der Sterilschleuse verbunden. Eine Öffnung zwischen dem ersten Verbindungsbereich und einem zweiten Verbindungsbereich der Sterilschleuse wird mit Hilfe einer Schleusenklappe verschlossen. Die Schleusenklappe wird beim Verbinden einer in einem sterilen Bereich angeordneten Sterileinheit mit dem zweiten Verbindungsbereich der Sterilschleuse automatisch geöffnet, sodass eine direkte Übertragung zwischen dem ersten Übertragungsmittel und dem zweiten Übertragungsmittel der Sterileinheit bei geöffneter Schleusenklappe möglich ist. Durch das Öffnen der Schleusenklappe ist die Öffnung zwischen dem ersten Verbindungsbereich und dem zweiten Verbindungsbereich geöffnet.

Die Schleusenklappe wird beim Trennen der Sterileinheit von dem zweiten Verbindungsbereich automatisch geschlossen, wodurch die Öffnung zwischen dem ersten Verbindungsbereich und dem zweiten Verbindungsbereich wieder steril verschlossen wird. Die Schleusenklappe wird beim Verbinden der im sterilen Bereich angeordneten Sterileinheit oder einer weiteren im sterilen Bereich angeordneten Sterileinheit mit dem zweiten Verbindungsbereich der Sterilschleuse wieder automatisch geöffnet, sodass eine direkte Übertragung zwischen dem ersten Übertragungsmittel und dem zweiten Übertragungsmittel oder einem weiteren zweiten Übertragungsmittel der weiteren Sterileinheit wieder möglich ist. Dadurch ist eine einfache Kopplung zwischen der Koppeleinheit und der Sterileinheit möglich, wobei die Sterileinheit erforderlichenfalls mehrmals von der Sterilschleuse getrennt werden kann, ohne dass der sterile Bereich kontaminiert wird. Dies ist selbst dann sichergestellt, wenn das zweite Übertragungsmittel, insbesondere durch ein Kontakt mit dem ersten Übertragungsmittel kontaminiert worden ist.

Insbesondere dann, wenn die Sterileinheit nur einmal verwendet wird, muss diese keine Sterilklappe haben. Die Sterileinheit wird dann nach dem Trennen von der Sterilschleuse während einer Operation sofort aus dem sterilen Bereich entfernt.

Jedoch ist es vorteilhaft, wenn die Sterileinheit mindestens eine Sterilklappe hat, die in einem geschlossenen Zustand das zweite Übertragungsmittel steril abschirmt. Die Schleusenklappe und die Sterilklappe werden beim Verbinden der Sterileinheit mit der Sterilschleuse jeweils vom geschlossenen Zustand in den geöffneten Zustand bewegt, sodass eine direkte Übertragung zwischen dem ersten Übertragungsmittel und dem zweiten Übertragungsmittel durch eine von der Sterilklappe und der Schleusenklappe im geöffneten Zustand freigegebene Öffnung möglich ist. Beim Trennen der Sterileinheit von der Sterilschleuse werden die Schleusenklappe und die Sterilklappe jeweils vom geöffneten Zustand in den geschlossenen Zustand bewegt, sodass nach dem Trennen das erste Übertragungsmittel mit Hilfe der Schleusenklappe und das zweite Übertragungsmittel mit Hilfe der Sterilklappe vom sterilen Bereich abgeschirmt sind.

Dabei ist es vorteilhaft, wenn die Öffnung der Sterileinheit durch die Sterilklappe in deren geschlossenen Zustand derart verschlossen wird, dass das zweite Übertragungsmittel hinter der Sterilklappe angeordnet ist und wenn die Sterilklappe beim Verbinden mit der Sterilschleuse derart geöffnet wird, dass ein Zugriff auf das zweite Übertragungsmittel möglich ist. Vorzugsweise wird die Sterilklappe beim Verbinden der Sterileinheit mit dem zweiten Verbindungsbereich der Sterilschleuse automatisch geöffnet. Beim Trennen der Sterileinheit vom zweiten Verbindungsbereich der Sterilschleuse wird die Sterilklappe automatisch geschlossen. Dadurch ist eine sichere Handhabung der Sterileinheit und der Sterilschleuse zusammen mit einer nicht sterilen Koppeleinheit möglich.

Des Weiteren ist es vorteilhaft, wenn das erste Übertragungsmittel mindestens ein Antriebselement umfasst und wenn das zweite Übertragungsmittel mindestens ein angetriebenes Element umfasst. Während des Verbindens der Sterileinheit mit dem zweiten Verbindungsbereich der Sterilschleuse werden die Schleusenklappe der Sterilschleuse und eine Sterilklappe der Sterileinheit derart geöffnet, dass während des Verbindens der Sterileinheit in dem zweiten Verbindungsbereich das Antriebselement mit dem angetriebenen Element direkt in Eingriff gebracht wird. Dies erfolgt insbesondere ohne Zwischenschaltung von weiteren Übertragungsmitteln, insbesondere ohne Zwischenschaltung eines bewegten Übertragungsmittels, sodass eine Kontamination zumindest der Außenseite der Sterileinheit sicher vermieden wird, wodurch diese auch nach einer Trennung von der Sterilschleuse einfach im sterilen Bereich verbleiben und dort abgelegt werden kann.

Dabei ist es nicht von Nachteil wenn das sterile angetriebene Element beim ersten Kontakt mit dem Antriebselement kontaminiert wird, weil das kontaminierte angetriebene Element während des Trennens der Sterileinheit von dem zweiten Verbindungsbereich durch die Sterilklappe steril abgeschirmt wird. Vorzugsweise werden beim Trennen der Sterileinheit vom ersten Verbindungsbereich sowohl die Schleusenklappe als auch die Sterilklappe derart geschlossen und vorzugsweise verriegelt, dass jeweils ein Zugangsbereich zum Antriebselement und zum angetriebenen Element steril abgeschirmt sind. Vorzugsweise sind die Sterilklappe und die Schleusenklappe in geschlossenem Zustand mechanisch verriegelt, sodass weder die Schleusenklappe noch die Sterilklappe manuell geöffnet werden kann. Dadurch ist eine sterile Abdeckung von nicht sterilen oder kontaminierten Elementen der Koppeleinheit und der Sterileinheit sichergestellt, sodass der sterile Bereich auch nach dem Trennen der Sterileinheit von der Sterilschleuse nicht kontaminiert wird.

Insgesamt ermöglicht ein erfindungsgemäßes Verfahren eine einfache und sichere Handhabung, insbesondere einen einfachen und sicheren Wechsel der Sterileinheit, insbesondere einer die Sterileinheit umfassenden Instrumenteneinheit mit einem chirurgischen Instrument während eines chirurgischen Eingriffs.

Bei allen Ausführungsformen und Weiterbildungen kann die Sterilschleuse zwei Schleusenklappen und die Sterileinheit zwei Sterilklappen haben.

Die Sterilschleuse ist bei allen beschriebenen Ausführungsformen kein Bestandteil der Funktionskette zur Übertragung von elektrischer Energie, von elektrischen oder optischen Signalen und/oder mechanischer Energie zwischen dem Manipulatorarm und der Sterileinheit. Vielmehr kann die Sterilschleuse ein festes Formteil und ein zumindest die Schleusenklappe umfassendes Schleusenklappensystem umfassen, das nicht sterile erste Übertragungsmittel der Koppeleinheit so abgeschirmt, dass dieses und die gesamte Koppeleinheit nach Anbringung der sterilen Abdeckung mit der Sterilschleuse gegenüber der sterilen Umgebung steril abgedeckt sind. Der Öffnungsmechanismus des Schleusenklappensystems ist dabei vorzugsweise konstruktiv so ausgeführt, dass dieser nicht durch versehentliche Betätigung von außen geöffnet werden kann. Weiterhin werden auch die zweiten Übertragungsmittel durch ein steriles Gehäuse der Sterileinheit und durch die mindestens eine Sterilklappe der Sterileinheit steril abgeschirmt. Diese zweiten Übertragungsmittel umfassen insbesondere mindestens ein angetriebenes Element, vorzugsweise mindestens ein angetriebenes Element zur Aufnahme von rotatorischen und/oder translatorischen Stellkräften. Vorzugsweise ist mindestens ein angetriebenes Element zur Aufnahme rotatorischer und ein angetriebenes Element zur Aufnahme translatorischer Stellkräfte vorgesehen.

Zusätzlich kann mindestens eine elektrische Verbindung zur Übertragung von hochfrequenter Energie zur Hochfrequenzchirurgie vorgesehen sein. Besonders vorteilhaft ist es, wenn die Sterileinheit mindestens zwei angetriebene Elemente zur Aufnahme rotatorischer Stellkräfte und zwei angetriebene Elemente zur Aufnahme translatorischer Stellkräfte hat. Die Koppeleinheit des Manipulatorarms hat dann als erstes Übertragungsmittel zwei Antriebseinheiten zum Erzeugen rotatorischer Stellkräfte, die jeweils in direktem Eingriff mit den als zweite Übertragungsmittel dienenden komplementären angetriebenen Elementen zur Aufnahme der rotatorischen Stellkräfte der Sterileinheit sind. Weiterhin hat die Koppeleinheit als erstes Übertragungsmittel zwei Antriebselemente zum Erzeugen translatorischer Stellkräfte, die im direkten Eingriff mit als zweites Übertragungsmittel dienenden angetriebenen Elementen zur Aufnahme translatorischer Stellkräfte sind. Ein die Sterileinheit umfassendes chirurgisches Instrument ist insbesondere ein laparoskopisches Instrument.

Vorzugsweise sind Öffnungsmechanismus des Sterilklappensystems und/oder des Schleusenklappensystems konstruktiv so ausgeführt, dass sie nicht durch versehentliche Betätigungen von außen ausgelöst werden können, sondern nur bei einer korrekten Kopplung der Sterileinheit mit der Sterilschleuse. Öffnungsmechanismus des Sterilklappensystems und des Schleusenklappensystems sind vorzugsweise konstruktiv so ausgeführt, dass beim Verbinden der Sterileinheit mit der Sterilschleuse die Klappen des Schleusenklappensystems und des Sterilklappensystems durch entsprechende Eingriffselemente automatisch entriegelt und geöffnet werden. Damit können die als Übertragungsmittel dienenden Antriebselemente und die angetriebenen Elemente sowie elektrische Kontaktelemente direkt miteinander in Eingriff bzw. in Kontakt gebracht werden. Somit wird eine direkte Verbindung zwischen den nicht sterilen Antriebselementen der Koppeleinheit und den angetriebenen Elementen der Sterileinheit eines chirurgischen Instruments hergestellt. Dadurch kann eine Kontamination der zuvor sterilen angetriebenen Elemente der Sterileinheit erfolgen.

Wird die Sterileinheit wieder von der Sterilschleuse getrennt, werden sowohl die Schleusenklappen des Schleusenklappensystems der Sterilschleuse als auch die Sterilklappe des Sterilklappensystems der Sterileinheit wieder geschlossen, insbesondere bevor die Sterileinheit vollständig aus der Sterilschleuse entnommen worden ist. Damit ist sichergestellt, dass zu keinem Zeitpunkt sowohl die nicht sterilen Teile der Koppeleinheit als auch die nicht mehr sterilen angetriebenen Elemente der Sterileinheit und/oder die elektrischen Kontakte der Sterileinheit in Kontakt mit dem sterilen Operationsfeld und dem Patientenumfeld kommen können und dies kontaminieren könnten. Dadurch kann die Sterileinheit mit dem geschlossenen Sterilklappensystem direkt im sterilen Patientenumfeld abgelegt werden und so bis zur erneuten Verwendung, d. h. bis zur erneuten Verbindung mit dem ersten Verbindungsbereich der Sterilschleuse, ohne eine Kontamination des sterilen Patientenumfelds bereitgehalten werden.

Die als erste Übertragungsmittel dienenden Antriebselemente und die als zweites Übertragungsmittel dienenden angetriebenen Elemente sind vorzugsweise derart ausgeführt, dass ein laparoskopisches chirurgisches Instrument in insgesamt vier Freiheitsgraden bewegt werden kann, nämlich:
1. Rotation des Instrumentenschaftes
2. Rotation der Instrumentenspitze unabhängig vom Instrumentenschaft
3. Abwinken der Instrumentenspitze gegenüber dem Instrumentenschaft
4. Betätigung des chirurgischen Instruments, insbesondere zum Erzeugen einer Relativbewegung von zwei zueinander beweglich angeordneten Elementen, wie der Greifbewegung der Instrumentenspitze oder von Scherenklingen.

Das sterile Gehäuse der Sterileinheit wird während der Verbindung mit der Sterilschleuse vorzugsweise in einen Aufnahmebereich des zweiten Verbindungsbereich hineingedrückt und durch eine mechanische Raste an der Sterilschleuse gegen unbeabsichtigtes Lösen gesichert. Die mechanische Raste erzeugt somit eine Rastverbindung zwischen der Sterilschleuse und der Sterileinheit. Zum Trennen der Sterileinheit von der Sterilschleuse wird manuell eine Entriegelungstaste betätigt, sodass die Sterileinheit vom zweiten Verbindungsbereich getrennt, vorzugsweise aus dem Aufnahmebereich des zweiten Verbindungsbereichs entnommen, werden kann.

Als proximal wird allgemein ein dem Patienten zugewandtes Ende eines beliebigen Elements angesehen. Als distal wird allgemein ein dem Patienten abgewandtes Ende eines Elements angesehen.

Weitere Merkmale und Vorteile ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische Darstellung eines Systems zur robotergestützten Chirurgie mit einem Manipulator, der vier Manipulatorarme hat, mit denen jeweils eine Sterileinheit einer Instrumenteneinheit verbindbar ist;
- Figur 2: eine Ansicht der in Figur 1 dargestellten Manipulators von vorn;
- Figur 3: eine perspektivische Darstellung eines Abschnitts eines Manipulatorarms mit einer Koppeleinheit zum Koppeln des Manipulatorarms mit einer eine Sterileinheit umfassenden Instrumenteneinheit, einer mit der Koppeleinheit gekoppelten Sterilschleuse und mit einer mit der Sterilschleuse gekoppelten Sterileinheit der Instrumenteneinheit;
- Figur 4: eine weitere perspektivische Darstellung der Anordnung nach Figur 3;
- Figur 5: eine Anordnung zum Verbinden der in einem sterilen Bereich einer angeordneten Instrumenteneinheit mit der nicht sterilen Koppeleinheit eines Manipulatorarms;
- Figur 6: eine schematische Darstellung der Koppeleinheit des Manipulatorarms;
- Figur 7: einen Längsschnitt der Koppeleinheit nach Figur 6;
- Figur 8: eine perspektivische Darstellung der Sterilschleuse mit geschlossenen und verriegelten Sterilklappen;
- Figur 9: eine perspektivische Darstellung der Sterilschleuse nach Figur 8 mit geöffneten Sterilklappen;
- Figur 10: eine teilgeschnittene Seitenansicht der Sterilschleuse;
- Figur 11: eine Schnittdarstellung der Sterilschleuse nach Figur 10 entlang der Schnittlinie A-A;
- Figur 12: eine Schnittdarstellung der Sterilschleuse nach Figur 10 entlang der Schnittlinie B-B;
- Figur 13: eine Schnittdarstellung der Sterilschleuse nach Figur 10 entlang der Schnittlinie C-C;
- Figur 14: eine Detailansicht mit teilweise geöffneten Sterilklappen und mit einer mit den Sterilklappen in Eingriff stehenden Führungsgabel;
- Figur 15: eine perspektivische Darstellung der Instrumenteneinheit mit geöffneten Sterilklappen der Sterileinheit;
- Figur 16: eine perspektivische Darstellung der Instrumenteneinheit nach Figur 15 mit geschlossenen Sterilklappen;
- Figur 17: eine Seitenansicht der Instrumenteneinheit;
- Figur 18: eine Schnittdarstellung der Instrumenteneinheit nach Figur 17 entlang der Schnittlinie E-E;
- Figur 19: eine Schnittdarstellung der Instrumenteneinheit nach Figur 17 entlang der Schnittlinie F-F;
- Figur 20: einen Ausschnitt einer Ansicht der Instrumenteneinheit von unten mit geschlossenen und verriegelten Sterilklappen;
- Figur 21: den Ausschnitt der Instrumenteneinheit nach Figur 20 mit entriegelten und geöffneten Sterilklappen;
- Figur 22: eine Draufsicht auf das Sterilklappensystem der Sterileinheit mit geschlossenen Sterilklappen,
- Figur 23: eine Schnittdarstellung des Sterilklappensystems Figur 22 entlang der Schnittlinie G-G;
- Figur 24: eine Draufsicht des Sterilklappensystems nach den Figuren 22 und 23 mit geöffneten Sterilklappen;
- Figur 25: eine Schnittdarstellung des Sterilklappensystems nach Figur 24 entlang der Schnittlinie H-H;
- Figur 26: eine perspektivische Detailansicht der Sterilklappe mit einer Führungsklappe der Sterileinheit;
- Figur 27: eine Draufsicht auf die Führungsklappe und die Sterilklappe nach Figur 26 in einem verriegelten Zustand;
- Figur 28: eine Draufsicht auf die Sterilklappe und die Führungsklappe in einem entriegelten Zustand;
- Figur 29: eine perspektivische Darstellung der Sterilklappe und der Führungsklappe im geöffneten Zustand;
- Figur 30: eine teilgeschnittene Darstellung einer Anordnung mit der Sterileinheit und der Sterilschleuse in einem verbundenen Zustand;
- Figur 31: eine Schnittdarstellung der Anordnung nach Figur 30 entlang der Schnittlinie I-I;
- Figur 32: eine Schnittdarstellung der Anordnung nach Figur 30 entlang der Schnittlinie J-J,
- Figur 33: eine Schnittdarstellung der Anordnung nach Figur 30 entlang der Schnittlinie K-K;
- Figur 34: eine Schnittdarstellung der Anordnung nach Figur 30 entlang der Schnittlinie L-L;
- Figur 35: Elemente eines Sterilklappensystem der Sterileinheit und eines Schleusenklappensystems der Sterilschleuse der Anordnung nach den Figuren 30 bis 34;
- Figur 36: eine Seitenansicht der Anordnung nach den Figuren 30 bis 35;
- Figur 37: eine Schnittdarstellung des Abschnitts der Anordnung nach Figur 36 entlang der Schnittlinie M-M
- Figur 38: eine Schnittdarstellung der Anordnung nach Figur 36 entlang der Schnittlinie N-N;
- Figur 39: eine Draufsicht auf eine Anordnung der Koppeleinheit, Sterilschleuse und Instrumenteneinheit;
- Figur 40: eine Schnittdarstellung der Anordnung nach Figur 39 entlang der Schnittlinie O-O in einer ersten Position zum Verbinden der Instrumenteneinheit mit der mit der Koppeleinheit gekoppelten Sterilschleuse;
- Figur 41: eine Schnittdarstellung der Anordnung nach Figur 39 entlang der Schnittlinie O-O in einer zweiten Position zum Verbinden der Instrumenteneinheit mit der mit der Koppeleinheit gekoppelten Sterilschleuse;
- Figur 42: eine Schnittdarstellung der Anordnung nach Figur 39 entlang der Schnittlinie O-O in einer dritten Position zum Verbinden der Instrumenteneinheit mit der mit der Koppeleinheit gekoppelten Sterilschleuse;
- Figur 43: einen Ausschnitt einer Instrumenteneinheit gemäß einer zweiten Ausführungsform;
- Figur 44: eine sterile Abdeckung mit einer Sterilschleuse gemäß einer zweiten Ausführungsform;
- Figur 45: einen Ausschnitt einer Instrumenteneinheit gemäß einer dritten Ausführungsform; und
- Figur 46: eine sterile Abdeckung mit einer Sterilschleuse gemäß einer dritten Ausführungsform.

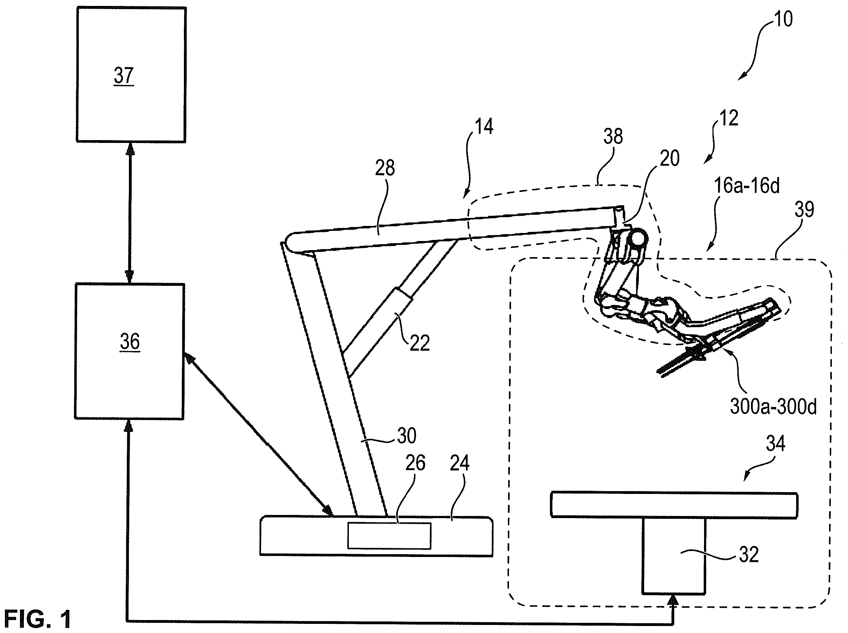

Figur 1 zeigt eine schematische Darstellung eines Systems 10 zur robotergestützten Chirurgie mit einem Manipulator 12, der ein Stativ 14 und vier Manipulatorarme 16a bis 16d hat. Bei anderen Ausführungsbeispielen kann der Manipulator 12 auch mehr oder weniger Manipulatorarme 16a bis 16d haben. Jeder Manipulatorarm 16a bis 16d ist mit einer sterilen Instrumenteneinheit 300a bis 300d über eine Koppeleinheit des Manipulatorarms 16a bis 16d verbunden. Die Instrumenteneinheit 300a bis 300d ist steril und umfasst neben einer Sterileinheit zum Koppeln der Instrumenteneinheit 300a bis 300d mit der Koppeleinheit des Manipulatorarms 16a bis 16d ein chirurgisches Instrument, insbesondere mit einem End-Effektor, wobei der End-Effektor mit Hilfe von der Koppeleinheit des Manipulatorarms 16a bis 16d bewegt und/oder betätigt werden kann. Alternativ zum chirurgischen Instrument kann die Instrumenteneinheit 300a bis 300d auch ein optisches Instrument, insbesondere ein Endoskop, und/oder ein medizinisches Gerät, insbesondere zum Applizieren eines Medikaments, zum Abgeben einer Spülflüssigkeit und/oder zum Absaugen von Spülflüssigkeit und/der Sekret, umfassen.

Das Stativ 14 hat ein auf dem Fußboden eines Operationssaals stehenden Stativfuß 24. Die Manipulatorarme 16a bis 16d sind mit einem Stativkopf 20 des Stativs 14 verbunden. Bei anderen Ausführungsformen kann das Stativ auch ein Deckenstativ sein.

Die Position des Stativkopfs 20 ist mit Hilfe einer ersten Antriebseinheit 22 und mit einer in dem Stativfuß 24 angeordneten zweiten Antriebseinheit 26 einstellbar. Mit Hilfe der Antriebseinheit 22 sind Stativarme 28, 30 relativ zueinander bewegbar. Mit Hilfe der Antriebseinheit 26 kann die Neigung des Stativarms 30 relativ zur Aufstellfläche des Stativfußes 24 geändert und/oder der Stativarm 30 um eine senkrechte Drehachse gedreht werden. Im Allgemeinen erfolgt die Positionierung des Stativkopfs 20 vor einer Operation eines Patienten. Während der Operation bleibt die Lage des Stativkopfs 20 zu der Säule 32 eines Operationstischs 34 üblicherweise unverändert. Der Manipulator 12 wird mit Hilfe einer Steuereinheit 36 gesteuert. Die Steuereinheit 36 ist über eine Daten- und/oder Steuerleitung mit einer Ein- und Ausgabeeinheit 37 verbunden, die insbesondere ein Bild des Operationsgebiets an eine Bedienperson in Echtzeit mit Hilfe mindestens einer Anzeigeeinheit ausgibt. Die Bedienperson macht Bedieneingaben, durch die die Instrumenteneinheiten 300a bis 300d während der Operation des Patienten positioniert und betätigt werden. Die Ein- und Ausgabeeinheit 37 dient somit als Mensch-Maschinen-Schnittstelle.

Die Steuereinheit 36 ist ferner über eine Steuer- und/oder Datenverbindung mit einer nicht dargestellten Steuereinheit des Operationstischs 34 verbunden. Über diese Steuer- und/oder Datenverbindung wird sichergestellt, dass die Lage der Patientenlagerfläche oder von Segmenten der Patientenlagerfläche des Operationstischs 34 nur dann verändert werden kann, wenn dies aufgrund der Positionierung der Instrumenteneinheiten 300a bis 300d für einen zu operierenden Patienten gefahrlos möglich ist.

Der Operationstisch 34 sowie die Instrumenteneinheiten 300a bis 300d sind in einem sterilen Operationsbereich 39 angeordnet. Die Manipulatorarme 16a bis 16d und das Stativ 14 sind nicht steril. Die in dem sterilen Operationsbereich 39 ragenden Bereiche des Manipulators 12, d.h. die Manipulatorarme 16a bis 16d, der Stativkopf 20 und ein Teil des Stativarms 28, sind in einer mit Hilfe der Strichlinie angedeuteten sterilen flexiblen Hülle 38, wie einer Sterilfolie, steril verpackt, sodass sie gefahrlos im sterilen Operationsbereich 39 angeordnet werden können. Die Ein- und Ausgabeeinheit 37 ist außerhalb des sterilen Bereichs 39 angeordnet und muss deshalb nicht steril verpackt werden.

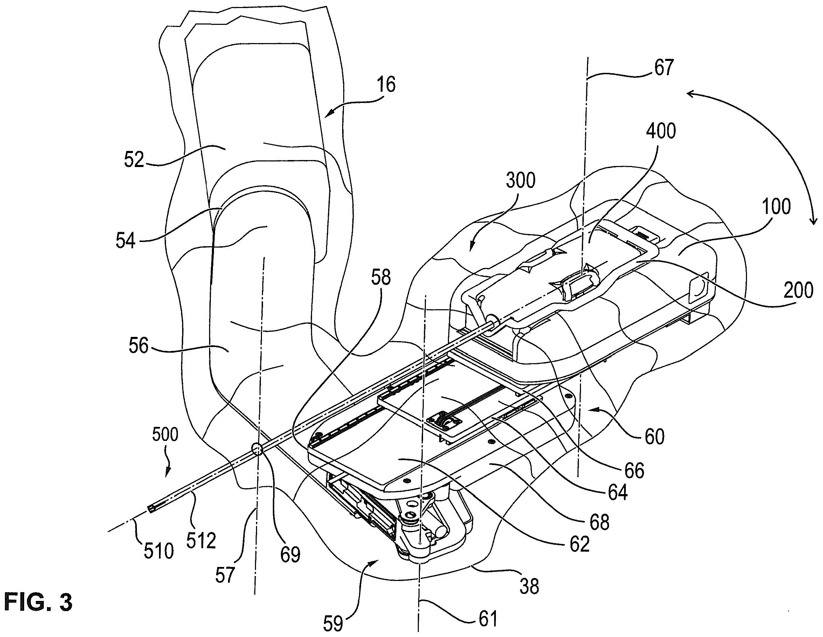

Bei einer Vielzahl von Operationen müssen die Instrumenteneinheiten 300a bis 300d während der Operation aufgrund des Operationsverlaufs mehrfach gewechselt werden. Somit muss zwischen dem Manipulatorarm 16a bis 16d und der Instrumenteneinheit 300a bis 300d eine sterile Schnittstelle vorgesehen werden, die sicherstellt, dass die nicht sterilen Teile der Koppeleinheit des Manipulatorarms 16a bis 16d auch nach dem Trennen der Instrumenteneinheit 300a bis 300d steril abgedeckt sind. Darüber hinaus müssen durch einen Kontakt der sterilen Elemente der Koppeleinheit des Manipulatorarms 16a bis 16d kontaminierte Elemente der Instrumenteneinheit 300a bis 300d nach dem Trennen der Instrumenteneinheit 300a bis 300d vom Manipulatorarm 16a bis 16d steril abgedeckt werden, damit die Instrumenteneinheit 300a bis 300d im sterilen Bereich 39 abgelegt werden kann, ohne weitere Elemente im sterilen Bereich 39 zu kontaminieren. Erfindungsgemäß wird eine Sterilschleuse zwischen der Koppeleinheit des Manipulatorarms 16a bis 16d und der Instrumenteneinheit 300a bis 300d vorgesehen, die mindestens eine Schleusenklappe hat, die geschlossen ist, wenn keine Instrumenteneinheit 300a bis 300d mit der Sterilschleuse verbunden ist, sodass dann die nicht sterile Koppeleinheit vom sterilen Bereich 39 mit Hilfe der flexiblen sterilen Hülle 38 und der in diese integrierten Sterilschleuse vom sterilen Bereich 39 abgeschirmt ist. Der Aufbau und die Funktion der Sterilschleuse werden im Nachfolgenden in Verbindung mit den Figuren 3 bis 42 noch näher erläutert.

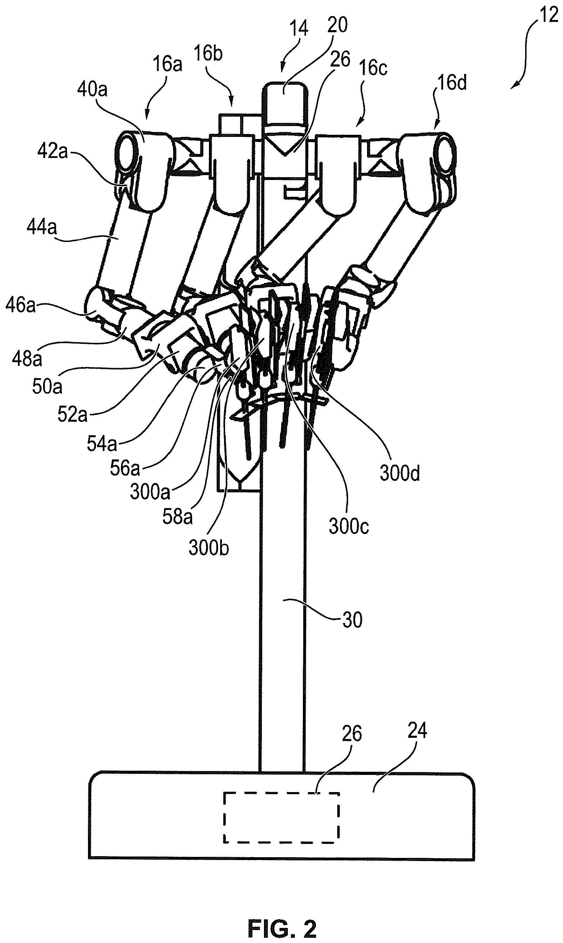

In Figur 2 ist eine Frontansicht des Manipulators 12 nach Figur 1 gezeigt. Die Manipulatorarme 16a bis 16d des Manipulators 12 haben jeweils mehrere Segmente 40a bis 58a, die mit Hilfe integrierter Antriebseinheiten relativ zueinander bewegbar sind, so dass die Instrumenteneinheiten 300a bis 300d ohne Kollision exakt positioniert werden können. Die sterilen Hüllen 38 zum Abschirmen eines Abschnitts der Manipulatorarme 16a bis 16d sind in Figur 2 nicht dargestellt. Die Segmente des Manipulatorarms 16a sind mit den Bezugszeichen 40a bis 58a bezeichnet. Die weiteren Manipulatorarme 16b bis 16d haben den gleichen Aufbau und haben die in Figur 2 aus Übersichtlichkeitsgründen nicht bezeichneten Segmente 40b bis 58b, 40c bis 58c und 40d bis 58d. Dieselben Elemente der Manipulatorarme 16a bis 16d sind mit derselben Bezugszeichenziffer und mit dem zusätzlichen Buchstaben zur Unterscheidung der Manipulatorarme 16a bis 16d bezeichnet. Die in der nachfolgenden Beschreibung gemachten Ausführungen beziehen sich auf den Manipulatorarm 16a und die Instrumenteneinheit 300a, die im Folgenden mit Manipulatorarm 16 und Instrumenteneinheit 300 bezeichnet sind. Die Segmente 40a bis 58a des Manipulatorarms 16a sind im Folgenden als Segmente 40 bis 58 bezeichnet. Die Ausführungen gelten jedoch in gleicher Weise für die gleich aufgebauten Manipulatorarme 16b bis 16d und die Instrumenteneinheiten 300b bis 300d. Elemente mit gleichem Aufbau und/oder gleicher Funktion sind mit denselben Bezugszeichen bezeichnet.

Figur 3 zeigt eine perspektivische Darstellung eines Abschnitts des Manipulatorarms 16 mit einer Koppeleinheit 100 zum Koppeln des Manipulatorarms 16 mit der eine Sterileinheit 400 umfassenden Instrumenteneinheit 300. Hierzu ist die Koppeleinheit 100 mit einer in die sterile Hülle 38 integrierten Sterilschleuse 200 verbunden. Die Sterilschleuse 200 ist sowohl mit der Koppeleinheit 100 als auch mit der Sterileinheit 400 koppelbar und wieder trennbar. In Figur 3 ist die Sterilschleuse 200 sowohl mit der Koppeleinheit 100 als auch mit der Sterileinheit 400 gekoppelt dargestellt. Die Koppeleinheit 100 ist am distalen Ende der Teleskopanordnung 60 angeordnet.

Die Teleskopanordnung 60 hat zueinander verschiebbare Abschnitte 62, 64, 66 und ist in Figur 3 in einem ausgefahrenen Zustand dargestellt. Die Abschnitte 62, 64, 66 der Teleskopanordnung 60 können mit Hilfe einer Antriebseinheit 68 eingefahren und ausgefahren werden, sodass ein chirurgisches Instrument 500 der Instrumenteneinheit 300 entlang der Längsachse 510 des Instrumentenschafts 512 zusammen mit der Koppeleinheit 100, der Sterilschleuse 200 und der Sterileinheit 400 bewegt werden kann. Mit Hilfe einer in das Segment 52 integrierten Antriebseinheit kann das Segment 54 zusammen mit dem als Gelenkarm ausgeführten Segment 56 um die Drehachse 57 gedreht werden. Das Segment 58 ist über ein Koppelgetriebe 59 mit dem Segment 56 verbunden, sodass das Segment 58 nach Aktivierung einer mit dem Koppelgetriebe 59 verbundenen Antriebseinheit um die Drehachse 61 geschwenkt werden kann. Ferner ist die Koppeleinheit 100 über ein in Figur 3 nicht sichtbares Koppelgetriebe gegenüber dem Segment 66 um die Drehachse 67 drehbar angeordnet. Auch dieses Koppelgetriebe ist über eine mit diesem Koppelgetriebe verbundenen Antriebseinheit antreibbar, sodass bei Aktivierung dieser Antriebseinheit die Koppeleinheit 100 um die Drehachse 67 gedreht wird. Dabei werden die Antriebseinheiten der Koppelgetriebe derart angesteuert, dass die Längsachse 510 des Instrumentenschafts 512 bei einer Bewegung des Manipulatorarms 16 und dessen Segmente um einen im Raum ortsfesten Pivot-Punkt 69 geschwenkt werden, durch den die Längsachse 510 des bei einer Operation vorzugsweise durch einen Trokar in einen Patienten eingeführten Instrumentenschaft 512 wird dann um den Pivot-Punkt 69 gedreht, sodass sichergestellt ist, dass durch eine Bewegung des Instruments 500 nur eine geringe Beanspruchung des Patienten an der Eintrittsstelle des Instruments 500 in dem Patienten erfolgt und insbesondere eine Verletzung des Patienten an der Eintrittsstelle des Instrumentenschafts 512 verhindert wird.

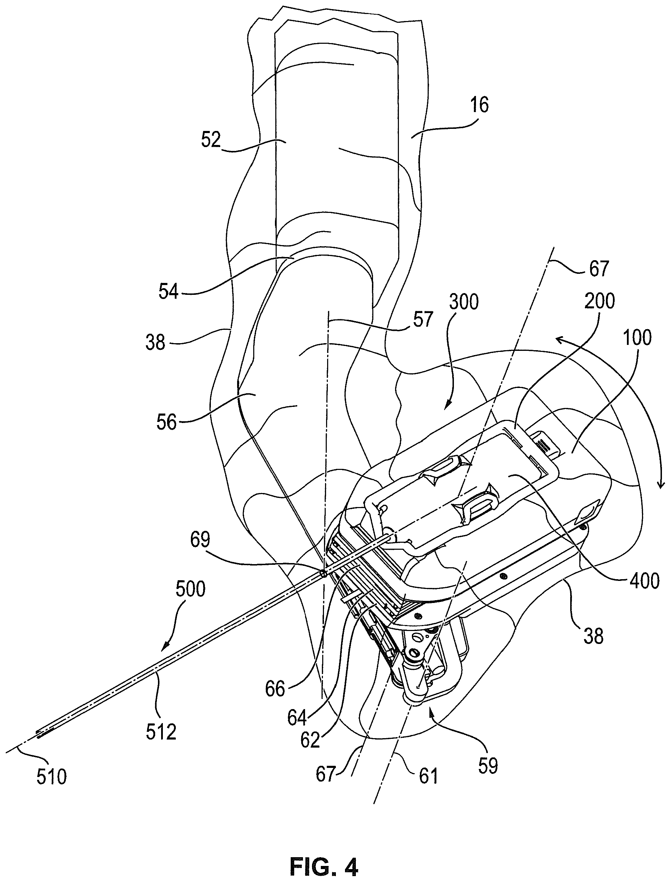

In Figur 4 ist eine weitere perspektivische Darstellung der Anordnung nach Figur 3 gezeigt, wobei die Abschnitte 62, 64, 66 der Teleskopanordnung 60 im Unterschied zu Figur 3 in einem eingefahrenen Zustand dargestellt sind, wodurch die Instrumenteneinheit 300 in Richtung der Längsachse 510 des Instrumentenschafts 512 zum proximalen Ende des chirurgischen Instruments 500 hin verschoben worden ist. Somit ist durch das Einfahren der Teleskopanordnung 60 die Instrumenteneinheit 300 in Richtung des proximalen Endes des Instruments 500 entlang der Längsachse 510 des Instruments 500 verschoben worden. Dabei ist jedoch die Lage des Pivot-Punkts 69 unverändert geblieben. Auch bei einer Drehung der Segmente 56, 58, 60 um die Drehachse 57 wird der Pivot-Punkt 69 durch eine entsprechende Ansteuerung der Antriebseinheiten der Koppelgetriebe 59 in seiner Raumposition unverändert gelassen, in dem eine entsprechende Drehung des Segments 60 um die Drehachse 61 und der Koppeleinheit 100 um die Drehachse 67 erfolgt. Ferner verläuft eine durch einen entsprechenden Antrieb der Koppelgetriebe erzeugte virtuelle Drehachse (nicht dargestellt), die parallel zu den Drehachsen 61, 67 und orthogonal zu der Drehachse 57 verläuft, durch den Pivot-Punkt 69.

In dem Pivot-Punkt 69 schneiden sich die Drehachse 57 des als Gelenkarm ausgeführten Segments 56 und die Längsachse 510 des Instruments 500. Der Pivot-Punkt 69 wird auch als Pivotalpunkt bezeichnet.

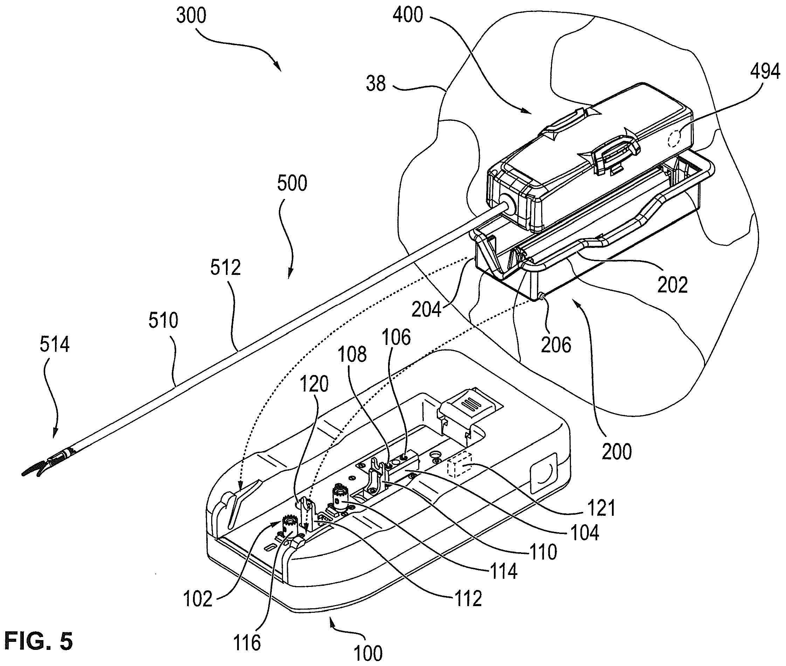

Figur 5 zeigt die Koppeleinheit 100, die Sterilschleuse 200 sowie die Instrumenteneinheit 300 mit der Sterileinheit 400 und dem chirurgischen Instrument 500, das einen Endeffektor 514 hat, vor dem Zusammenfügen der Sterilschleuse 200 mit der Koppeleinheit 100 und vor dem nachfolgenden Zusammenfügen der Sterileinheit 400 mit der Sterilschleuse 200. Die als Sterilfolie ausgeführte flexible sterile Hülle 38 ist an einem umlaufenden Anschlussrand 202 der Sterilschleuse 200 mit dieser über eine geeignete Verbindung, wie eine Klemm-, Klebe- und/oder Schweißverbindung, fest verbunden, so dass die Sterilfolie 38 zusammen mit der Sterilschleuse 200 eine geschlossene sterile Abdeckung um die vom sterilen Bereich 39 abzuschirmenden nicht sterilen Elemente 16, 100 bildet, wie dies auch in den Figuren 1, 3 und 4 gezeigt ist. Zur besseren Darstellung ist in Figur 5 nur ein Ausschnitt der Sterilfolie 38 um die Sterilschleuse 200 herum dargestellt. In den nachfolgenden Figuren ist die Sterilfolie 38 teilweise nicht dargestellt.

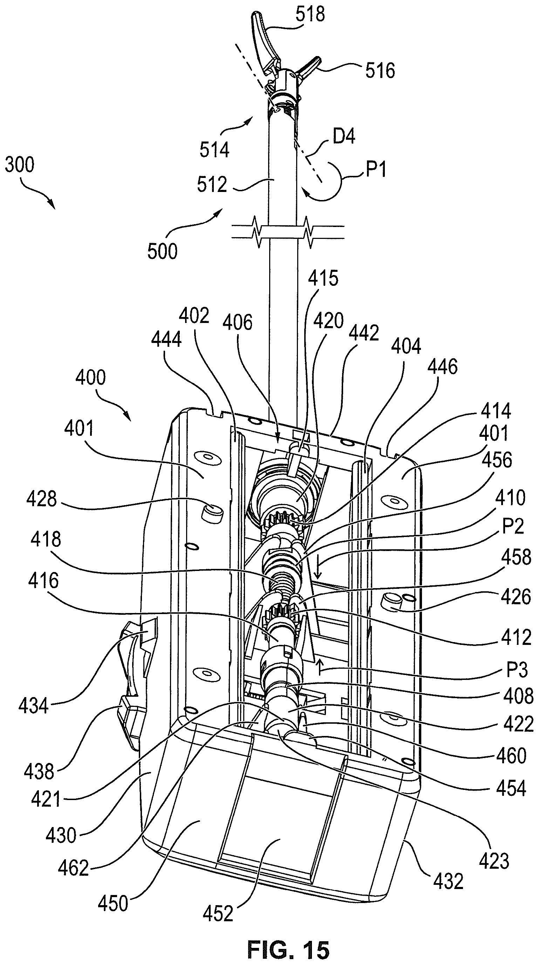

Zur Kopplung der Sterileinheit 400 mit der Koppeleinheit 100 ist die Sterilschleuse 200 zwischen der Sterileinheit 400 und er Koppeleinheit 100 angeordnet und ermöglicht im gekoppelten Zustand der Sterileinheit 400 mit der Koppeleinheit 100 eine direkte Kopplung eines ersten Übertragungsmittels 102 der Koppeleinheit 100 und eines zweiten Übertragungsmittels der Sterileinheit 400. Das zweite Übertragungsmittel ist in Figur 15 mit dem Bezugszeichen 406 bezeichnet.

Mit Hilfe des ersten Übertragungsmittels 102 wird im vorliegenden Ausführungsbeispiel sowohl mechanische Energie als auch elektrische Energie zwischen der Koppeleinheit 100 und der Sterileinheit 400 übertragen. Hierzu hat das erste Übertragungsmittel 102 der Koppeleinheit 100 mindestens vier mechanische Antriebselemente 110 bis 116 und das zweite Übertragungsmittel 406 der Sterileinheit 400 vier zu den Antriebselementen 110 bis 116 in Figur 15 dargestellte komplementäre angetriebene Elemente 412 bis 418. Weiterhin hat das erste Übertragungsmittel 102 ein elektrisches Übertragungselement 104 mit zwei elektrischen Kontakten 106, 108 und das zweite Übertragungsmittel 406 ein zum elektrischen Übertragungselement 104 des ersten Übertragungsmittels 102 komplementäres elektrisches Übertragungselement. Das komplementäre elektrische Übertragungselement umfasst zwei in Figur 15 dargestellte elektrische Kontakte 422, 423.

Bei anderen Ausführungsbeispielen können die ersten und zweiten Übertragungsmittel auch mehr oder weniger Antriebselemente, angetrieben Elemente und elektrische Übertragungselemente umfassen, die mechanische und/oder elektrische Energie durch direkte Kopplung übertragen. Als direkte Kopplung wird dabei eine Kopplung der Übertragungsmittel angesehen, bei der keine weiteren Übertragungselemente zwischen den ersten Übertragungsmitteln und den zweiten Übertragungsmitteln für eine Übertragung von mechanischer und/oder elektrischer Energie und/oder optischen Strahlen vorgesehen sind, wobei insbesondere keine elektrischen, mechanischen oder optischen Übertragungselemente in einer zwischen der Koppeleinheit 100 und der Sterileinheit 400 angeordneten Sterilbarriere, wie der Sterilschleuse 200, vorgesehen sind. Die Koppeleinheit 100 hat ferner eine RFID-Lese-und-Schreibeinheit 121, mit deren Hilfe ein RFID-Transponder 494 der Sterileinheit 400 lesbar und/oder schreibbar ist.

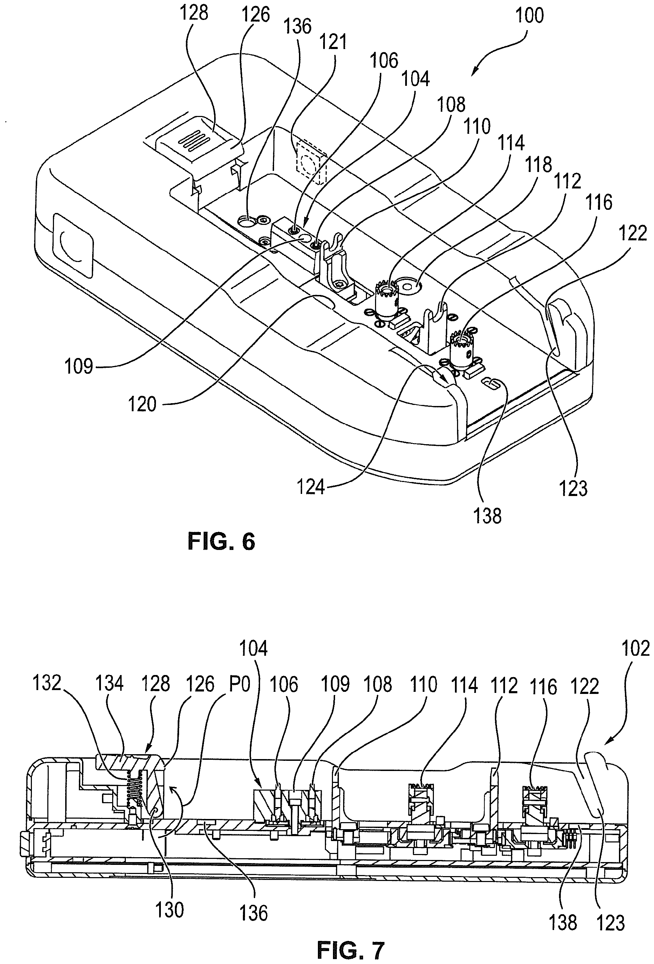

Figur 6 zeigt eine schematische perspektivische Darstellung der Koppeleinheit 100 des Manipulatorarms 16. Das erste Übertragungsmittel 102 der Koppeleinheit 100 hat ein elektrisches Übertragungselement 104 mit zwei elektrischen Kontakten 106, 108, ein optisches Übertragungsmittel 109 zur Übertragung von Licht und/oder optischen Signalen, ein erstes translatorisches Antriebselement 110 und ein zweites translatorisches Antriebselement 112 jeweils zur Übertragung einer translatorischen Bewegung sowie ein erstes rotatorisches Antriebselement 114 und ein zweites rotatorisches Antriebselement 116 zur Übertragung einer Rotationsbewegung. Das erste und das zweite translatorische Antriebselement 110, 112 sind jeweils als Linearhubgabel und das erste und das zweite rotatorische Antriebselement 114, 116 sind als Antriebsritzel mit stirnseitiger Zahnung ausgebildet. Ferner hat die Koppeleinheit 100 einen in einer Vertiefung angeordneten ersten Koppelsensor 118, der ein durch einen aus der Sterileinheit 400 vorstehenden ersten Detektionsstift gebildetes erstes Detektionselement detektiert, wenn die Sterilschleuse 200 korrekt mit der Koppeleinheit 100 und die Sterileinheit 400 korrekt mit der Sterilschleuse 200 gekoppelt ist. In diesem Fall ragt ein erster Detektionsstift der Sterileinheit 400 in die Vertiefung, in der der erste Koppelsensor 118 angeordnet ist, so dass dieser das Vorhandensein des als erstes Detektionselement dienenden ersten Detektionsstifts detektiert. Der erste Detektionsstift ist in Figur 15 gezeigt und dort mit dem Bezugszeichen 426 bezeichnet.

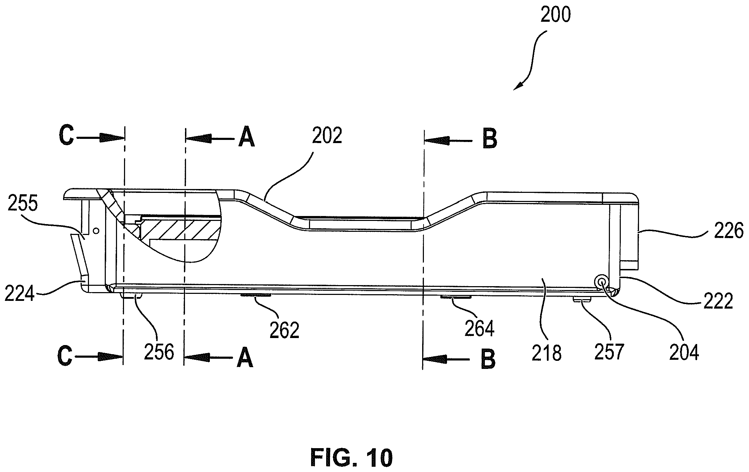

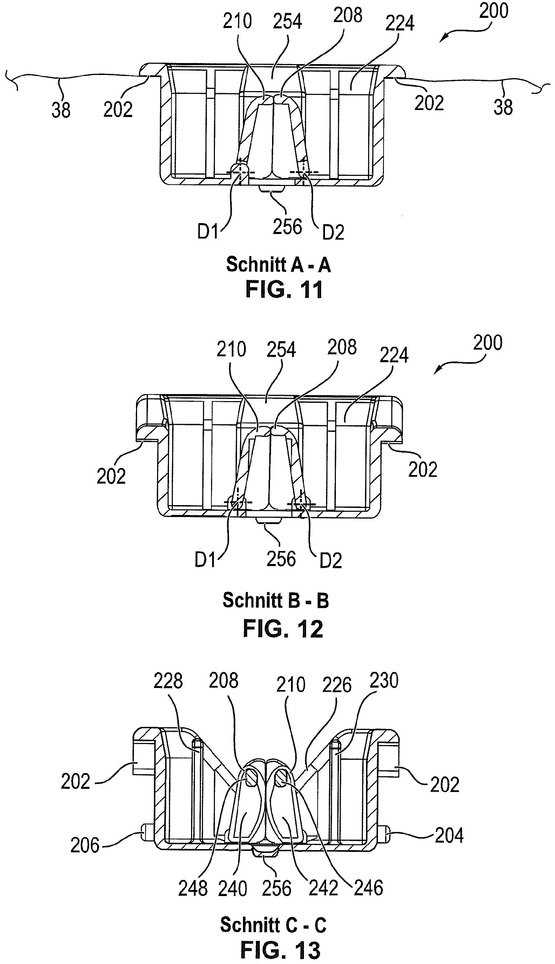

Die Koppeleinheit 100 hat einen zweiten Koppelsensor 120, der seitlich neben den Antriebselementen 112, 114 in einer weiteren Vertiefung angeordnet ist, wie dies in Figur 5 deutlicher zu sehen ist. Der zweite Koppelsensor 120 detektiert ein durch einen zweiten Detektionsstift der Sterileinheit 400 gebildetes zweites Detektionselement, wenn sowohl die Koppeleinheit 100 korrekt mit der Sterilschleuse 200 als auch die Sterilschleuse 200 korrekt mit der Sterileinheit 400 gekoppelt sind. Der zweite Detektionsstift ist in Figur 11 gezeigt und dort mit dem Bezugszeichen 428 bezeichnet. Somit wird mit Hilfe der Koppelsensoren 118, 120 sicher festgestellt, ob die Sterileinheit 400 korrekt mit der Koppeleinheit 100 gekoppelt ist, so dass eine direkte Übertragung zwischen dem ersten Übertragungsmittel 102 der Koppeleinheit 100 und dem zweiten Übertragungsmittel der Sterileinheit 400 möglich ist. Zum Verbinden der Koppeleinheit 100 mit der Sterilschleuse 200 hat die Koppeleinheit 100 einander gegenüberliegende Führungsnuten 122, 124, in die Führungsstifte 204, 206 der Sterilschleuse 200 eingeführt werden, bis sie das vordere Ende 123, 125 der jeweiligen Führungsnut 122, 124 erreicht haben, wie dies in Figur 10 gezeigt ist. Die Führungsstifte 204, 206 ragen an einem ersten Ende der Sterilschleuse 200 auf gegenüberliegenden Seiten nach außen, wie dies in den Figuren 5 und 10 zu sehen ist. Anschließend wird das gegenüberliegende zweite Ende der Sterilschleuse 200 nach unten gedrückt, so dass die Sterilschleuse 200 um eine durch die Führungsstifte 204, 206 verlaufende Drehachse gedreht wird, bis eine Rastnase 126 eines Rastelements 128 in einen komplementären Rastbereich der Sterilschleuse 200 eingreift.

Figur 7 zeigt einen Längsschnitt durch die Koppeleinheit 100. Die Entriegelungstaste 128 ist um eine Drehachse 130 schwenkbar angeordnet und wird durch eine Feder 132 in ihrer in Figur 7 gezeigten Rastposition gehalten. Zum Lösen der Rastverbindung wird mit einem Finger auf eine Entriegelungstaste 134 des Rastelements 128 gedrückt, so dass eine Feder 132 gespannt und das Rastelement 128 zusammen mit der Rastnase 126 in Richtung des Pfeils P0 gedreht wird, so dass die Rastnase 126 außer Eingriff mit dem komplementären Rastelement der Sterilschleuse 200 gebracht wird. Dadurch kann das zuvor mit der Rastnase 126 in Eingriff stehende zweite Ende der Sterilschleuse 200 aus der Koppeleinheit 100 herausgeschwenkt werden. Nachdem dieses zweite Ende der Sterilschleuse 200 aus der Koppeleinheit 100 herausgeschwenkt worden ist, kann die Sterilschleuse 200 vollständig von der Koppeleinheit 100 getrennt werden, indem die Sterilschleuse 200 mit den mit den Führungsnuten 122, 124 in Eingriff stehenden Führungsstiften 204, 206 entlang der Führungsnuten 122, 124 aus diesem heraus gezogen wird, bis die Führungselemente 204, 206 nicht mehr in Eingriff mit den Führungsnuten 122, 124 stehen. Zwischen den Führungsnuten 122, 124 und dem Rastelement 128 ist ein durch eine entsprechende Vertiefung im Gehäuse der Koppeleinheit 100 gebildeter Aufnahmebereich vorhanden, der im vorliegenden Ausführungsbeispiel die Sterilschleuse 200 auf drei Seiten und bodenseitig zumindest teilweise umgibt.

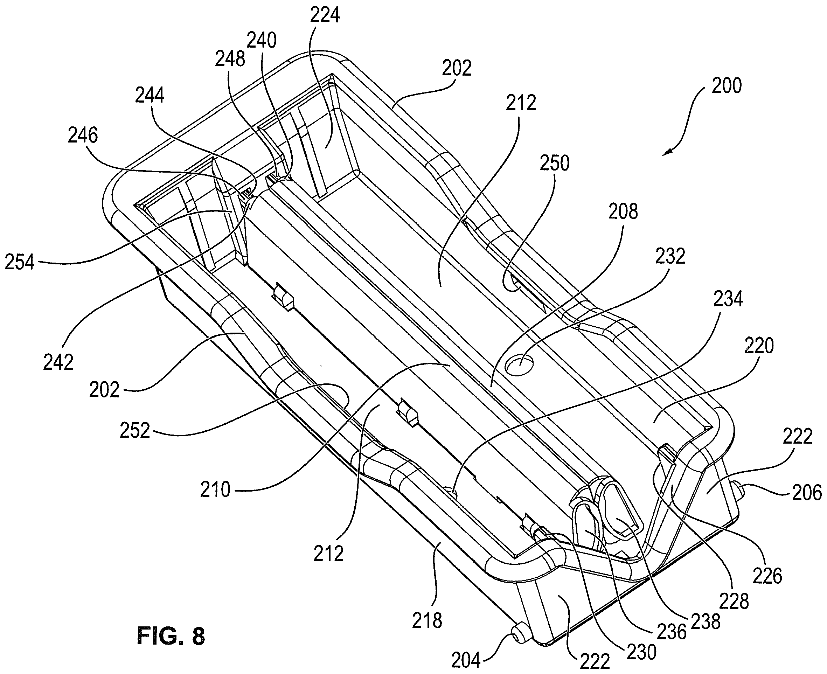

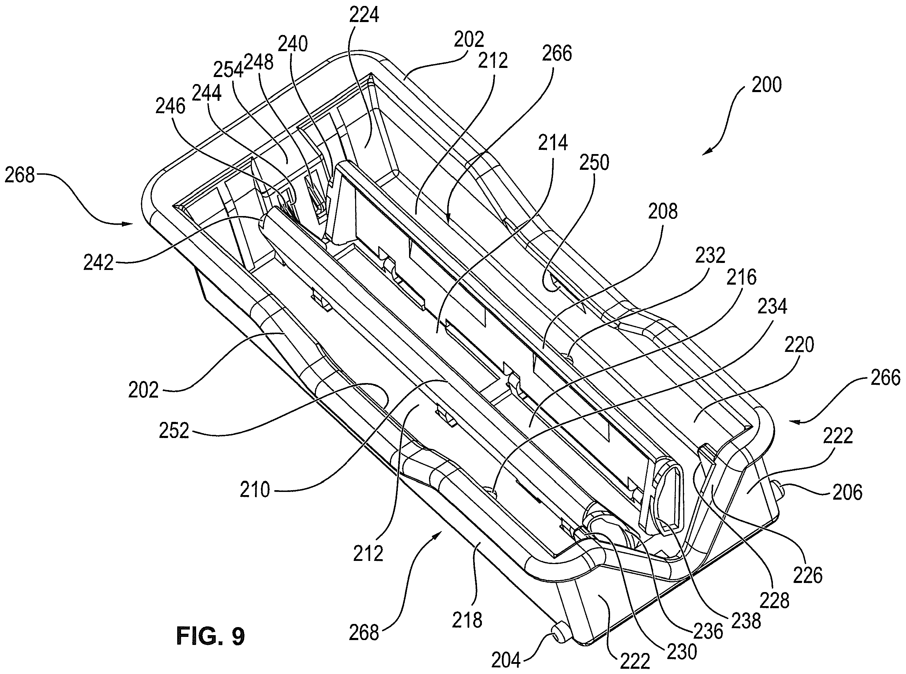

Figur 8 zeigt eine perspektivische Ansicht der Sterilschleuse 200 mit geschlossenen Schleusenklappen 208, 210. Figur 9 zeigt eine perspektivische Ansicht der Sterilschleuse 200 mit geöffneten Schleusenklappen 208, 210. Die Sterilschleuse 200 hat einen Boden 212, in dem zwei mit Hilfe der Schleusenklappen 208, 210 abdeckbare Öffnungen 214, 216 vorgesehen sind. Die Schleusenklappen 208, 210 sind über Scharniere schwenkbar mit dem Boden 212 verbunden. Mit Hilfe dieser Scharniere sind die Schleusenklappen 208, 210 von dem in Figur 8 gezeigten geschlossenen Zustand in den in Figur 9 gezeigten geöffneten Zustand schwenkbar. Im geöffneten Zustand der Schleusenklappen 208, 210 kann eine direkte Kopplung des ersten Übertragungsmittels 102 der Koppeleinheit 100 mit dem zweiten Übertragungsmittel der Sterileinheit 400 erfolgen.

Die Sterilschleuse 200 hat ferner zwei Seitenwände 218, 220, eine vordere Stirnwand 222 und eine hintere Stirnwand 224. An den Außenseiten der Seitenwände 218, 220 und den Stirnwänden 222, 224 ist der umlaufende Rand 202 ausgebildet, mit dem, wie bereits in Verbindung mit Figur 5 beschrieben, die Sterilfolie der sterilen Abdeckung 38 auf geeignete Art und Weise verbunden ist.

An der Innenseite der vorderen Stirnwand 222 sind jeweils seitlich neben einer V-förmigen Aussparung 226 der Stirnwand 222 zwei Führungs- und Entriegelungsstege 228, 230 fest angeordnet, die bei einer Verbindung der Sterilschleuse 200 mit der Sterileinheit 400 als Entriegelungselemente zum Entriegeln von Sterilklappen der Sterileinheit 400 dienen, wie nachfolgend noch ausführlich beschrieben wird.

Im Boden 212 der Sterilschleuse 200 sind ein erstes Detektionsfenster 232 und ein zweites Detektionsfenster 234 in Form jeweils eines Durchgangslochs vorhanden, durch die die bereits erwähnten Detektionselemente 426, 428 der Sterileinheit 400 hindurchgeführt werden, so dass sie vom ersten Koppelsensor 118 und vom zweiten Koppelsensor 120 der Koppeleinheit 100 detektiert werden können.

Am vorderen und hinteren Ende der Schleusenlappen 208, 210 ist jeweils eine Führungssicke 236 bis 242 vorgesehen. Die vorderen Führungssicken 236, 238 sind ohne Funktion. In die hinteren Führungssicken 240, 242 greifen im geschlossenen Zustand der Schleusenkappen 208, 210 die Zinken 246, 248 einer Führungsgabel 244 ein. Die Führungsgabel 244 wird mit Hilfe einer Feder in ihre obere in Figur 8 gezeigte Position gedrückt und schließt durch den Eingriff ihrer Zinken 246, 248 in die Führungssicken 240, 242 die Schleusenklappen 208, 210 und hält diese in ihrer geschlossenen Position. Die Schleusenklappen 208, 210 können durch den Eingriff der Gabelzinken 246, 248 nicht auseinandergedrückt werden, so dass das nicht sterile Übertragungsmittel 102 der Koppeleinheit 100 bei geschlossenen Schleusenklappen 208, 210 sicher abgedeckt ist und die nicht sterilen Elemente der Koppeleinheit 100 sicher vom sterilen Bereich 39 abgeschirmt sind.

Die Schleusenklappen 208, 210 sind baugleich, so dass zur beidseitigen Verwendung an beiden Stirnseiten der Schleusenklappen 208, 210 jeweils eine Führungssicke 236 bis 242 vorgesehen ist. Bei anderen Ausführungsformen können die Schleusenklappen 208, 210 auch unterschiedlich ausgebildet sein und nur auf einer Seite eine Führungssicke 240, 242 haben, in die die Zinken 246, 248 der Führungsgabel 244 eingreifen.

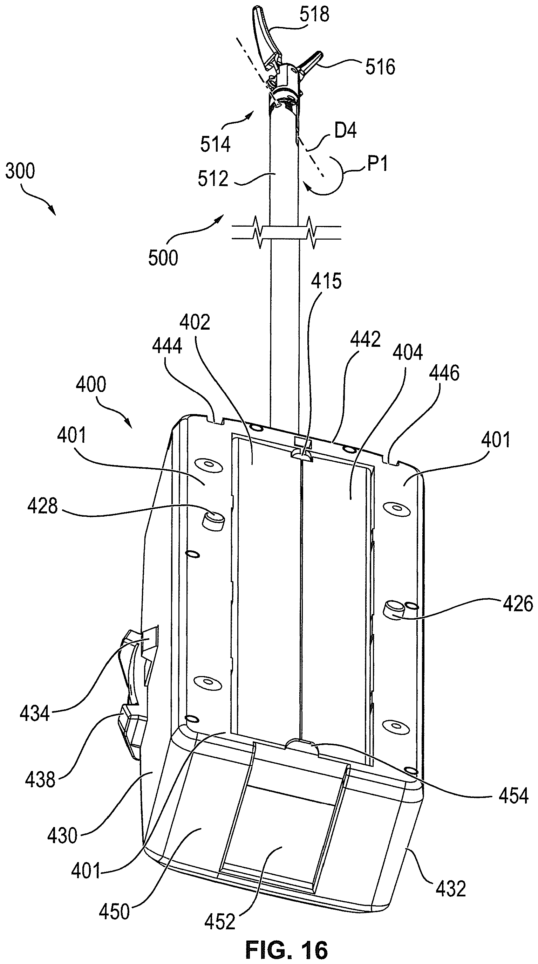

In den Seitenwänden 218, 220 ist jeweils eine Rastsicke 250, 252 vorgesehen, in die ein Rastelement der Sterileinheit 400 bei einer Verbindung der Sterilschleuse 200 mit der Sterileinheit 400 eingreift. An der hinteren Stirnwand 224 der Sterilschleuse 200 ist ein Führungssteg 254 vorgesehen, der bei einer Verbindung der Sterilschleuse 200 mit der Sterileinheit 400 in eine Führungsnut 452 der Sterileinheit 400 eingreift, wie dies in Figur 16 gezeigt ist.

Figur 10 zeigt eine teilgeschnittene Seitenansicht der Sterilschleuse 200. An der Außenseite der hinteren Stirnwand 224 der Sterilschleuse 200 ist eine Rastnase 255 ausgebildet, in die die Rastnase 126 des Rastelements 128 der Koppeleinheit 100 eingreift, wenn die Koppeleinheit 100 mit der Sterilschleuse 200 verbunden ist.

Zur korrekten Positionierung der Sterilschleuse 200 im Aufnahmebereich der Koppeleinheit 100 sind zwei unten aus dem Boden stehende Positionierungselemente 256, 257 vorgesehen, die in entsprechende Öffnungen 136, 138 im Boden des Aufnahmebereichs der Koppeleinheit 100 eingreifen. Die Positionierungselemente 256, 257 sind angefast oder alternativ konisch, damit sie einfach in die in Figur 7 gezeigten Öffnungen 136, 138 der Koppeleinheit 100 eingeführt werden können.

Die Detektionsfenster 232 und 234 sind jeweils mit einer Folie 262, 264 abgedeckt, die die Detektionselemente 426, 428 der Sterileinheit 400 auch noch dann steril abschirmt, wenn diese durch die Detektionsfenster 232, 234 bis in die Vertiefungen der Sensoren 118, 120 der Koppeleinheit 100 ragen. Dabei wird die Folie 262, 264 elastisch und/oder plastisch verformt und reißt nicht.

Figur 11 zeigt eine Schnittdarstellung der Sterilschleuse 200 nach Figur 10 entlang der Schnittlinie A-A. In dieser Figur sind die Drehachse, um die die Schleusenklappe 110 vom geschlossenen in den geöffneten Zustand und vice versa geschwenkt wird, mit D1 und die Drehachse, um die die Schleusenklappe 208 geschwenkt wird, mit D2 bezeichnet.

Figur 12 zeigt eine Schnittdarstellung der Sterilschleuse 200 nach Figur 10 entlang der Schnittlinie B-B und Figur 13 eine Schnittdarstellung der Sterilschleuse 200 nach Figur 10 entlang der Schnittlinie C-C. Wie aus den Figuren 8 bis 13 ersichtlich ist, bilden die Seitenwände 218, 220, die Stirnwände 222, 224 und der Boden 218 eine Gehäusewanne, in die die Sterileinheit 400 zum Verbinden der Sterileinheit 400 mit der Koppeleinheit 100 zumindest teilweise einsetzbar ist. Die Gehäusewanne dient somit allgemein als erster Verbindungsbereich 266 der Sterilschleuse 200. Die Außenseite der Sterilschleuse 200 dient als zweiter Verbindungsbereich 268, mit dem die Sterilschleuse 200 mit der Koppeleinheit 100 verbindbar ist.

Wie in Figur 13 ersichtlich, greifen die vorderen Enden der Zinken 246, 248 der Führungsgabel 244 in die Führungssicken 240, 242 ein. Die einander zugewandten Seitenwände der Führungssicken 240, 242 bilden zusammen mit den vorderen Enden der Zinken 246, 248 der Führungsgabel 244 eine Kulissenführung, durch die die Schleusenklappen 208, 210 geschlossen werden, wenn die vorderen Enden der Zinken 246, 248 der Gabel 244 nach oben geschwenkt werden.

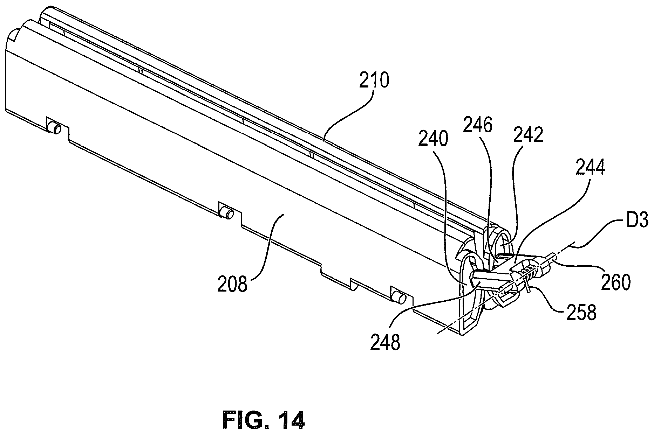

Figur 14 zeigt eine Detailansicht mit teilweise geöffneten Schleusenklappen 208, 210 und der mit den Führungssicken 240, 242 in Eingriff stehenden Zinken 246, 248 der Führungsgabel 244. Die Führungsgabel 244 wird mit Hilfe einer Führungsgabelfeder 258 um eine durch einen in der hinteren Stirnwand 224 gelagerten Führungsstift 260 gebildete Drehachse D3 in der in den Figuren 8 bis 14 gezeigten liegenden Darstellung der Sterilschleuse 200 nach oben geschwenkt, so dass die Schleusenklappen 208, 210 mit Hilfe der Federkraft der Führungsgabelfeder 258 geschlossen und in einer geschlossenen Position gehalten werden. Der Führungsstift 260 dient zur Führung und Lagerung der Feder 258 sowie zur Lagerung der Führungsgabel 244. Beim Einsetzen der Sterileinheit 400 in die Sterilschleuse 200 wird dann die Führungsgabel 244 entgegen der Federkraft der Führungsgabelfeder 258 nach unten geschwenkt, so dass die Sterilklappen 208, 210 durch an der Sterileinheit 400 vorgesehene Eingriffselemente vom geschlossenen Zustand in den geöffneten Zustand geschwenkt werden.

Figur 15 zeigt eine perspektivische Darstellung der Instrumenteneinheit 300 mit der Sterileinheit 400 und dem chirurgischen Instrument 500. Am proximalen Ende des drehbaren äußeren Instrumentenschafts 512 ist der abwinkelbare und drehbare Endeffektor 5124 mit betätigbaren Greifarmen 516, 518 angeordnet. Die Bewegungen des Endeffektors 514 sind mit Hilfe der Antriebselemente 110 bis 116 der Koppeleinheit 100 und der angetriebenen Elemente 408 bis 414 der Sterileinheit 400 ausführbar, wenn die Sterileinheit 400 über die Sterilschleuse 200 mit der Koppeleinheit 100 verbunden ist. Die Sterileinheit 400 hat Sterilklappen 402, 404, die in Figur 15 in einem geöffneten und in Figur 16 in einem geschlossenen Zustand gezeigt sind. Im Inneren der Sterileinheit 400 ist das zweite Übertragungsmittel angeordnet, das bei geöffneten Sterilklappen 402, 404 sichtbar und mit dem Bezugszeichen 406 bezeichnet ist. Das zweite Übertragungsmittel 406 umfasst ein bei einer Kopplung mit der Koppeleinheit mit dem ersten translatorischen Antriebselement 110 in Eingriff stehendes erstes translatorisches angetriebenes Element 408 und ein mit dem zweiten translatorischen Antriebselement 112 der Koppeleinheit 100 in Eingriff stehendes zweites translatorisches angetriebenes Element 410 jeweils zur Übertragung einer Translation. Ferner sind ein mit dem ersten rotatorischen Antriebselement 114 der Koppeleinheit 100 koppelbares erstes rotatorisches angetriebenes Element 412 sowie ein mit dem zweiten rotatorischen Antriebselement 116 der Koppeleinheit 100 in Eingriff stehendes zweites rotatorisches angetriebenes Element 414 jeweils zur Übertragung einer Rotationsbewegung vorgesehen. Bei dem mit der Koppeleinheit 400 verbundenen chirurgischen Instrument 500 wird der Endeffektor 514 um die Kippachse D4 in Pfeilrichtung P1 um bis zu 90° geschwenkt, wenn das zweite translatorische angetriebene Element 410 der Sterileinheit 400 von dem zweiten translatorischen Antriebselement 112 der Koppeleinheit 100 in Richtung des Pfeils P2 bewegt wird. Bei einer Bewegung des ersten translatorisch angetriebenen Elements 408 in Richtung des Pfeils P3 werden die Greifarme 516, 518 des Endeffektors 514 auseinanderbewegt und in entgegengesetzte Richtungen aufeinander zu bewegt. Beim Antrieb des ersten rotatorisch angetriebenen Elements 412 der Sterileinheit 400 mit Hilfe des ersten rotatorischen Antriebselements 114 der Koppeleinheit 100 kann der Endeffektor 514 unabhängig vom Instrumentenschaft 512 gedreht werden. Mit Hilfe des zweiten rotatorisch angetriebenen Elements 414 kann bei einer Kopplung und Antrieb mit dem zweiten rotatorischen Antriebselement 116 der Koppeleinheit 100 eine Rotation des Instrumentenschafts 512 um seine Längsachse 510 erfolgen, um die Lage der Kippachse D4 des Endeffektors 514 um die Drehachse 510 des äußeren Instrumentenschafts 512 zu drehen, ohne dass der Endeffektor 514 selbst mitgedreht wird.

Ferner ist eine erste Feder 416 vorgesehen, die das erste translatorische angetriebene Element 408 der Sterileinheit 400 entgegen der Pfeilrichtung des Pfeils P3 in seine Endlage drückt. Ferner ist eine zweite Feder 418 vorgesehen, die das zweite translatorisch angetriebene Element 410 der Sterileinheit 400 entgegen der Pfeilrichtung des Pfeils P2 in seine Endlage drückt. Ferner hat die Sterileinheit 400 ein Lager 420 zum drehbaren Lagern des äußeren Instrumentenschafts 512 in der Sterileinheit 400. Alternativ zum chirurgischen Instrument 500 lassen sich mit der Sterileinheit 400 auch andere Instrumente, wie eine Schere, Nadelhalter, optische Instrumente, Spüleinheiten, Absaugeinheiten, Instrumente der Hochfrequenzchirurgie und weitere bei Operationen, insbesondere bei laparoskopischen Operationen, eingesetzte Instrumente, koppeln, wobei die zweiten Übertragungsmittel 406 für die Realisierung der entsprechenden Funktionen ausgelegt sind.

Das zweite Übertragungsmittel 406 umfasst gemäß dem Ausführungsbeispiel weiterhin ein elektrisches Übertragungselement mit einem ersten als Schleifring ausgebildeten elektrischen Kontakt 422 und einen zweiten als Schleifring ausgebildeten elektrischen Kontakt 423, die bei einer Kopplung der Sterileinheit 400 mit der Koppeleinheit 100 über die Sterilschleuse 200 mit den elektrischen Kontakten 106, 108 der Koppeleinheit 100 eine elektrische Verbindung zur Übertragung von hochfrequenter elektrischer Energie zur Hochfrequenzchirurgie herstellen. Bei anderen Ausführungsbeispielen können auch keine elektrischen Übertragungsmittel vorgesehen sein.

Die Sterileinheit 400 hat zwei vorstehende Nocken 415, 417, die beim Einsetzen der Sterileinheit 400 in die Sterilschleuse 200 in die entriegelten Sterilklappen 208, 210 zumindest so weit auseinander drücken, bis die Nocken 415, 417 zwischen den Sterilklappen 208, 210 angeordnet sind. Beim weiteren Einsetzen der Sterileinheit 400 in die Sterilschleuse 200 drücken keilförmige Eingriffselemente 456 bis 462 der Sterileinheit 400 die Sterilklappen 208, 210 weiter auseinander, bis diese in ihrer in Figur 9 gezeigten geöffneten Position angeordnet sind.

Die in den Figuren 15 und 16
nach oben zeigende Bodenplatte 401 der Sterileinheit 400 hat, wie bereits erwähnt, zwei als vorstehende Detektionsstifte ausgebildete Detektionselemente 426, 428. Bei einer Kopplung der Sterileinheit 400 mit der Koppeleinheit 100 mit zwischen der Strileinheit400 und der Koppeleinheit 100 angeordneter Sterilschleuse 200 ragt das Detektionselement 426 durch das erste Detektionsfenster 232 der Sterilschleuse 200 in die Vertiefung des ersten Koppelsensors 118 der Koppeleinheit 100 und das zweite Detektionselement 428 ragt durch das zweite Detektionsfenster 234 in die Vertiefung des zweiten Koppelsensors 120 der Koppeleinheit 100. Bei einer Detektion der Detektionselemente 426, 428 mit Hilfe der Koppelsensoren 118, 120 kann eine korrekte Kopplung der Sterilschleuse 200 mit der Koppeleinheit 100 und der Sterileinheit 400 mit der Sterilschleuse 200 detektiert werden, so dass erst nach einer Detektion der Detektionselemente 426, 428 mit Hilfe der Koppelsensoren 118, 120 ein Antrieb der Übertragungselemente 110 bis 116 von einer Steuereinheit freigegeben wird. Ferner wird die Übertragung von hochfrequenter Energie erst nach der korrekten Detektion der Detektionselemente 426, 428 mit Hilfe der Koppelsensoren 118, 120 über die Übertragungselemente 106, 108 freigegeben.

Die Sterileinheit 400 hat ferner zwei an gegenüberliegenden Seitenwänden 430, 432 angeordnete Rastelemente 434, 436, die mit Hilfe eines aus der Seitenwand 430, 432 vorstehenden Betätigungselements 438, 440 betätigbar sind. Die Rastelemente 434, 436 greifen in die in den Seitenwänden 218, 220 der Sterilschleuse 200 vorgesehenen Rastsicken 250, 252 ein, wenn die Sterileinheit 400 korrekt mit der Sterilschleuse 200 verbunden ist.

Die vordere Stirnwand 442 der Sterileinheit 400 hat zwei Nuten 444, 446, in die die Führungs- und Entriegelungsstege 228, 230 der Sterilschleuse 200 bei einer Verbindung der Sterileinheit 400 mit der Sterilschleuse 200 eingeführt werden und dabei, wie nachfolgend noch detailliert ausgeführt wird, die Sterilklappen 402, 404 entriegeln.

Ferner greift der Führungssteg 254 der Sterilschleuse 200 in die an der hinteren Stirnseite 450 der Sterileinheit 400 vorhandene Führungsnut 452 ein. Am unteren Ende der Führungsnut 452 steht ein Betätigungssteg 454 nach außen aus der Bodenplatte 401 hervor, der beim Einsetzen der Sterileinheit 400 in die Sterilschleuse 200 die Führungsgabel 244 nach unten drückt und dadurch die Verriegelung der Schleusenklappen 208, 210 durch die Führungsgabel 244 löst.

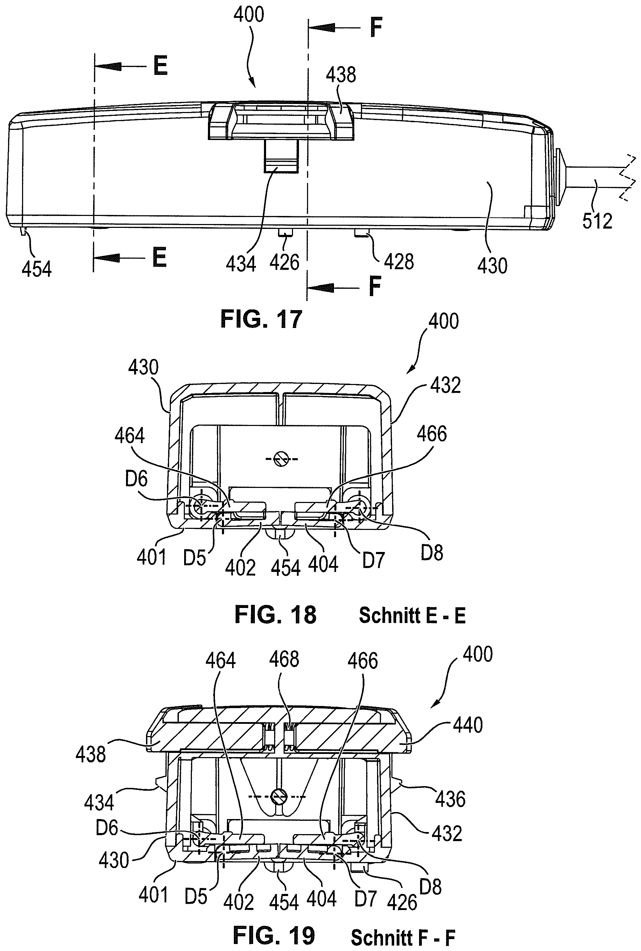

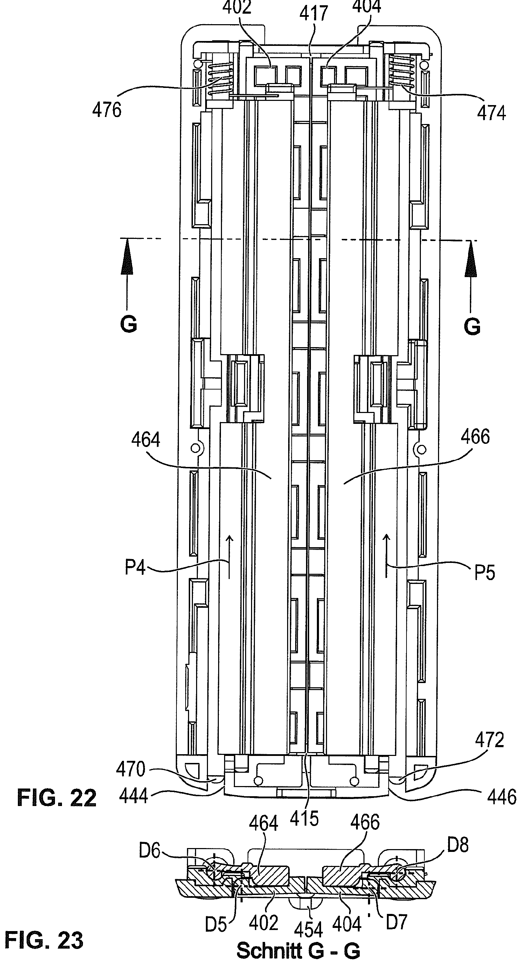

Figur 17 zeigt eine Seitenansicht der Sterileinheit 400 mit einem Teil des Instrumentenschafts 512 des chirurgischen Instruments 500. Figur 18 zeigt einen Schnitt der Sterileinheit nach Figur 17 entlang der Schnittlinie E-E. Wie aus dieser Schnittdarstellung ersichtlich ist, steht die Sterilklappe 402 mit einer in der Sterileinheit 400 vorhandenen Führungsklappe 464 in Eingriff. Die Sterilklappe 404 steht mit einer im Inneren der Sterileinheit 400 angeordneten Führungsklappe 464 in Eingriff. Zum Öffnen der Sterilklappe 402 ist diese um die Drehachse D5 und die Führungsklappe 464 um die Drehachse D6 schwenkbar angeordnet. Zum Öffnen der Sterilklappe 404 ist diese um die Drehachse D7 und die Führungsklappe 464 um die Drehachse D8 verschwenkbar angeordnet. Im geschlossenen Zustand werden die Sterilklappen 402, 404 mit Hilfe der Führungsklappen 464, 466 verriegelt und zum Öffnen der Sterilklappen 402, 404 entriegelt, wie nachfolgend in Verbindung mit den Figuren 22 bis 28 noch näher erläutert wird.

In Figur 19 ist ein Schnitt der Sterileinheit 400 nach Figur 17 entlang der Schnittlinie F-F gezeigt. In Figur 19 ist eine Feder 468 sichtbar, mit deren Hilfe die Betätigungselemente 438, 440 und mit diesen die Rastnasen 434, 436 nach außen gedrückt werden, so dass die Rastnasen 434, 436 in die Rastsicken 250, 252 der Sterilschleuse 200 gedrückt werden, wenn die Sterileinheit 400 korrekt in die Sterilschleuse 200 eingesetzt worden ist.

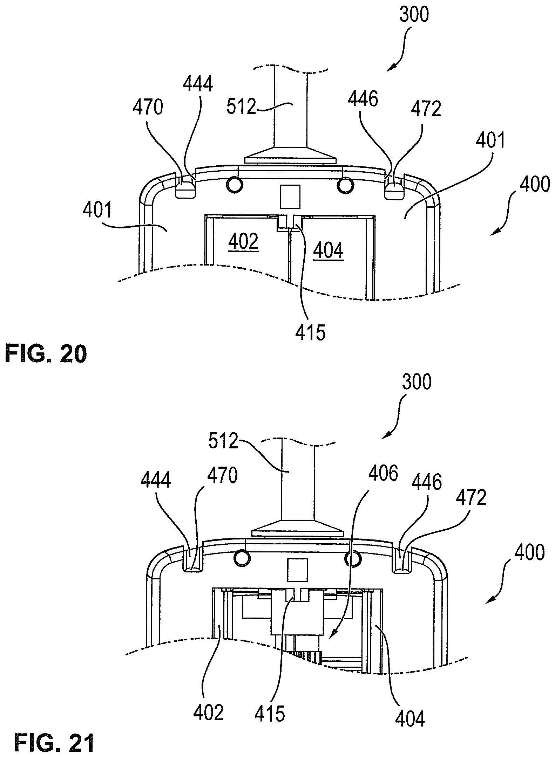

Figur 20 zeigt einen Ausschnitt einer Ansicht der Instrumenteneinheit 300 von unten mit geschlossenen Sterilklappen 402, 404 der Sterileinheit 400. In dieser Darstellung sind ein Entriegelungsstift 470 der Führungsklappe 464 und ein Entriegelungsstift 472 der Führungsklappe 466 sichtbar. Der Entriegelungsstift 470 ragt in die Führungs-und Entriegelungsnut 444 und der Entriegelungsstift 472 ragt in die Entriegelungs- und Führungsnut 446. Beim Verbinden der Sterileinheit 400 mit der Sterilschleuse 200 werden die Führungs- und Entriegelungsstege 228, 230 der Sterilschleuse 200 in die Führungsnuten 444, 446 eingeführt und drücken die Entriegelungsstifte 470, 472 ausgehend von der in Figur 20 gezeigten verriegelten Position in die in Figur 21 gezeigte entriegelte Position. In Figur 21 sind die Sterilklappen 202, 204 und die Führungsklappen 464, 466 entriegelt und geöffnet dargestellt.

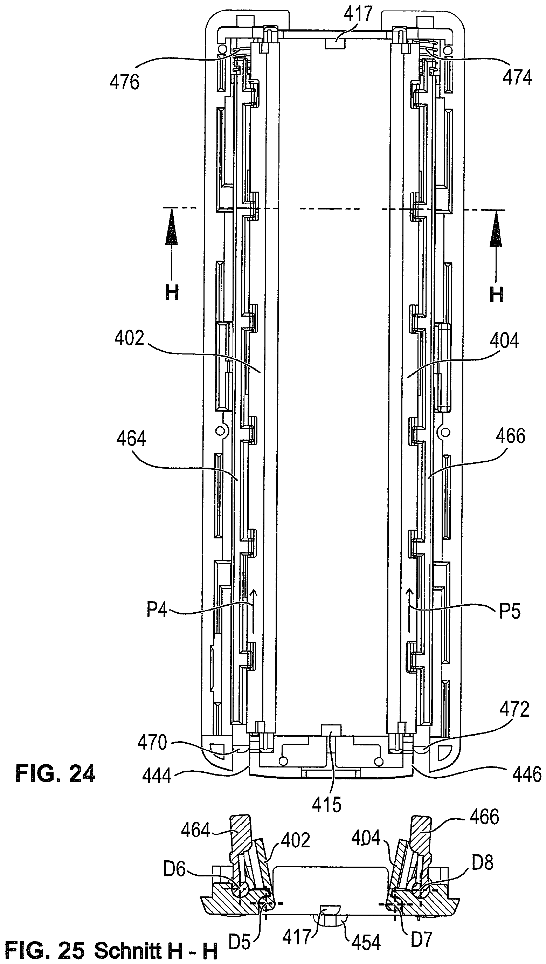

Figur 22 zeigt eine Draufsicht auf das Sterilklappensystem der Sterileinheit 400 mit geschlossenen Sterilklappen 402, 404 und geschlossenen und verriegelten Führungsklappen 464, 466. Das Sterilklappensystem hat neben den Sterilklappen 402, 404 und den Führungsklappen 464, 466 eine Feder 474, die vorgespannt ist und die Führungsklappe 466 und die mit der Führungsklappe 466 in Eingriff stehende Sterilklappe 404 in dem in Figur 22 gezeigten geschlossenen Zustand hält. Nach der Entriegelung der Führungsklappe 466 können die Führungsklappe 466 und die Sterilklappe 404 entgegen der Federkraft der Feder 474 geöffnet werden. Das Sterilklappensystem hat ferner eine Feder 476, die vorgespannt ist und die Führungsklappe 464 und die Sterilklappe 402 in ihrem geschlossenen Zustand hält. Die Führungsklappe 464 und die Sterilklappe 402 können entgegen der Federkraft der Feder 476 geöffnet werden, wenn die Führungsklappe 464 entriegelt worden ist. Zum Entriegeln der Führungsklappen 464, 466 werden die Entriegelungsstifte 470, 472 durch die Führungs- und Entriegelungsstege 228, 230 in Richtung der Pfeile P4, P5 innerhalb ihres geringen Spiels bis zu der in Figur 24 dargestellten Position bewegt. Dabei werden die Sterilklappen 402, 404 nicht oder nur unwesentlich in Richtung der Pfeile P4, P5 verschoben, so dass zum Entriegeln eine Relativbewegung der Führungsklappen 464, 466 zu den Sterilklappen 402, 404 in Richtung der Pfeile P4, P5 erfolgt. Figur 23 ist eine Schnittdarstellung des Sterilklappensystems nach Figur 22 entlang der Schnittlinie G-G mit geschlossenen Führungsklappen 464, 466 und geschlossenen Sterilklappen 402, 404.

In Figur 24 sind die Führungsklappen 464, 466 und die Sterilklappen 402, 404 in ihren geöffneten Positionen dargestellt. Figur 25 ist eine Schnittdarstellung des Sterilklappensystems nach Figur 24 entlang der Schnittlinie H-H mit geöffneten Führungsklappen 464, 466 und geöffneten Sterilklappen 402, 404.

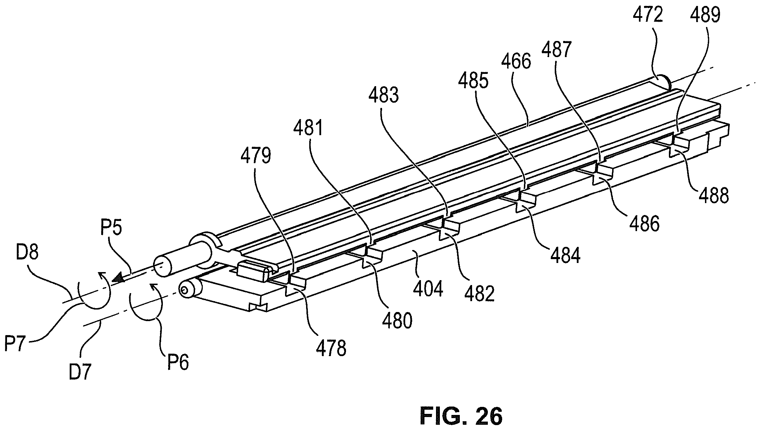

Figur 26 zeigt eine perspektivische Detailansicht der Sterilklappe 404 und der mit dieser in Eingriff stehenden Sterilklappe 404 in einer geschlossenen verriegelten Position. Zusätzlich drückt die in Figur 26 nicht dargestellte Feder 474 die Führungsklappe 466 entgegen des Pfeils P5 in deren verriegelte Position und die Feder 474 drückt die Führungsklappe 466 zusätzlich entgegen der Richtung des des Pfeils P4 in deren verriegelte Position, so dass zum Entriegeln der Führungsklappen 464, 466 die Entriegelungsstifte 470, 472 gegen die Federkraft der Federn 474, 476 in Richtung der Pfeile P4, P5 bewegt werden.

Die Sterilklappe 404 hat auf der der Führungsklappe 466 zugewandten Seite sieben Führungs- und Verriegelungsnuten 478 bis 488, in die jeweils ein Führungs- und Verriegelungssteg 479 bis 489 der Führungsklappe 466 eingreift. Die Führungs- und Verriegelungsstege 479 bis 489 sind auf der der Sterilklappe 404 zugewandten Seite der Führungsklappe 466 angeordnet. Die Sterilklappe 404 und die Führungsklappe 466 sind in Figur 26 in ihrer verriegelten Position dargestellt. In diesem verriegelten Zustand sind die Führungs- und Verriegelungsstege 479 bis 489 jeweils hinter einem Vorsprung der Führungs- und Verriegelungsnuten 478 bis 488 angeordnet, so dass dadurch eine Drehung der Sterilklappe 404 und der Führungsklappe 466 um die Drehachsen D5, D6 in Richtung der Pfeile P6 und P7 verhindert wird. Zum Entriegeln der Führungsklappe 466 wird diese mit Hilfe des Entriegelungsstifts 472 in Richtung des Pfeils P5 bewegt, so dass die Führungs-und Verriegelungsstege 479 bis 489 aus den Vorsprüngen der Führungs- und Verriegelungsnuten 478 bis 488 herausbewegt werden, so dass die Führungsklappe 466 und die Sterilklappe 404 um die Drehachsen D7, D8 in Richtung der Pfeile P6 und P7 bewegt werden können.

Die Sterilklappenanordnung der Sterilklappe 402 und der Führungsklappe 464 sind spiegelsymmetrisch zu der in Figur 26 gezeigten Klappenanordnung der Sterilklappe 404 und der Führungsklappe 466, so dass deren Ver- und Entriegelung in gleicher Weise wie für die Sterilklappe 404 und die Führungsklappe 466 beschrieben erfolgt.

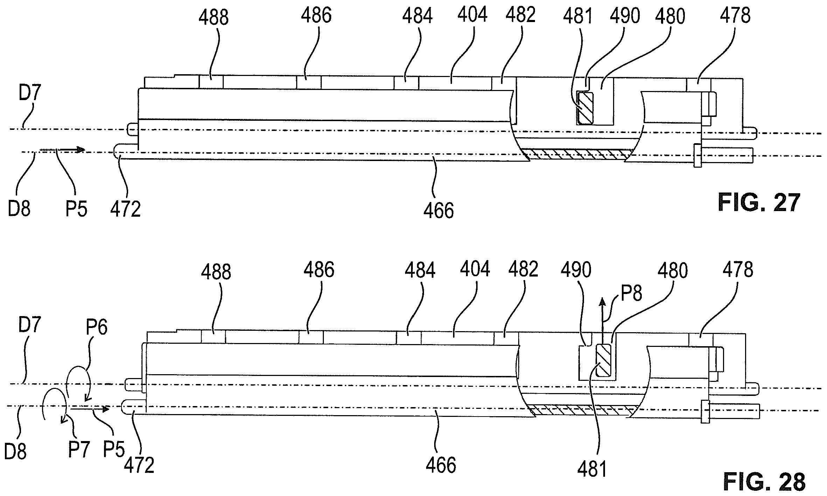

Figur 27 zeigt eine Draufsicht auf die Führungsklappe 466 und die Schleusenklappe 404 in einer teilgeschnittenen Darstellung im geschlossenen und verriegelten Zustand. In dem geschnittenen Bereich ist die Führungs- und Verriegelungsnut 480 der Führungsklappe 466 sichtbar. Die Führungs- und Verriegelungsnut 480 ist dabei hinter einem als Sperrnase dienenden Vorsprung 490 der Führungs- und Verriegelungsnut 480 angeordnet, so dass bei einem auf die Führungsklappe 466 und/oder auf die Sterilklappe 404 wirkendem Moment für eine Drehbewegung der Führungsklappe 466 in Richtung des Pfeils P7 oder der Sterilklappe 404 in Richtung des Pfeils P6 das Führungs- und Verriegelungselement 480 gegen die Sperrnase 490 gedrückt werden würde. Dadurch ist weder eine Drehung der Führungsklappe 466 um die Drehachse D8 in Richtung des Pfeils P7 noch eine Drehung der Sterilklappe 404 um die Drehachse D7 in Richtung des Pfeils P6 möglich ist. Erst nachdem die Führungsklappe 466 in Richtung des Pfeils P5 verschoben worden ist, wie dies in Figur 28 dargestellt ist, sind die Führungsklappe 466 um die Drehachse D8 in Richtung des Pfeils P7 und die Sterilklappe 404 um die Drehachse D7 in Richtung des Pfeils P6 drehbar. Weiterhin wird dabei die Führungsklappe 466 in Pfeilrichtung P8 relativ zur Sterilklappe 404 bewegt. Durch die Verschiebung der Führungsklappe 466 in Richtung des Pfeils P5 ist die Führungsklappe 466 relativ zur Sterilklappe 404 verschoben worden, so dass der Führungs- und Verriegelungssteg 481 nicht mehr hinter dem Vorsprung 490, sondern im Bereich der nach außen offenen Führungsnut 480 angeordnet ist. Dadurch kann die Sterilklappe 404 in Richtung des Pfeils P6 und die Führungsklappe 466 in Richtung des Pfeils P7 gedreht werden, so dass die Sterilklappe 404 und die Führungsklappe 466 jeweils in ihre geöffnete Position bewegt werden können. Die weiteren Führungs- und Verriegelungsstege 479 bis 489 werden zusammen mit dem Führungs-und Verriegelungssteg 481 aus dem durch den Vorsprung gebildeten Sperrbereich der jeweiligen Führungs-und Verriegelungsnut 478 bis 488 bewegt, wie dies für den Führungs- und Verriegelungssteg 481 und die Führungs- und Verriegelungsnut 480 erläutert worden ist.

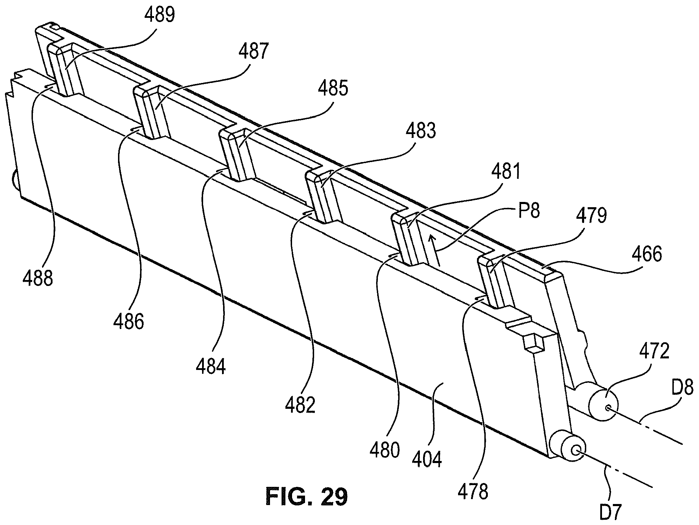

In Figur 29 sind die Führungsklappe 466 und die Sterilklappe 404 in ihrer geöffneten Position dargestellt, in der die Führungs- und Verriegelungsstege 479 bis 489 durch die Relativbewegung in Pfeilrichtung P8 teilweise aus den Führungs- und Verriegelungsnuten 478 bis 488 herausragen.

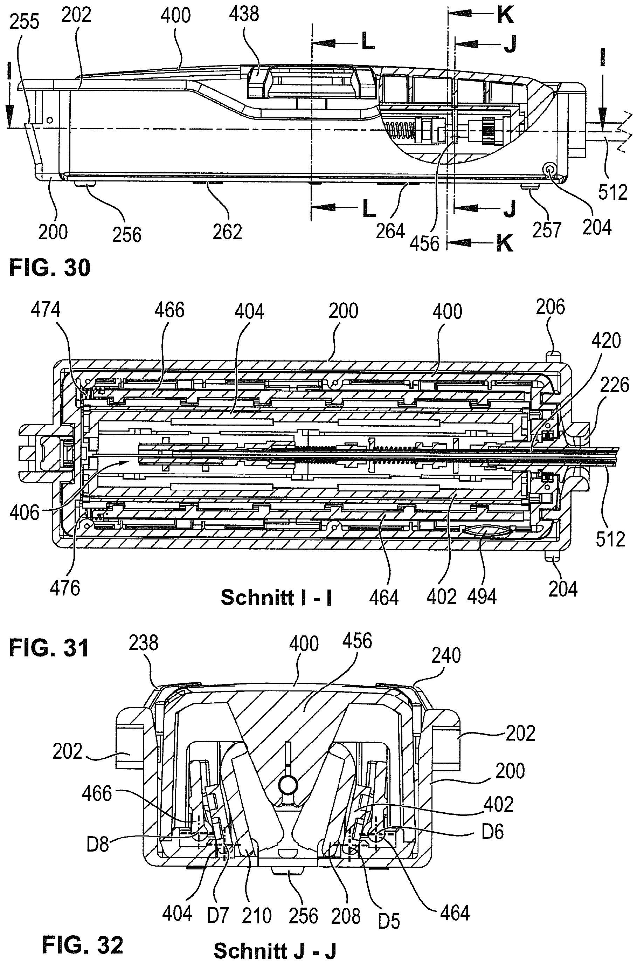

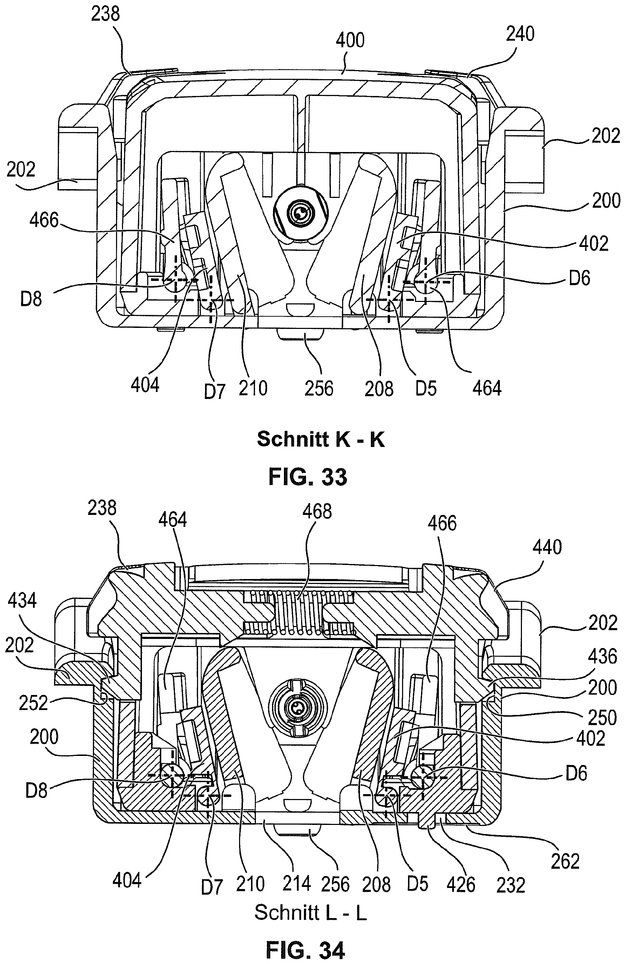

Figur 30 zeigt eine teilgeschnittene Darstellung einer Anordnung mit einer Sterilschleuse 200 und einer mit der Sterilschleuse 200 verbundenen Sterileinheit 400. Figur 31 zeigt eine Schnittdarstellung der Anordnung nach Figur 30 entlang der Schnittlinie I-I, Figur 32 eine Schnittdarstellung der Anordnung nach Figur 30 entlang der Schnittlinie J-J, Figur 33 eine Schnittdarstellung der Anordnung nach Figur 30 entlang der Schnittlinie K-K und Figur 34 eine Schnittdarstellung der Anordnung nach Figur 30 entlang der Schnittlinie L-L. Wie in Figur 31 zu sehen ist, hat die Sterileinheit 400 einen RFID-Transponder 494, in dem vorzugsweise zumindest eine eindeutige Kennung der Sterileinheit 400 und vorzugsweise Informationen über das mit der Sterileinheit 400 gekoppelte chirurgische Instrument 500 gespeichert sind. Ferner können weitere Informationen, wie Herstellerinformationen der Sterileinheit 400, Chargeninformationen, Haltbarkeitsinformationen, vorzugsweise in einem nicht überschreibbaren Bereich des RFID-Transponders 494, gespeichert sein. In einem vorzugsweise nur einmal beschreibbaren Speicherbereich des RFID-Transponders 494 können Informationen über die Erstverwendung der Sterileinheit 400, insbesondere der Zeitpunkt der Erstverwendung und/oder eine eindeutige Operationskennung der Operation, bei der die Erstverwendung der Sterileinheit 400 stattgefunden hat, gespeichert werden, so dass durch diese Information ausgeschlossen werden kann, dass die Sterileinheit 400 bei einer weiteren Operation, insbesondere bei einem weiteren Patienten, eingesetzt wird. Die Kennung des RFID-Transponders 494 und/oder die im RFID-Transponder 494 gespeicherten Informationen können mit Hilfe der RFID-Lese- und Schreibeeinheit 121 der Koppeleinheit 100 gelesen und erforderlichenfalls Informationen in Form von Daten im RFID-Transponder 494 gespeichert werden.

Figur 32 zeigt, dass bei einer Verbindung der Sterileinheit 400 mit der Sterilschleuse 200 sowohl die Schleusenklappen 208, 210 als auch die Führungsklappen 464, 466 und die Sterilklappen 402, 404 geöffnet sind, so dass das zweite Übertragungsmittel 406 der Sterileinheit 400 durch die Klappen 208, 210, 402, 404, 464, 466 hindurch mit dem ersten Übertragungsmittel 102 in direktem Kontakt gebracht werden kann. Insbesondere kann sowohl eine direkte elektrische Verbindung zwischen den elektrischen Kontaktelementen 106, 108 der Koppeleinheit 100 und den elektrischen Kontaktelementen 422, 423 der Sterileinheit 400 hergestellt werden als auch ein direkter Eingriff der mechanischen Übertragungselemente 110 bis 116 der Koppeleinheit 100 mit den Übertragungselementen 408 bis 414 der Sterileinheit 400 erfolgen. Somit werden keine zusätzlichen elektrischen und/oder mechanischen Übertragungselemente zwischen der Koppeleinheit 100 und der Sterileinheit 400 benötigt. Dies ist sowohl kostengünstiger als auch weniger störanfällig als das Vorsehen von zusätzlichen Übertragungselementen, insbesondere mechanischen Übertragungselementen, zwischen der Koppeleinheit 100 und der Sterileinheit 400, wie sie beispielsweise im Stand der Technik eingesetzt werden.

Wie dies in Figur 32 gut zu sehen ist, sind die Sterilklappen 208, 210 mit Hilfe der V-förmigen Elemente 456 bis 462 bis in ihre in Figur 32 gezeigte Lage auseinandergedrückt worden, nachdem sie mit Hilfe der Nocken 415, 417 bereits so weit auseinander gedrückt worden sind, dass die V-förmigen Elemente 456 bis 462 in den dadurch erzeugten Öffnungsspalt eingreifen und die Schleusenklappen 208, 210 sowie die Sterilklappen 402, 404 zusammen mit den Führungsklappen 464, 466 jeweils in ihre geöffnete Position drücken.

Im geöffneten Zustand sind die sterile Außenseite der Sterilklappe 402 der sterilen Außenseite der Schleusenklappe 208 gegenüberliegend angeordnet. In gleicher Weise ist die sterile Außenseite der Sterilklappe 404 der sterilen Außenseite der Schleusenklappe 210 gegenüberliegend angeordnet, so dass auch bei einem direkten Kontakt der Sterilklappe 402 mit der Schleusenklappe 208 bzw. bei einem direkten Kontakt der Sterilklappe 404 mit der Schleusenklappe 210 weder eine Kontamination der Schleusenklappen 208, 210 noch eine Kontamination der Sterilklappen 402, 404 erfolgt.

Somit kommen nur sterile nicht kontaminierte Bereiche der Sterilschleuse 200 mit sterilen nicht kontaminierten Bereichen der Sterileinheit 400 in Kontakt, so dass nach einer Trennung der Sterileinheit 400 von der Sterilschleuse 200 keine Gefahr einer Kontamination des sterilen Bereichs 39 besteht.

In Figur 34 ist gezeigt, wie die Rastelemente 434, 436 der Sterileinheit 400 in die Rastsicken 250, 252 der Sterilschleuse 200 greifen, wenn die Sterileinheit 400 korrekt mit der Sterilschleuse 200 verbunden ist. Des Weiteren ist in Figur 34 zu sehen, wie das Detektionselement 426 durch das erste Detektionsfenster 232 hindurch ragt, wobei das Detektionselement 426 bei einer Verbindung der Sterilschleuse 200 mit der Koppeleinheit 100 in die Vertiefung des ersten Sensors 118 der Koppeleinheit 100 ragt, so dass dieser das Detektionselement 426 detektiert. Ferner ist in Figur 34 die Folie 162 dargestellt, die durch das Detektionselement 426 elastisch und/oder plastisch verformt wird und das Detektionselement 426 dadurch steril abdeckt.

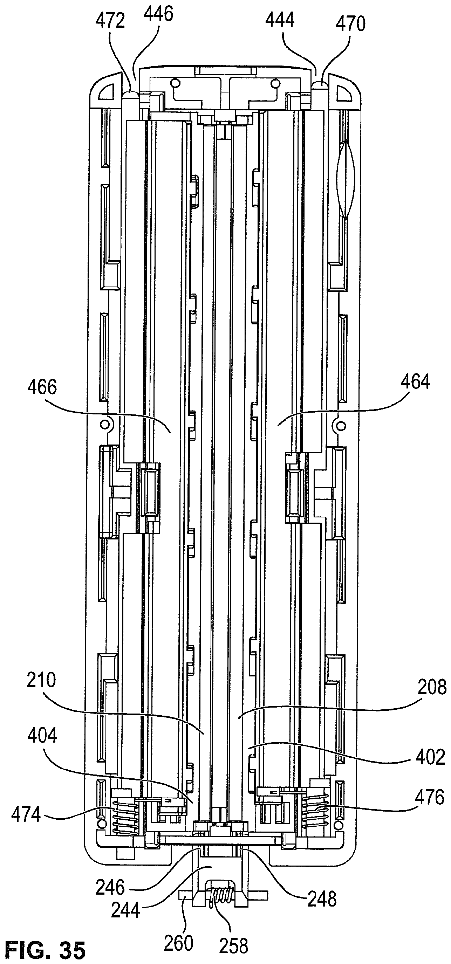

Figur 35 zeigt Elemente des Sterilklappensystems mit den Sterilklappen 402, 404 und den Führungsklappen 464, 466 und Elemente des Schleusenklappensystems der Sterilschleuse 200 mit den Schleusenklappen 208, 210. Wie bereits erläutert, greifen die Zinken 246, 248 der Führungsgabel 244 in die Führungssicken 240, 242 im geschlossenen Zustand der Schleusenklappen 208, 210 ein, so dass über diesen Eingriff eine formschlüssige Verbindung vorhanden ist, die ein Aufdrücken der Schleusenklappen 208, 210 verhindert. In der in Figur 35 gezeigten Position der Führungsgabel 244 stehen deren Zinken 246, 248 nicht mehr in Eingriff mit den Führungssicken 240, 242 der Schleusenklappen 208, 210, so dass diese durch die V-förmigen Eingriffselemente 456 bis 462 der Sterileinheit 400 weiter geöffnet werden können. In Figur 35 sind die Schleusenklappen 208, 210 und die Führungsklappen 464, 466 sowie die Sterilklappen 202, 204 in einer Position dargestellt, bevor die Schleusenklappen 208, 210 mit Hilfe der V-förmigen Eingriffselemente 456 bis 462 weiter geöffnet werden. Dabei werden sowohl die Schleusenklappen 208, 210 als auch die Führungsklappen 464, 466 und die Sterilklappen 402, 404 weiter bis in die in den Figuren 33, 34 gezeigte Position geöffnet. Um die Unterschiede der verriegelten Position und der entriegelten Position der Entriegelungsstifte 470, 472 zu verdeutlichen, ist der Verriegelungsstift 470 in Figur 35 in seiner verriegelten Position und der Entriegelungsstift 472 in seiner entriegelten Position dargestellt. Hierbei ist zu beachten, dass die Führungsklappe 464 und die Sterilklappe 402 trotz der Anordnung des Entriegelungsstifts 470 in der verriegelten Position um ihre Drehachsen D5, D6 bis in die in Figur 35 dargestellte teilgeöffnete Position verschwenkt worden sind, obwohl dies bei einer tatsächlichen Verriegelung nicht möglich wäre.

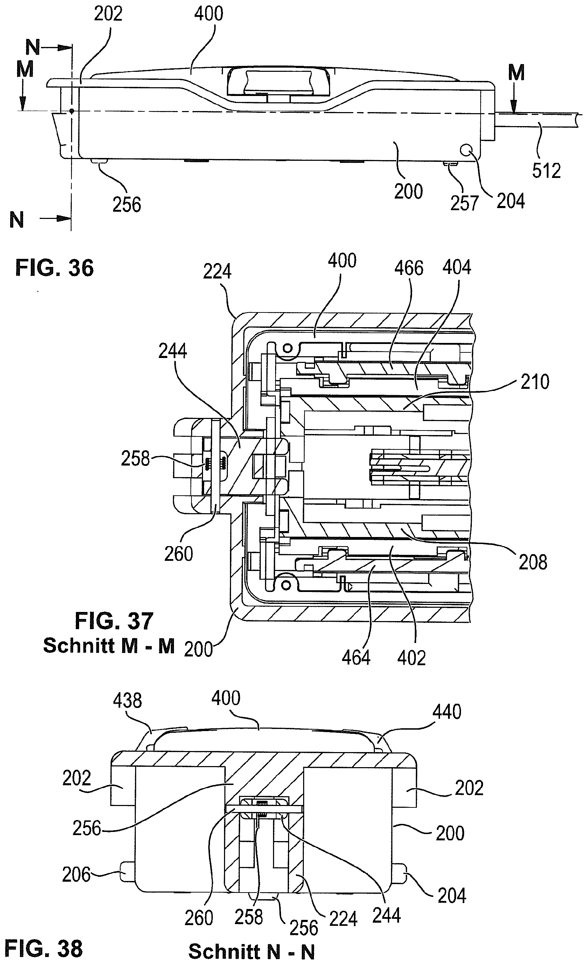

In Figur 36 ist eine Seitenansicht der Anordnung nach den Figuren 30 bis 35 gezeigt und in Figur 37 eine Schnittdarstellung eines Abschnitts der Anordnung nach Figur 36 entlang der Schnittlinie M-M. In Figur 38 ist eine Schnittdarstellung der Anordnung nach Figur 36 entlang der Schnittlinie N-N gezeigt.

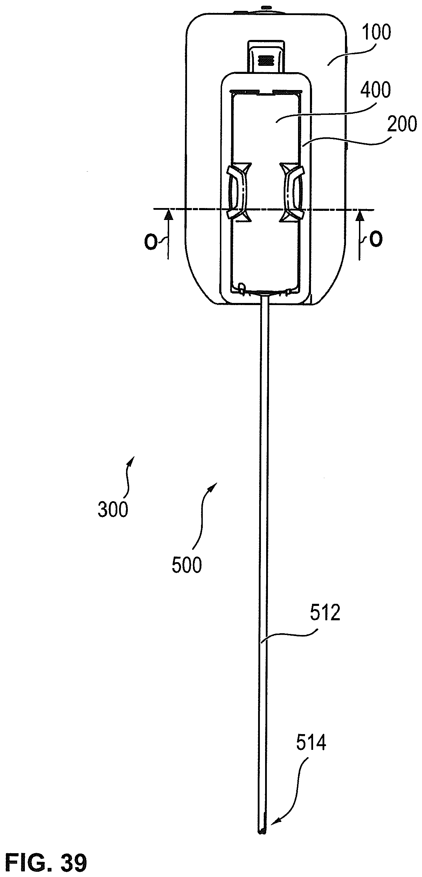

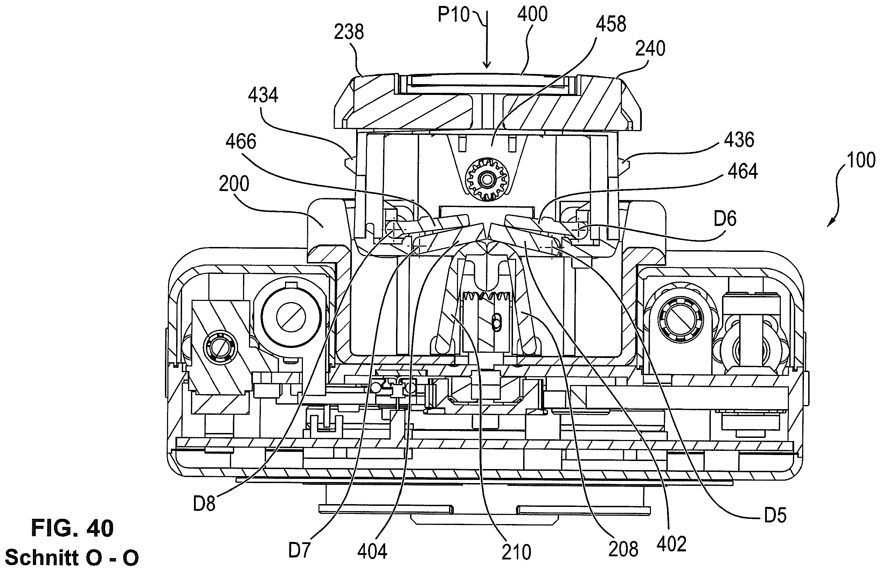

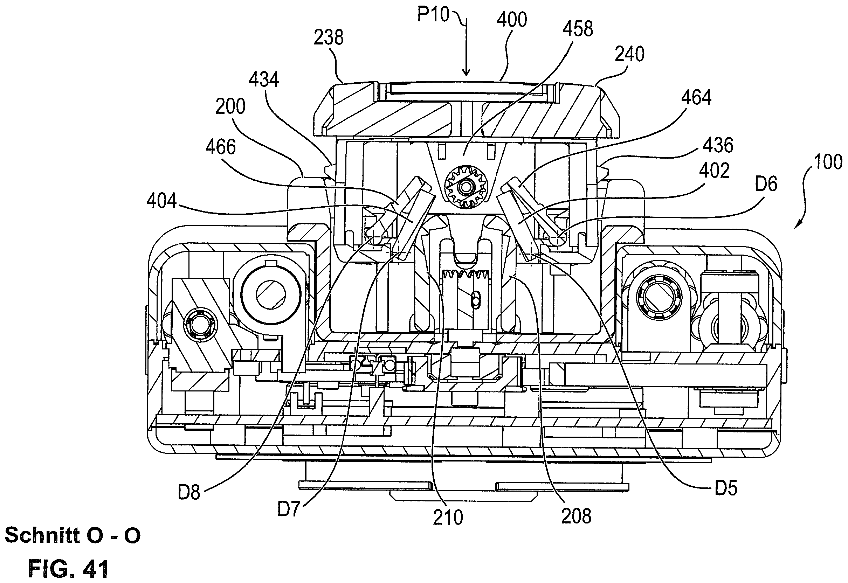

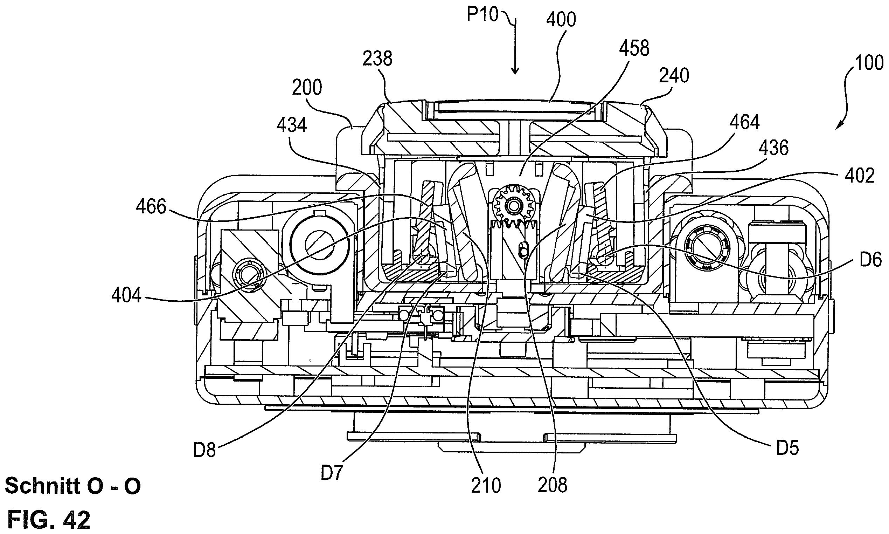

Figur 39 zeigt eine Draufsicht auf eine Anordnung mit der Koppeleinheit 100, der Sterilschleuse 200 und der Instrumenteneinheit 300, welche die sterile Sterileinheit 400 und das sterile chirurgische Instrument 500 umfasst. Figur 40 zeigt eine Schnittdarstellung der Anordnung nach Figur 39 entlang der Schnittlinie O-O in einer ersten Position unmittelbar vor dem Verbinden der Sterileinheit 400 der Instrumenteneinheit 300 mit der bereits mit der Koppeleinheit 100 gekoppelten Sterilschleuse 200. Figur 41 zeigt eine Schnittdarstellung der Anordnung nach Figur 39 entlang der Schnittlinie O-O in einer zweiten Position beim Verbinden der Sterileinheit 400 der Instrumenteneinheit 300 mit der bereits mit der Koppeleinheit 100 gekoppelten Sterilschleuse 200. Figur 42 zeigt eine Schnittdarstellung der Anordnung nach Figur 39 entlang der Schnittlinie O-O in einer dritten Position, in der die Sterileinheit 400 der Instrumenteneinheit 300 mit der mit der Koppeleinheit 100 gekoppelten Sterilschleuse 200 verbunden ist, so dass die ersten Übertragungsmittel 102 der Koppeleinheit 100 zur direkten Koppelung mit den Übertragungselementen der zweiten Übertragungsmittel 406 in Eingriff stehen.

Bei der in Figur 40 gezeigten Position sind die Führungsklappen 464, 466 durch das Einführen der Führungs- und Entriegelungsstege 228, 230 in die Nuten 444, 446 bewegt und dadurch bereits entlang ihrer Drehachse D6, D8 in Richtung der Pfeile P4, P5 von ihrer verriegelten Position in ihre entriegelte Position bewegt worden, so dass die Sterilklappen 402, 404 zusammen mit den Führungsklappen 464, 466 durch eine Bewegung der Sterileinheit 400 in Richtung des Pfeils P10 und den dadurch bewirkten Kontakt der Sterilklappen 402, 404 mit den Schleusenklappen 208, 210 aufgedrückt worden sind. Dadurch gelangt die Sterileinheit 400 tiefer in den zur Aufnahme der Sterileinheit 400 vorgesehenen Aufnahmebereich der Sterilschleuse 200, so dass der Betätigungssteg 454 in Eingriff mit der Führungsgabel 244 gelangt und diese entgegen der Federkraft der Führungsgabelfeder 258 verschwenkt. Dadurch stehen die Zinken 246, 248 der Führungsgabel 244 so in Eingriff mit den Führungssicken 240, 242, dass die Schleusenklappen 208, 210 durch die Nocken 415, 417 auseinander gedrückt und dadurch geöffnet werden können. Bei einer weiteren Bewegung der Sterileinheit 400 in Richtung des Pfeils P10 kommen die V-förmigen Eingriffselemente 458 bis 462 in Eingriff mit den Schleusenklappen 208, 210 und drücken diese und die Sterilklappen 402, 404 zusammen mit den Führungsklappen 464, 466 weiter nach außen in ihre in Figur 42 gezeigte voll geöffnete Position. Durch den Kontakt der Schleusenklappen 208, 210 mit den Sterilklappen 402, 404 werden diese zusammen mit den Führungsklappen 464, 466 weiter geöffnet, bis alle Klappen 208, 210, 402, 404, 464, 466 in der in Figur 42 gezeigten geöffneten Position angeordnet sind.

Bei einer umgekehrten Bewegung der Sterileinheit 400 beim Entnehmen der Sterileinheit 400 aus der Sterilschleuse 200, d.h. von der in Figur 42 gezeigten Position in die in Figur 40 gezeigte Position entgegen der Richtung des Pfeils P10, erfolgt ein umgekehrter Bewegungsablauf der Steril-, Führungs- und Schleusenklappen, so dass diese Klappen insbesondere durch die Federkraft der Federn 474, 476 geschlossen werden und die Zinken 246, 248 der Führungsgabel 244 wieder in Eingriff mit den Führungssicken 240, 242 gebracht werden und die Schleusenklappe 208, 210 vollständig schließen. Durch den so erzeugten Formschluss der Gabelzinken 246, 248 mit den Führungssicken 240, 244 werden die Schleusenklappen 208, 210 sicher in ihrer geschlossenen Position gehalten, so dass die Schleusenklappen 208, 210 nicht von außen geöffnet werden können. Auch werden die Führungsklappen 464, 466 beim Entfernen der Sterileinheit 400 aus der Sterilschleuse 200 mit Hilfe der Federn 474, 476 vollständig geschlossen und in ihre verriegelte Position bewegt, so dass anschließend auch bei äußerer Krafteinwirkung auf die Sterilklappen 402, 404 diese nicht geöffnet werden können.

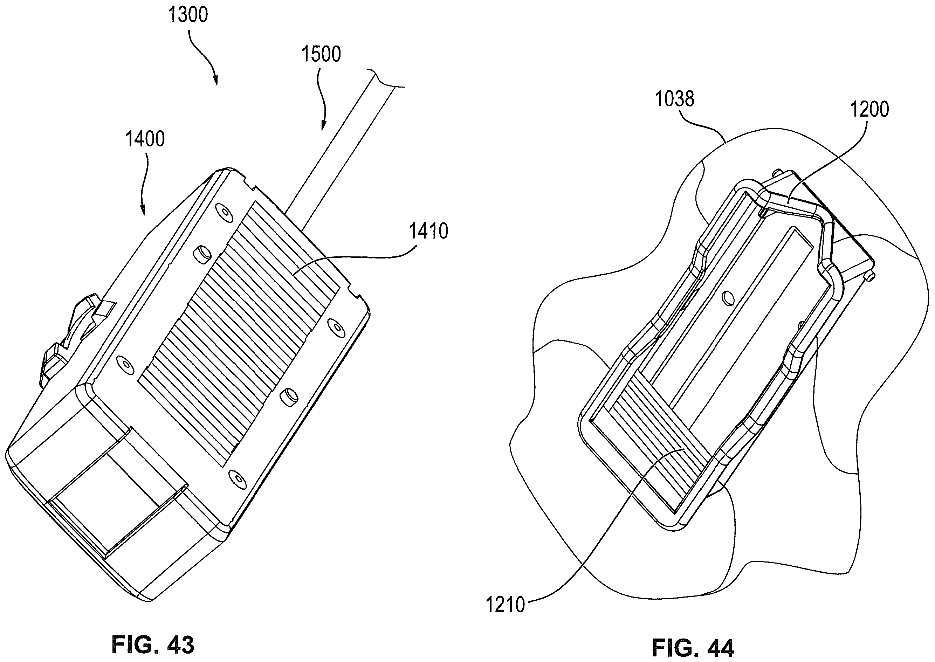

Figur 43 zeigt einen Ausschnitt einer Instrumenteneinheit 1300 mit einer Sterileinheit 1400 und einem chirurgischen Instrument 1500 gemäß einer zweiten Ausführungsform. Im Unterschied zu der Instrumenteneinheit 300 hat die Sterileinheit 1400 der Instrumenteneinheit 1300 keine Sterilklappen sondern eine Jalousie 1410 zum sterilen Abdecken der angetriebenen Elemente. Der weitere Aufbau und die Funktion der Instrumenteneinheit 1300 stimmt mit dem Aufbau und der Funktion der Instrumenteneinheit 300 nach den Figuren 1 bis 42 überein.

Figur 44 zeigt einen Ausschnitt einer sterilen Abdeckung 1038 mit einer Sterilschleuse 1200, die im Unterschied zur Sterilschleuse 200 keine Sterilklappen sondern eine Jalousie 1210 zum sterilen Abschirmen von Antriebselementen der Koppeleinheit 100 hat. Die Jalousie 1410 der Koppeleinheit 1400 als auch die Jalousie 1210 der Sterilschleuse 1200 werden auf geeignete Weise durch einen mechanischen Eingriff beim Verbinden der Sterileinheit 1400 mit der Sterilschleuse 1200 bzw. der Sterileinheit 1400 mit der Sterilschleuse 200 bzw. der Sterileinheit 400 mit der Sterilschleuse 1200 geöffnet. Alternativ können aktive Antriebselemente, wie beispielsweise jeweils ein Elektromotor, zum Öffnen oder Schließen der jeweiligen Jalousie 1410, 1210 vorgesehen sein.

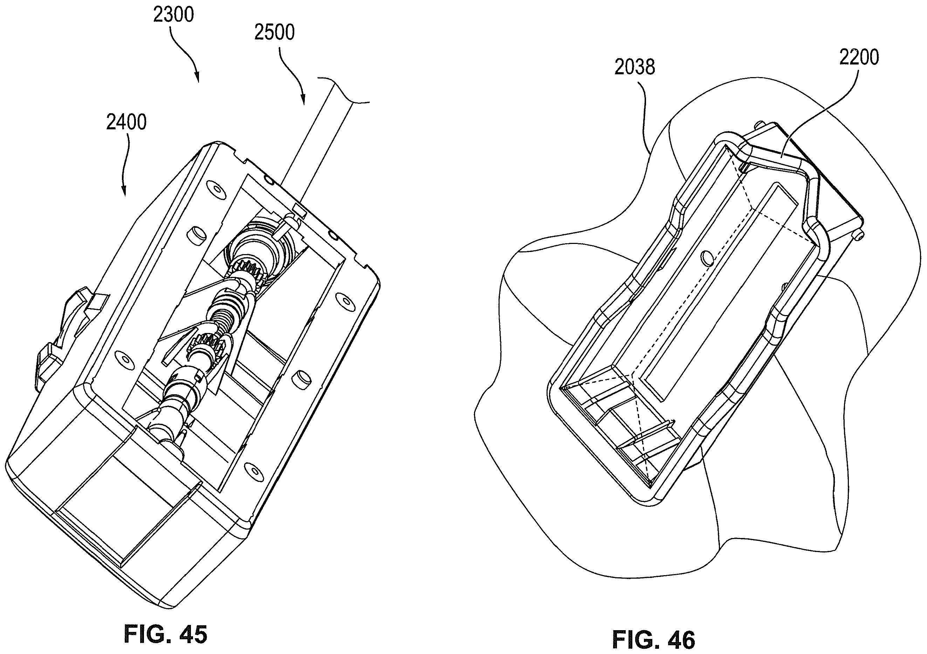

In Figur 45 ist ein Ausschnitt einer Instrumenteneinheit 2300 mit einer Sterileinheit 2400 gemäß einer dritten Ausführungsform gezeigt. Die Sterileinheit 2400 hat keine Elemente zur sterilen Abdeckung der angetriebenen Elemente, sodass diese Instrumenteneinheit 2400 nach dem Trennen von der Sterilschleuse 200, 1200 oder nach dem Trennen von einer weiteren in Figur 46 gezeigten Sterilschleuse 2200 aus dem sterilen Bereich 39 sofort entfernt wird.

Die in Figur 46 gezeigte sterile Abdeckung 2038 umfasst eine Sterilschleuse 2200, die in gleicher Weise wie die Sterileinheit 200 mit der Koppeleinheit 100 verbindbar ist. Im Unterschied zu der Sterilschleuse 200 hat die Sterilschleuse 2200 keine Schleusenklappen sondern umfasst eine mit mithilfe von Punktlinien angedeuteten Sollbruchstellen versehenen Folie, die beim Verbinden einer Sterileinheit 400, 1400, 2400 mit der Sterilschleuse 2200 an den Sollbruchstellen aufgerissen wird, sodass eine direkte Kopplung der Antriebselemente 110 bis 116 der Koppeleinheit 100 mit den angetriebenen Elementen 408 bis 414 einfach möglich ist. Vorzugsweise wird bei einer Verwendung der Sterilschleuse 2200 die Sterileinheit 400, 1400, 2400 während einer Operation nicht getrennt, sondern erst nachdem die Operation beendet worden ist.

### Bezugszeichenliste

- 10: System
- 12: Manipulator
- 14: Stativ
- 16, 16a bis 16d: Manipulatorarm
- 20: Stativkopf
- 22, 26: Antriebseinheit
- 24: Stativfuß
- 28,30: Stativarme
- 32: Operationstischsäule
- 34: Operationstisch
- 36: Steuereinheit
- 37: Ein - und Ausgabeeinheit
- 38: sterile Hülle
- 39: steriler Operationsbereich
- 40a bis 58a, 40b bis 58b, 40c bis 58c, 40d bis 58d: Segmente
- 57, 61, 67: Drehachsen
- 59: Koppelgetriebe
- 60: Segment
- 62, 64, 66: Abschnitte der Teleskopanordnung
- 68: Antriebseinheit
- 69: Pivot-Punkt
- 100: Koppeleinheit
- 102: erstes Übertragungsmittel
- 104: elektrisches Übertragungselement
- 106, 108: elektrischer Kontakt
- 109: optisches Übertragungselement
- 110: erstes translatorisches Antriebselement
- 112: zweites translatorisches Antriebselement
- 114: erstes rotatorisches Antriebselement
- 116: zweites rotatorisches Antriebselementl
- 118, 120: Koppelsensor
- 121: RFID-Lese- und Schreibeinheit
- 122, 124: Führungsnut
- 123, 125: vorderes Ende der Führungsnut
- 126: Rastnase
- 128: Rastelement
- 130: Drehachse
- 132: Feder
- 134: Entriegelungstaste
- 136, 138: Öffnung
- 200: Sterilschleuse
- 202: Anschlussrand
- 204, 206: Führungsstift
- 208, 210: Schleusenklappe
- 212: Boden
- 214, 216: Öffnung
- 218, 220: Seitenwand
- 222: vordere Stirnwand
- 224: hintere Stirnwand
- 226: V-förmige Aussparung
- 228, 230: Führungs- und Entriegelungssteg
- 232, 234: Detektionsfenster
- 236 bis 242: Führungssicke
- 244: Führungsgabel
- 246, 248: Zinken
- 250, 252: Rastsicke
- 254: Führungssteg
- 255: Rastnase
- 256, 257: Positionierungselement
- 258: Führungsgabelfeder
- 260: Führungsstift
- 262, 264: Folie
- 266: erster Verbindungsbereich
- 268: zweiter Verbindungsbereich
- 300, 300a bis 300d: Instrumenteneinheit
- 400: Sterileinheit
- 401: Bodenplatte
- 402,404: Sterilklappe
- 406: zweites Übertragungsmittel
- 408: erstes translatorisch angetriebenes Element
- 410: zweites translatorisch angetriebenes Element
- 412: erstes rotatorisch angetriebenes Element
- 414: zweites rotatorisch angetriebenes Element
- 415, 417: Nocken
- 416, 418: Feder
- 420: Instrumentenschaft
- 421: optische Schnittstelle
- 422, 423: elektrischer Kontakt
- 424: vordere Stirnwand
- 426, 428: Detektionselement
- 430,432: Seitenwand
- 434, 436: Rastelement
- 438,440: Betätigungselement
- 442: vordere Seitenwand
- 444,446: Nut
- 450: hintere Stirnseite
- 452: Führungsnut
- 454: Betätigungssteg
- 456 bis 462: V-förmiges Eingriffselement
- 464,466: Führungsklappe
- 468: Feder
- 470,472: Entriegelungsstift
- 474,476: Feder
- 478 bis 488: Führungs- und Verriegelungsnut
- 479 bis 489: Führungs- und Verriegelungssteg
- 490: Vorsprung
- 494: RFID-Transponder
- 500: Instrument
- 510: Längsachse
- 512: Instrumentenschaft
- 514: Endeffektor
- 516, 518: Greifarm
- D1, D2, D3, D4, D5, D6, D7, D8: Drehachse
- P1 bis P10: Richtungspfeile

## Patentansprüche

1. Vorrichtung zur robotergestützten Chirurgie,
mit mindestens einem nicht sterilen Manipulatorarm (16) mit einer Koppeleinheit (100), die mindestens ein erstes Übertragungsmittel (102) hat,
mit mindestens einer in einem sterilen Bereich (39) angeordneten sterilen Sterileinheit (400), die mindestens ein zweites Übertragungsmittel (406) hat, mit einer sterilen Abdeckung (38) zum Abschirmen zumindest eines Teils des Manipulatorarms (16) von dem sterilen Bereich (39),
wobei die sterile Abdeckung (38) eine Sterilschleuse (200) umfasst, mit der die Koppeleinheit (100) und mit der die Sterileinheit (400) jeweils verbindbar sind,
**dadurch gekennzeichnet, dass** die Sterilschleuse (200) mindestens eine Schleusenklappe (208, 210) hat,
dass die Schleusenklappe (208, 210) in einem geschlossenen Zustand das erste Übertragungsmittel (102) steril abschirmt,
dass beim Verbinden der Sterileinheit (400) mit der Sterilschleuse (200) eine Bewegung der Schleusenklappe (208, 210) vom geschlossenen Zustand in den geöffneten Zustand erfolgt, so dass eine direkte Übertragung zwischen dem ersten Übertragungsmittel (102) und dem zweiten Übertragungsmittel (406) durch eine von der Schleusenklappe (208, 210) im geöffneten Zustand freigegebenen Öffnung (214, 216) möglich ist, und
dass beim Trennen der Sterileinheit (400) von der Sterilschleuse (200) eine Bewegung der Schleusenklappe (208, 210) vom geöffneten Zustand in den geschlossenen Zustand erfolgt, so dass die Schleusenklappe (208, 210) nach dem Trennen das erste Übertragungsmittel (102) vom sterilen Bereich (39) abschirmt.

2. Vorrichtung nach Anspruch 1, **dadurch gekennzeichnet, dass** die Sterileinheit (400) mindestens eine Sterilklappe (402, 404) hat, die in einem geschlossenen Zustand das zweite Übertragungsmittel (406) steril abschirmt; dass beim Verbinden der Sterileinheit (400) mit der Sterilschleuse (200) jeweils eine Bewegung der Schleusenklappe (208, 210) und der Sterilklappe (402, 404) vom geschlossenen Zustand in den geöffneten Zustand erfolgt, so dass eine direkte Übertragung zwischen dem ersten Übertragungsmittel (102) und dem zweiten Übertragungsmittel (406) durch eine von der Schleusenklappe (208, 210) und der Sterilklappe (402, 404) im geöffneten Zustand freigegebenen Öffnung (214, 216) möglich ist; und dass beim Trennen der Sterileinheit (400) von der Sterilschleuse (200) eine Bewegung der Schleusenklappe (208, 210) und der Sterilklappe (402, 404) jeweils vom geöffneten Zustand in den geschlossenen Zustand erfolgt, so dass die Schleusenklappe (208, 210) nach dem Trennen das erste Übertragungsmittel (102) und die Sterilklappe (402, 404) nach dem Trennen das zweite Übertragungsmittel (406) vom sterilen Bereich (39) abschirmen.

3. Vorrichtung nach Anspruch 1 oder 2, **dadurch gekennzeichnet, dass** das erste Übertragungsmittel (102) der Koppeleinheit (100) mindestens ein Antriebselement (110 bis 116) und/oder mindestens eine erstes elektrisches Übertragungselement (104) Schnittstelle und/oder mindestens ein optisches Übertragungselement (109) umfasst,
dass das zweite Übertragungsmittel (406) der Sterileinheit (400) mindestens ein angetriebenes Element (408 bis 414) und/oder mindestens einen elektrischen Kontakt (422, 423) und/oder mindestens ein optisches Übertragungselement (421) umfasst,
dass die Sterilschleuse (200) mit der Koppeleinheit (100) und der Sterileinheit (400) derart verbindbar ist, dass das mindestens eine Antriebselement (110 bis 116) mit dem mindestens einen angetrieben Element (408 bis 414) direkt in Eingriff steht und/oder das erste elektrische Übertragungselement (104) mit dem elektrischen Kontakt (122, 123) und/oder das optische Übertragungselement (109) der Koppeleinheit (100) mit dem optischen Übertragungselement (421) der Sterileinheit (400) direkt gekoppelt ist.

4. Vorrichtung nach einem der vorhergehenden Ansprüche, **dadurch gekennzeichnet, dass** die Koppeleinheit (100) am proximalen Ende des Manipulatorarms (16) angeordnet ist,
dass die Sterileinheit (400) Bestandteil eines chirurgischen Instruments (500), eines Endoskops und/oder eines medizinischen Geräts ist, wobei die Sterileinheit (400) insbesondere am distalen Ende des chirurgischen Instruments (500), des Endoskops und/oder des medizinischen Geräts angeordnet ist,
dass die Koppeleinheit (100) mit einem ersten Verbindungsbereich (266) der Sterilschleuse (200) verbindbar ist,
dass die Sterileinheit (400) mit einem zweiten Verbindungsbereich (268) der Sterilschleuse (200) verbindbar ist, und
dass der erste Verbindungsbereich (266) und der zweite Verbindungsbereich (268) vorzugsweise auf voneinander abgewandten Seiten der Sterilschleuse (200) angeordnet sind.

5. Vorrichtung nach einem der vorhergehenden Ansprüche, **dadurch gekennzeichnet, dass** der erste Verbindungsbereich (266) der Sterilschleuse (200) über eine erste lösbare Rastverbindung mit der Koppeleinheit (100) verbindbar ist, und dass der zweite Verbindungsbereich (268) der Sterilschleuse (200) über eine zweite lösbare Rastverbindung mit der Sterileinheit (400) verbindbar ist.

6. Vorrichtung nach einem der vorhergehenden Ansprüche, **dadurch gekennzeichnet, dass** die Koppeleinheit (100) einen Koppelsensor (118, 120) umfasst, der das Vorhandensein einer korrekt mit der Sterilschleuse (200) verbundenen Sterileinheit (400) detektiert,
dass die Vorrichtung eine Steuereinheit (36) hat, die eine Übertragung zwischen dem ersten Übertragungsmittel (102) und dem zweiten Übertragungsmittel (406) nur dann zulässt, wenn mit Hilfe des Koppelsensors (118, 120) eine korrekt mit der Sterilschleuse (200) verbundene Sterileinheit (400) detektiert worden ist.

7. Vorrichtung nach einem der vorhergehenden Ansprüche, **dadurch gekennzeichnet, dass** die Koppeleinheit (100) als erstes Übertragungsmittel (102) mehrere Antriebselemente (110 bis 116) hat, dass die Sterileinheit (400) mehrere angetriebene Elemente (406 bis 414) hat, wobei die Antriebselemente (110 bis 116) mit den angetriebenen Elementen (408 bis 414) zur mechanischen Kopplung der Koppeleinheit (100) mit der Sterileinheit (400) bei einer Kopplung der Sterileinheit (400) mit der Sterilschleuse (200) und bei einer Kopplung der Koppeleinheit (100) mit der Sterilschleuse (200) in Eingriff stehen, und/oder
dass die Koppeleinheit (100) mindestens einen elektrischen Kontakt (106, 108) als erstes Übertragungsmittel (102) hat und dass die Sterileinheit (400) mindestens einen komplementären elektrischen Kontakt (422, 423) als zweites Übertragungsmittel (406) hat, wobei der elektrische Kontakt (106, 108) der Koppeleinheit (100) und der elektrische Kontakt (422, 423) der Sterileinheit (400) bei einer Kopplung der Koppeleinheit (100) mit der Sterilschleuse (200) und bei einer Kopplung der Sterileinheit (400) mit der Sterilschleuse (200) eine elektrische Verbindung zwischen der Koppeleinheit (100) und der Sterileinheit (400) herstellen, insbesondere zur Übertragung von hochfrequenter elektrischer Energie und/oder
dass die optischen Übertragungselemente (109, 421) eine optische Schnittstelle zwischen Koppeleinheit (100) und Sterileinheit (400) bilden.

8. Vorrichtung nach einem der vorhergehenden Ansprüche, **dadurch gekennzeichnet, dass** die Schleusenklappe (208, 210) den ersten Verbindungsbereich (266) vom zweiten Verbindungsbereich (268) trennt, dass die Schleusenklappe (208, 210) beim Verbinden der Sterileinheit (400) mit dem zweiten Verbindungsbereich (268) automatisch öffnet, und dass die Schleusenklappe (208, 210) beim Trennen der Sterileinheit (400) von dem zweiten Verbindungsbereich (266) automatisch schließt.

9. Vorrichtung nach Anspruch 8, **dadurch gekennzeichnet, dass** die Schleusenklappe (208, 210) beim Verbinden der Sterileinheit (400) mit dem zweiten Verbindungsbereich (268) automatisch entriegelt wird, und dass die Schleusenklappe (208, 210) beim Trennen der Sterileinheit (400) von dem zweiten Verbindungsbereich (268) automatisch verriegelt wird.

10. Vorrichtung nach einem der vorhergehenden Ansprüche, **dadurch gekennzeichnet, dass** die Sterileinheit (400) mindestens eine Sterilklappe (402, 404) hat, die das mindestens eine zweite Übertragungsmittel (406) abdeckt, dass die Sterilklappe (402, 404) beim Verbinden der Sterileinheit (400) mit dem zweiten Verbindungsbereich (268) automatisch öffnet, und dass die Sterilklappe (402, 404) beim Trennen der Sterileinheit (400) vom zweiten Verbindungsbereich (268) automatisch schließt.

11. Vorrichtung nach Anspruch 10, **dadurch gekennzeichnet, dass** die Sterilklappe (402, 404) beim Verbinden der Sterileinheit (400) mit dem zweiten Verbindungsbereich (268) automatisch entriegelt wird, und dass die Sterilklappe (402, 404) beim Trennen der Sterileinheit (400) von dem zweiten Verbindungsbereich (268) automatisch verriegelt wird.

12. Vorrichtung nach einem der vorhergehenden Ansprüche 4 bis 11, **dadurch gekennzeichnet, dass** die sterile Außenseite der Sterilklappe (402, 404) der beim Verbinden der Sterileinheit (400) mit dem zweiten Verbindungsbereich (268) der Sterilschleuse (200) der sterilen Außenseite der Schleusenklappe (208, 210) gegenüberliegend angeordnet ist, wenn sowohl die Sterilklappe (204, 404) als auch die Schleusenklappe (208, 210) geöffnet sind, wobei die sterilen Außenseiten der Sterilklappe (402, 404) und der Schleusenklappe (208, 210) im geöffneten Zustand einander zugewandt sind, sich vorzugsweise berühren können oder gerade noch nicht berühren.

13. Anordnung zur robotergestützten Chirurgie, insbesondere zu einer telerobotergestützten Prozedur innerhalb eines sterilen Bereichs (39),
mit mindestens einer Vorrichtung nach einem der vorhergehenden Ansprüche 1 bis 12,
mit mindestens einer Anzeigeeinheit, die mindestens ein Bild des Operationsgebiets in Echtzeit ausgibt,
mit mindestens einer Eingabeeinrichtung (37) zur Eingabe mindestens eines Eingabekommandos,
mit einer Steuereinheit (9), die den Manipulatorarm (16) und die über die Sterilschleuse (200) mit der Koppeleinheit (100) des Manipulatorarms (16) verbundene Sterileinheit (400) abhängig von dem Eingabekommando mit Hilfe mindestens einer Antriebseinheit positioniert.

14. Sterilschleuse zur robotergestützten Chirurgie,
mit einem ersten Verbindungsbereich (266) zum Verbinden der Sterilschleuse (200) mit einer nicht sterilen Koppeleinheit (100),
mit einem zweiten Verbindungsbereich (268) zum Verbinden der Sterilschleuse (200) mit einer in einem sterilen Bereich angeordneten Sterileinheit (400),
mit einem umlaufenden dritten Verbindungsbereich (202) zum Verbinden der Sterilschleuse (200) mit einer flexiblen sterilen Abdeckung (38) zum Abschirmen des sterilen Bereichs von nicht sterilen Elementen (16, 100),
**dadurch gekennzeichnet, dass** die Sterilschleuse (200) mindestens eine Schleusenklappe (208, 210) hat,
dass die Schleusenklappe (208, 210) in einem geschlossenen Zustand eine Öffnung (214, 216) zwischen dem ersten Verbindungsbereich (266) und dem zweiten Verbindungsbereich (268) steril verschließt,
dass die Schleusenklappe (208, 210) in einem geöffneten Zustand die Öffnung (214, 216) zwischen dem ersten Verbindungsbereich (266) und dem zweiten Verbindungsbereich (268) freigibt.

15. Sterilschleuse nach Anspruch 14, **dadurch gekennzeichnet, dass** die Sterilschleuse (200) derart ausgebildet ist, dass wenn die Sterilschleuse (200) mit der Koppeleinheit (100) verbunden ist beim Verbinden der Sterileinheit (400) mit der Sterilschleuse (200) eine Bewegung der Schleusenklappe (208, 210) vom geschlossenen Zustand in den geöffneten Zustand erfolgt, so dass eine direkte Übertragung zwischen einem ersten Übertragungsmittel (102) der Koppeleinheit (100) und einem zweiten Übertragungsmittel (406) der Sterileinheit (400) durch die von der Schleusenklappe (208, 210) im geöffneten Zustand freigegebenen Öffnung (214, 216) möglich ist, und
dass beim Trennen der Sterileinheit (400) von der Sterilschleuse (200) eine Bewegung der Schleusenklappe (208, 210) vom geöffneten Zustand in den geschlossenen Zustand erfolgt, so dass die Schleusenklappe (208, 210) nach dem Trennen das erste Übertragungsmittel (102) vom sterilen Bereich abschirmt,
und dass die Schleusenklappe (208, 210) im geschlossenen Zustand vorzugsweise verriegelt ist.

16. Verfahren zum sterilen Abdecken eines Manipulatorarms für robotergestützte Chirurgie, insbesondere unter Verwendung einer Vorrichtung nach einem der Ansprüche 1 bis 12, einer Anordnung nach Anspruch 13 und/oder einer Sterilschleuse nach Anspruch 14 oder 15,
bei dem ein nicht steriler Manipulatorarm (16) mit Hilfe einer sterilen Abdeckung (38) und einer in die Abdeckung (38) integrierten Sterilschleuse (200) von einem sterilen Bereich (39) abgeschirmt wird, **dadurch gekennzeichnet, dass** eine nicht sterile Koppeleinheit (100) des Manipulatorarms (16) mit einem ersten Verbindungsbereich (266) der Sterilschleuse (200) verbunden wird, sodass eine Schleusenklappe (208, 210) der Sterilschleuse (200) in einem geschlossenen Zustand eine Öffnung (214, 216) zu mindestens einem in der Koppeleinheit (100) angeordneten ersten Übertragungsmittel (102) steril verschließt,
dass eine in dem sterilen Bereich (39) angeordnete Sterileinheit (400) mit einem zweiten Verbindungsbereich (268) der Sterilschleuse (200) verbunden wird, wobei die Schleusenklappe (208, 210) automatisch geöffnet wird, wobei die Öffnung (214, 216) den ersten Verbindungsbereich (266) mit dem zweiten Verbindungsbereich (268) verbindet, so dass eine Übertragung zwischen dem ersten Übertragungsmittel (102) der Koppeleinheit (100) und einem zweiten Übertragungsmittel (406) der Sterileinheit (400) ermöglicht,
dass die Sterileinheit (400) von dem zweiten Verbindungsbereich (268) getrennt wird, wobei die Schleusenklappe (208, 210) automatisch geschlossen wird und die Öffnung (214, 216) steril verschließt.

17. Verfahren nach Anspruch 16, **dadurch gekennzeichnet, dass** die Sterileinheit (400) mindestens eine Sterilklappe (402, 404) hat; dass das zweite Übertragungsmittel (406) durch die Sterilklappe (402, 404) in deren geschlossenen Zustand steril abgeschirmt wird; dass die Schleusenklappe (208, 210) und die Sterilklappe (402, 404) beim Verbinden der Sterileinheit (400) mit der Sterilschleuse (200) jeweils vom geschlossenen Zustand in den geöffneten Zustand bewegt werden, so dass eine direkte Übertragung zwischen dem ersten Übertragungsmittel (102) und dem zweiten Übertragungsmittel (406) durch eine von der Schleusenklappe (208, 210) und der Sterilklappe (402, 404) im geöffneten Zustand freigegebenen Öffnung (214, 216) möglich ist, und dass beim Trennen der Sterileinheit (400) von der Sterilschleuse (200) die Schleusenklappe (208, 210) und die Sterilklappe (402, 404) jeweils vom geöffneten Zustand in den geschlossenen Zustand bewegt werden, so dass nach dem Trennen das erste Übertragungsmittel (102) mit Hilfe der Schleusenklappe (208, 210) und das zweite Übertragungsmittel (406) mit Hilfe der Sterilklappe (402, 404) vom sterilen Bereich (39) abgeschirmt werden,

## Claims

1. A device for robot-assisted surgery,
with at least one non-sterile manipulator arm (16) having a coupling unit (100) which has at least a first transmitting means (102),
with at least one sterile unit (400) arranged in a sterile area (39) and having at least a second transmitting means (406),
with a sterile cover (38) for shielding at least a part of the manipulator arm (16) from the sterile area (39),
wherein the sterile cover (38) comprises a sterile lock (200) with which the coupling unit (100) and with which the sterile unit (400) are each connectable,
**characterized in that** the sterile lock (200) has at least one lock flap (208, 210),
that the lock flap (208, 210) in a closed state shields the first transmitting means (102) in a sterile manner,
that when connecting the sterile unit (400) to the sterile lock (200) a movement of the lock flap (208, 210) from the closed state into the open state takes place so that a direct transmission between the first transmitting means (102) and the second transmitting means (406) through an opening (214, 216) uncovered by the lock flap (208, 210) in the open state is possible, and
that when separating the sterile unit (400) from the sterile lock (200) a movement of the lock flap (208, 210) from the open state into the closed state takes place so that after separation the lock flap (208, 210) shields the first transmitting means (102) from the sterile area (39).

2. The device according to claim 1, **characterized in that** the sterile unit (400) has at least one sterile flap (402, 404) which in a closed state shields the second transmitting means (406) in a sterile manner; that when connecting the sterile unit (400) to the sterile lock (200) each time a movement of the lock flap (208, 210) and of the sterile flap (402, 404) from the closed state into the open state takes place so that a direct transmission between the first transmitting means (102) and the second transmitting means (406) through an opening (214, 216) uncovered by the lock flap (208, 210) and the sterile flap (402, 404) in the open state is possible; and that when separating the sterile unit (400) from the sterile lock (200), a movement of the lock flap (208, 210) and of the sterile flap (402, 404) each time from the open state into the closed state takes place so that after separation the lock flap (208, 210) shields the first transmitting means (102) from the sterile area (39) and the sterile flap (402, 404) shields the second transmitting means (406) from the sterile area (39).

3. The device according to claim 1 or 2, **characterized in that** the first transmitting means (102) of the coupling unit (100) comprises at least one drive element (110 to 116) and/or at least a first electrical transmitting element (104) interface and/or at least an optical transmitting element (109),
that the second transmitting means (406) of the sterile unit (400) comprises at least a driven element (408 to 414) and/or at least an electrical contact (422, 423) and/or at least an optical transmitting element (421),
that the sterile lock (200) is connectable to the coupling unit (100) and the sterile unit (400) such that the at least one drive element (110 to 116) is directly engaged with the at least one driven element (408 to 414) and/or the first electrical transmitting means (104) is directly coupled with the electrical contact (122, 123) and/or the optical transmitting element (109) of the coupling unit (100) is directly coupled with the optical transmitting element (421) of the sterile unit (400).

4. The device according to one of the preceding claims, **characterized in that** the coupling unit (100) is arranged at the proximal end of the manipulator arm (16),
that the sterile unit (400) forms part of a surgical instrument (500), an endoscope and/or a medical device, wherein the sterile unit (400) is arranged in particular at the distal end of the surgical instrument (500), the endoscope and/or the medical device,
that the coupling unit (100) is connectable to the first connecting area (266) of the sterile lock (200),
that the sterile unit (400) is connectable to a second connecting area (268) of the sterile lock (200), and
that the first connecting area (266) and the second connecting area (268) are preferably arranged on sides of the sterile lock (200) facing away from each other.

5. The device according to one of the preceding claims, **characterized in that** the first connecting area (266) of the sterile lock (200) is connectable to the coupling unit (100) via a first releasable snap-in connection and that the second connecting area (268) of the sterile lock (200) is connectable to the sterile unit (400) via a second releasable snap-in connection.

6. The device according to one of the preceding claims, **characterized in that** the coupling unit (100) comprises a coupling sensor (118, 120) which detects the presence of a sterile unit (400) that is correctly connected to the sterile lock (200),
that the device has a control unit (36) which only allows a transmission between the first transmitting means (102) and the second transmitting means (406) when a sterile unit (400) that is correctly connected to the sterile lock (200) has been detected by means of the coupling sensor (118, 120).

7. The device according to one of the preceding claims, **characterized in that** the coupling unit (100) has several drive elements (110 to 116) as first transmitting means (102), that the sterile unit (400) has several driven elements (406 to 414), wherein the drive elements (110 to 116) are engaged with the driven elements (408 to 414) for a mechanical coupling of the coupling unit (100) with the sterile unit (400) when the sterile unit (400) is coupled with the sterile lock (200) and when the coupling unit (100) is coupled with the sterile lock (200), and/or
that the coupling unit (100) has at least one electrical contact (106, 108) as a first transmitting means (102) and that the sterile unit (400) has at least a complementary electrical contact (422, 423) as a second transmitting means (406), wherein the electrical contact (106, 108) of the coupling unit (100) and the electrical contact (422, 423) of the sterile unit (400) establish an electrical connection between the coupling unit (100) and the sterile unit 400), in particular for transmitting high-frequency electrical energy, when the coupling unit (100) is coupled with the sterile lock (200) and when the sterile unit (400) is coupled with the sterile lock (200), and/or
that the optical transmitting elements (109, 421) form an optical interface between coupling unit (100) and sterile unit (400).

8. The device according to one of the preceding claims, **characterized in that** the lock flap (208, 210) separates the first connecting area (266) from the second connecting area (268), that the lock flap (208, 210) automatically opens when connecting the sterile unit (400) to the second connecting area (268), and that the lock flap (208, 210) automatically closes when separating the sterile unit (400) from the second connecting area (266).

9. The device according to claim 8, **characterized in that** the lock flap (208, 210) is automatically unlocked when connecting the sterile unit (400) to the second connecting area (268) and that the lock flap (208, 210) is automatically locked when separating the sterile unit (400) from the second connecting area (268).

10. The device according to one of the preceding claims, **characterized in that** the sterile unit (400) has at least one sterile flap (402, 404) which covers the at least one second transmitting means (406), that the sterile flap (402, 404) automatically opens when connecting the sterile unit (400) to the second connecting area (268), and that the sterile flap (402, 404) automatically closes when separating the sterile unit (400) from the second connecting area (268).

11. The device according to claim 10, **characterized in that** the sterile flap (402, 404) is automatically unlocked when connecting the sterile unit (400) to the second connecting area (268) and that the sterile flap (402, 404) is automatically locked when separating the sterile unit (400) from the second connecting area (268).

12. The device according to one of the preceding claims 4 to 11, **characterized in that** the sterile outside of the sterile flap (402, 404) and the sterile outside of the lock flap (208, 210) are arranged opposite to each other when connecting the sterile unit (400) to the second connecting area (268), when both the sterile flap (204, 404) and the lock flap (208, 210) are open, wherein the sterile outsides of the sterile flap (402, 404) and the lock flap (208, 210) face each other in the open state, preferably can contact each other or just do not contact each other yet.

13. An arrangement for robot-assisted surgery, in particular for a telerobot-assisted procedure within a sterile area (39),
with at least one device according to one of the preceding claims 1 to 12,
with at least one display unit which outputs at least one image of the operating area in real time,
with at least one input device (37) for the input of at least one input command,
with a control unit (9) which positions the manipulator arm (16) and the sterile unit (400) connected via the sterile lock (200) to the coupling unit (100) of the manipulator arm (16) dependent on the input command by means of at least one drive unit.

14. A sterile lock for robot-assisted surgery,
with a first connecting area (266) for connecting the sterile lock (200) to a non-sterile coupling unit (100),
with a second connecting area (268) for connecting the sterile lock (200) to a sterile unit (400) arranged in a sterile area,
with a circumferential third connecting area (202) for connecting the sterile lock (200) to a flexible sterile cover (38) for shielding the sterile area from the non-sterile elements (16, 100),
**characterized in that** the sterile lock (200) has at least one lock flap (208, 210),
that the lock flap (208, 210) in a closed state closes an opening (214, 216) between the first connecting area (266) and the second connecting area (268) in a sterile manner,
that the lock flap (208, 210) in an open state uncovers the opening (214, 216) between the first connecting area (266) and the second connecting area (268).

15. The sterile lock according to claim 14, **characterized in that** the sterile lock (200) is designed such that when the sterile lock (200) is connected to the coupling unit (100), a movement of the lock flap (208, 210) from the closed state into the open state takes place when connecting the sterile unit (400) to the sterile lock (200) so that a direct transmission between a first transmitting means (102) of the coupling unit (100) and a second transmitting means (406) of the sterile unit (400) through the opening (214, 216) uncovered by the lock flap (208, 210) in the open state is possible, and
that when separating the sterile unit (400) from the sterile lock (200) a movement of the lock flap (208, 210) from the open state into the closed state takes place so that after separation the lock flap (208, 210) shields the first transmitting means (102) from the sterile area,
and that the lock flap (208, 210) is preferably locked in the closed state.

16. A method for the sterile covering of a manipulator arm for robot-assisted surgery, in particular by using a device according to one of the claims 1 to 12, an arrangement according to claim 13 and/or a sterile lock according to claim 14 or 15,
in which a non-sterile manipulator arm (16) is shielded from a sterile area (39) by means of a sterile cover (38) and a sterile lock (200) integrated in the cover (38), **characterized in that** a non-sterile coupling unit (100) of the manipulator arm (16) is connected to a first connecting area (266) of the sterile lock (200) so that in a closed state a lock flap (208, 210) of the sterile lock (200) closes an opening (214, 216) to at least one first transmitting means (102) arranged in the coupling unit (100) in a sterile manner,
that a sterile unit (400) arranged in the sterile area (39) is connected to a second connecting area (268) of the sterile lock (200), wherein the lock flap (208, 210) is automatically opened, wherein the opening (214, 216) connects the first connecting area (266) to the second connecting area (268) so that a transmission between the first transmitting means (102) of the coupling unit (100) and a second transmitting means (406) of the sterile unit (400) is possible,
that the sterile unit (400) is separated from the second connecting area (268), wherein the lock flap (208, 210) is automatically closed and closes the opening (214, 216) in a sterile manner.

17. The method according to claim 16, **characterized in that** the sterile unit (400) has at least one sterile flap (402, 404); that the second transmitting means (406) is shielded by the sterile flap (402, 404) in its closed state in a sterile manner; that the lock flap (208, 210) and the sterile flap (402, 404) are each moved from the closed state into the open state when connecting the sterile unit (400) to the sterile lock (200) so that a direct transmission between the first transmitting means (102) and the second transmitting means (406) through an opening (214, 216) uncovered by the lock flap (208, 210) and the sterile flap (402, 404) in the open state is possible, and that when separating the sterile unit (400) from the sterile lock (200), the lock flap (208, 210) and the sterile flap (402, 404) are each moved from the open state into the closed state so that after the separation the first transmitting means (102) is shielded from the sterile area (39) by means of the lock flap (208, 210) and the second transmitting means (406) is shielded from the sterile area (39) by means of the sterile flap (402, 404).

## Revendications

1. Dispositif pour la chirurgie robotisée,
avec au moins un bras manipulateur (16) non stérile avec une unité d'accouplement (100), qui présente au moins un premier moyen de transmission (102),
avec au moins une unité stérile (400) disposée dans une zone stérile (39), qui présente au moins un deuxième moyen de transmission (406),
avec un cache stérile (38) pour protéger au moins une partie du bras manipulateur (16) de la zone stérile (39),
dans lequel le cache stérile (38) comprend un sas stérile (200), auquel l'unité d'accouplement (100) et auquel l'unité stérile (400) peuvent être reliées respectivement,
**caractérisé en ce**
**que** le sas stérile (200) présente au moins un clapet de sas (208, 210),
**que** le clapet de sas (208, 210) dans un état fermé protège le premier moyen de transmission (102) de manière stérile,
**que** lors de la liaison de l'unité stérile (400) au sas stérile (200), un mouvement du clapet de sas (208, 210) de l'état fermé dans l'état ouvert s'effectue, de sorte qu'une transmission directe entre le premier moyen de transmission (102) et le deuxième moyen de transmission (406) à travers une ouverture (214, 216) libérée par le clapet de sas (208, 210) dans l'état ouvert est possible, et
**que** lors de la séparation de l'unité stérile (400) du sas stérile (200), un mouvement du clapet de sas (208, 210) de l'état ouvert dans l'état fermé s'effectue, de sorte que le clapet de sas (208, 210) après la séparation protège le premier moyen de transmission (102) de la zone stérile (39).

2. Dispositif selon la revendication 1, **caractérisé en ce que** l'unité stérile (400) présente au moins un clapet stérile (402, 404), qui dans un état fermé protège le deuxième moyen de transmission (406) de manière stérile ; que lors de la liaison de l'unité stérile (400) au sas stérile (200) respectivement un mouvement du clapet de sas (208, 210) et du clapet stérile (402, 404) de l'état fermé dans l'état ouvert s'effectue, de sorte qu'une transmission directe entre le premier moyen de transmission (102) et le deuxième moyen de transmission (406) à travers une ouverture (214, 216) libérée par le clapet de sas (208, 210) et le clapet stérile (402, 404) dans l'état ouvert est possible ; et que lors de la séparation de l'unité stérile (400) du sas stérile (200), un mouvement du clapet de sas (208, 210) et du clapet stérile (402, 404) respectivement de l'état ouvert dans l'état fermé s'effectue, de sorte que le clapet de sas (208, 210) après la séparation protège le premier moyen de transmission (102) et le clapet stérile (402, 404) après la séparation protège le deuxième moyen de transmission (406) de la zone stérile (39).

3. Dispositif selon la revendication 1 ou 2, **caractérisé en ce que** le premier moyen de transmission (102) de l'unité d'accouplement (100) comprend au moins un élément d'entraînement (110 à 116) et/ou au moins un premier élément de transmission électrique (104) interface et/ou au moins un élément de transmission optique (109),
**que** le deuxième moyen de transmission (406) de l'unité stérile (400) comprend au moins un élément entraîné (408 à 414) et/ou au moins un contact électrique (422, 423) et/ou au moins un élément de transmission optique (421),
**que** le sas stérile (200) peut être relié à l'unité d'accouplement (100) et à l'unité stérile (400), de telle sorte que l'au moins un élément d'entraînement (110 à 116) est directement en prise avec l'au moins un élément entraîné (408 à 414) et/ou le premier élément de transmission électrique (104) est accouplé directement au contact électrique (122, 123) et/ou l'élément de transmission optique (109) de l'unité d'accouplement (100) à l'élément de transmission optique (421) de l'unité stérile (400).

4. Dispositif selon l'une quelconque des revendications précédentes, **caractérisé en ce que** l'unité d'accouplement (100) est disposée à l'extrémité proximale du bras manipulateur (16),
**que** l'unité stérile (400) fait partie d'un instrument chirurgical (500), d'un endoscope et/ou d'un appareil médical, dans lequel l'unité stérile (400) est disposée en particulier à l'extrémité distale de l'instrument chirurgical (500), de l'endoscope et/ou de l'appareil médical,
**que** l'unité d'accouplement (100) peut être reliée à une première zone de liaison (266) du sas stérile (200),
**que** l'unité stérile (400) peut être reliée à une deuxième zone de liaison (268) du sas stérile (200), et
**que** la première zone de liaison (266) et la deuxième zone de liaison (268) sont disposées de préférence sur les faces opposées l'une à l'autre du sas stérile (200).

5. Dispositif selon l'une quelconque des revendications précédentes, **caractérisé en ce que** la première zone de liaison (266) du sas stérile (200) peut être reliée à l'unité d'accouplement (100) par l'intermédiaire d'une première liaison d'encliquetage libérable, et que la deuxième zone de liaison (268) du sas stérile (200) peut être reliée à l'unité stérile (400) par l'intermédiaire d'une deuxième liaison d'encliquetage libérable.

6. Dispositif selon l'une quelconque des revendications précédentes, **caractérisé en ce que** l'unité d'accouplement (100) comprend un capteur d'accouplement (118, 120), qui détecte la présence d'une unité stérile (400) reliée correctement au sas stérile (200),
**que** le dispositif présente une unité de commande (36), qui ne permet une transmission entre le premier moyen de transmission (102) et le deuxième moyen de transmission (406) que lorsqu'une unité stérile (400) reliée correctement au sas stérile (200) a été détectée à l'aide du capteur d'accouplement (118, 120).

7. Dispositif selon l'une quelconque des revendications précédentes, **caractérisé en ce que** l'unité d'accouplement (100) présente comme premier moyen de transmission (102) plusieurs éléments d'entraînement (110 à 116), que l'unité stérile (400) présente plusieurs éléments entraînés (406 à 414), dans lequel les éléments d'entraînement (110 à 116) sont en prise avec les éléments entraînés (408 à 414) pour l'accouplement mécanique de l'unité d'accouplement (100) à l'unité stérile (400) lors d'un accouplement de l'unité stérile (400) au sas stérile (200) et lors d'un accouplement de l'unité d'accouplement (100) au sas stérile (200), et/ou
**que** l'unité d'accouplement (100) présente au moins un contact électrique (106, 108) comme premier moyen de transmission (102) et que l'unité stérile (400) présente au moins un contact électrique (422, 423) complémentaire comme deuxième moyen de transmission (406), dans lequel le contact électrique (106, 108) de l'unité d'accouplement (100) et le contact électrique (422, 423) de l'unité stérile (400) lors d'un accouplement de l'unité d'accouplement (100) au sas stérile (200) et lors d'un accouplement de l'unité stérile (400) au sas stérile (200) établissent une liaison électrique entre l'unité d'accouplement (100) et l'unité stérile (400), en particulier pour la transmission d'énergie électrique haute fréquence et/ou
**que** les éléments de transmission optiques (109, 421) forment une interface optique entre l'unité d'accouplement (100) et l'unité stérile (400).

8. Dispositif selon l'une quelconque des revendications précédentes, **caractérisé en ce que** le clapet de sas (208, 210) sépare la première zone de liaison (266) de la deuxième zone de liaison (268), que le clapet de sas (208, 210) lors de la liaison de l'unité stérile (400) à la deuxième zone de liaison (268) s'ouvre automatiquement, et que le clapet de sas (208, 210) lors de la séparation de l'unité stérile (400) de la deuxième zone de liaison (266) se ferme automatiquement.

9. Dispositif selon la revendication 8, **caractérisé en ce que** le clapet de sas (208, 210) lors de la liaison de l'unité stérile (400) à la deuxième zone de liaison (268) est déverrouillé automatiquement, et que le clapet de sas (208, 210) lors de la séparation de l'unité stérile (400) de la deuxième zone de liaison (268) est verrouillé automatiquement.

10. Dispositif selon l'une quelconque des revendications précédentes, **caractérisé en ce que** l'unité stérile (400) présente au moins un clapet stérile (402, 404), qui recouvre l'au moins un deuxième moyen de transmission (406), que le clapet stérile (402, 404) lors de la liaison de l'unité stérile (400) à la deuxième zone de liaison (268) s'ouvre automatiquement, et que le clapet stérile (402, 404) lors de la séparation de l'unité stérile (400) de la deuxième zone de liaison (268) se ferme automatiquement.

11. Dispositif selon la revendication 10, **caractérisé en ce que** le clapet stérile (402, 404) lors de la liaison de l'unité stérile (400) à la deuxième zone de liaison (268) est déverrouillé automatiquement, et que le clapet stérile (402, 404) lors de la séparation de l'unité stérile (400) de la deuxième zone de liaison (268) est verrouillé automatiquement.

12. Dispositif selon l'une quelconque des revendications 4 à 11, **caractérisé en ce que** la face extérieure stérile du clapet stérile (402, 404) de lors de la liaison de l'unité stérile (400) à la deuxième zone de liaison (268) du sas stérile (200) est disposée à l'opposé de la face extérieure stérile du clapet de sas (208, 210), lorsqu'aussi bien le clapet stérile (204, 404) que le clapet de sas (208, 210) sont ouverts, dans lequel les faces extérieures stériles du clapet stérile (402, 404) et du clapet de sas (208, 210) dans l'état ouvert sont tournées l'une vers l'autre, peuvent se toucher de préférence ou justement ne se touchent pas encore.

13. Ensemble pour la chirurgie robotisée, en particulier pour une procédure télérobotisée à l'intérieur d'une zone stérile (39),
avec au moins un dispositif selon l'une quelconque des revendications précédentes 1 à 12,
avec au moins une unité d'affichage, qui sort au moins une image du champ opératoire en temps réel,
avec au moins un système d'entrée (37) pour l'entrée d'au moins une instruction d'entrée,
avec une unité de commande (9), qui positionne le bras manipulateur (16) et l'unité stérile (400) reliée à l'unité d'accouplement (100) du bras manipulateur (16) par l'intermédiaire du sas stérile (200) en fonction de l'instruction d'entrée à l'aide d'au moins une unité d'entraînement.

14. Sas stérile pour la chirurgie robotisée,
avec une première zone de liaison (266) pour la liaison du sas stérile (200) à une unité d'accouplement (100) non stérile,
avec une deuxième zone de liaison (268) pour la liaison du sas stérile (200) à une unité stérile (400) disposée dans une zone stérile,
avec une troisième zone de liaison (202) périphérique pour la liaison du sas stérile (200) à un cache stérile (38) flexible pour protéger la zone stérile d'éléments (16, 100) non stériles,
**caractérisé en ce que** le sas stérile (200) présente au moins un clapet de sas (208, 210),
**que** le clapet de sas (208, 210) dans un état fermé ferme une ouverture (214, 216) entre la première zone de liaison (266) et la deuxième zone de liaison (268) de manière stérile,
**que** le clapet de sas (208, 210) dans un état ouvert libère l'ouverture (214, 216) entre la première zone de liaison (266) et la deuxième zone de liaison (268).

15. Sas stérile selon la revendication 14, **caractérisé en ce que** le sas stérile (200) est réalisé de telle sorte que lorsque le sas stérile (200) est relié à l'unité d'accouplement (100), lors de la liaison de l'unité stérile (400) au sas stérile (200), un mouvement du clapet de sas (208, 210) de l'état fermé dans l'état ouvert s'effectue, de sorte qu'une transmission directe entre un premier moyen de transmission (102) de l'unité d'accouplement (100) et un deuxième moyen de transmission (406) de l'unité stérile (400) à travers l'ouverture (214, 216) libérée par le clapet de sas (208, 210) dans l'état ouvert est possible, et
**que** lors de la séparation de l'unité stérile (400) du sas stérile (200), un mouvement du clapet de sas (208, 210) de l'état ouvert dans l'état fermé s'effectue, de sorte que le clapet de sas (208, 210) après la séparation protège le premier moyen de transmission (102) de la zone stérile,
et que le clapet de sas (208, 210) dans l'état fermé est de préférence verrouillé.

16. Procédé pour le recouvrement stérile d'un bras manipulateur pour la chirurgie robotisée, en particulier au moyen d'un dispositif selon l'une quelconque des revendications 1 à 12, d'un ensemble selon la revendication 13 et/ou d'un sas stérile selon la revendication 14 ou 15,
selon lequel un bras manipulateur (16) non stérile est protégé d'une zone stérile (39) à l'aide d'un cache stérile (38) et d'un sas stérile (200) intégré dans le cache (38), **caractérisé en ce**
**qu'**une unité d'accouplement (100) non stérile du bras manipulateur (16) est reliée à une première zone de liaison (266) du sas stérile (200), de sorte qu'un clapet de sas (208, 210) du sas stérile (200) dans un état ouvert ferme de manière stérile une ouverture (214, 216) par rapport à au moins un premier moyen de transmission (102) disposé dans l'unité d'accouplement (100),
**qu'**une unité stérile (400) disposée dans la zone stérile (39) est reliée à une deuxième zone de liaison (268) du sas stérile (200), dans lequel le clapet de sas (208, 210) est ouvert automatiquement, dans lequel l'ouverture (214, 216) relie la première zone de liaison (266) à la deuxième zone de liaison (268), de sorte qu'une transmission entre le premier moyen de transmission (102) de l'unité d'accouplement (100) et un deuxième moyen de transmission (406) de l'unité stérile (400) permise,
**que** l'unité stérile (400) est séparée de la deuxième zone de liaison (268), dans lequel le clapet de sas (208, 210) est fermé automatiquement et ferme l'ouverture (214, 216) de manière stérile.

17. Procédé selon la revendication 16, **caractérisé en ce que** l'unité stérile (400) présente au moins un clapet stérile (402, 404) ; que le deuxième moyen de transmission (406) est protégé de manière stérile par le clapet stérile (402, 404) dans l'état fermé de celui-ci ; que le clapet de sas (208, 210) et le clapet stérile (402, 404) lors de la liaison de l'unité stérile (400) au sas stérile (200) sont déplacés respectivement de l'état fermé dans l'état ouvert, de sorte qu'une transmission directe entre le premier moyen de transmission (102) et le deuxième moyen de transmission (406) à travers une ouverture (214, 216) libérée par le clapet de sas (208, 210) et le clapet stérile (402, 404) dans l'état ouvert est possible, et que lors de la séparation de l'unité stérile (400) du sas stérile (200) le clapet de sas (208, 210) et le clapet stérile (402, 404) sont déplacés respectivement de l'état ouvert dans l'état fermé, de sorte qu'après la séparation le premier moyen de transmission (102) est protégé de la zone stérile (39) à l'aide du clapet de sas (208, 210) et le deuxième moyen de transmission (406) à l'aide du clapet stérile (402, 404).
